# EUROPEAN PATENT APPLICATION

(11) **EP 4 763 210 A1**
(43) Date of publication of application: **24.06.2026**
(21) Application number: 24222812.0
(22) Date of filing: 23.12.2024
(51) Int. Cl.: A61K 35/15, A61K 35/16, A61K 35/28, A61K 35/50, A61K 35/51, A61P 1/00, A61P 1/16, A61P 1/18, A61P 31/12, A61P 35/00, A61P 37/00, C12N 5/074, C12N 15/00

(54) **IMMUNE CELL THERAPY FOR FIBROTIC DISEASES**

(71) Applicant: Technische Universität Dresden, 01069 Dresden (DE)
(72) Inventor: SIEWEKE, Michael, 01069 Dresden (DE); SUBRAMANIAN, Sethuraman, 01069 Dresden (DE)
(74) Representative: Maikowski & Ninnemann Patentanwälte Partnerschaft mbB

(57) **Abstract**

The present invention relates to the treatment of fibrosis with a cell therapy based on myeloid cells. The invention provides myeloid cells, such as monocytes or macrophages that are resistant to M-CSF induced expression of a profibrotic gene signature. Said myeloid cells may be genetically modified in that the expression or activity of the transcription factors MAFB and/or MAF is reduced, inhibited or abolished. The administration of such myeloid cells to a subject leads to the reduction of fibrosis. Even more surprisingly, it is demonstrated by the invention that cell therapy with genetically engineered myeloid cells shows a dominant effect against the background of resident macrophages and monocytes after administration to mice in vivo. In particular, a dominant effect of Maf-DKO monocytes on the fibrosis-generating microenvironment of the lung is demonstrated, which indicates that these cells can also be used for the treatment of other fibrotic diseases.

## Description

### BACKGROUND OF THE INVENTION

Fibrosis is an important cause of global morbidity and mortality. Common diseases associated with fibrosis include hepatitis virus, metabolic dysfunction-associated steatotic liver disease (MASLD) including liver fibrosis and cirrhosis, chronic kidney diseases, idiopathic pulmonary fibrosis (IPF), pneumonconiosis, and cystic fibrosis. The annual combined incidence of major fibrosis-related diseases is approximately 4968 per 100,000 person-years, causing huge disease burden (Zhao X. et al., 2020). Fibrosis-related diseases accounted for a large proportion of global disability-adjusted life-years (DALYs) in 2019 (Global burden of 369 diseases and injuries in 204 countries and territories, 1990-2019: a systematic analysis for the Global Burden of Disease Study 2019. Lancet. 396, 1204-1222 (2020). Therefore, fibrosis is increasingly recognized as a major health challenge.

Fibrosis is characterized by the excessive extracellular matrix deposition due to dysregulated wound and connective tissue repair response. Multiple organs can develop fibrosis, including the liver, kidney, heart, and lung. Fibrosis such as liver cirrhosis, idiopathic pulmonary fibrosis, and cystic fibrosis caused substantial disease burden. Persistent abnormal activation of myofibroblasts mediated by various signals, such as transforming growth factor, platelet-derived growth factor, and fibroblast growth factor, has been recognized as a major event in the occurrence and progression of fibrosis.

Accordingly, fibrosis is a pathological wound healing process that is observed in several chronic inflammatory diseases. The excessive deposition of fibrous connective tissue including collagen and other components of extracellular matrix (ECM) leads to an irreversible and aberrant tissue remodeling which results in development of scar tissue. Progressive fibrosis leads to permanent scarring and organ dysfunction with high morbidity and mortality. Fibrosis has been observed as a major pathological feature in several pulmonary diseases including idiopathic pulmonary fibrosis (IPF), fibrosis occurring in chronic obstructive pulmonary disease (COPD), interstitial lung disease (ILD), viral infections (severe COVID-19 and pneumonia) and in other diseases such as end-stage liver diseases (including MASH, NASH and liver cirrhosis), autoimmune related diseases (SLE and GvHD) and several cancers (hepatic, pancreatic, gastric, oesophagal, colonic, lung).

The small molecules nintedanib and pirfenidone are approved drugs against IPF. Experiments are also underway to prevent the polarization of resident macrophages and/or monocytes in vivo. Nintedanib and pirfenidone only slow down IPF, so there is a medical need for fibrosis treatments. There have also been trials with mesenchymal stem cells that indirectly influenced the macrophage composition and activation state in fibrotic tissue. However, attempts to treat fibrosis in vivo via macrophage repolarization have so far been less successful (Perrot et al., 2023; for an overview of the current state of research).

Macrophages have also been used as cell therapy in the treatment of liver fibrosis (Starkey et al., 2019). However, M-CSF, a key cytokine supporting macrophage growth, survival and activation is also induced under fibrotic conditions and has been shown to aggravate fibrosis. Indeed, its depletion has been shown to improve lung fibrosis in animal models (Joshi et al. 2020), putting the utility of macrophage cellular therapy for the treatment of fibrosis into question, as they might be converted into a profibrogenic phenotype by endogenously released M-CSF in the fibrotic microenvironment.

Recently, several single cell transcriptomic analyses of fibrotic lungs from mice and humans have identified several genes that are upregulated in fibrotic conditions among which the transcription factors of the Maf family, MafB and cMaf, were also present. It was shown previously that MafB and cMaf double knock-out (Maf-DKO) mouse macrophages could be expanded indefinitely ex vivo without loss of function or tumorigenic transformation (Aziz et al., 2009). It was also shown that MafB and cMaf bind to macrophage-specific enhancers of self-renewal target genes, partially shared with ES cells, thereby inactivating their gene expression and subsequently prevent macrophage self-renewal. (Soucie et al., 2016)

Interestingly, these transcription factors seem to play an important role beyond IPF, more broadly in various fibrotic diseases including liver fibrosis, cancer and COVID-19 related fibrosis.

Current available therapies have been able to slow down fibrosis progression while still unable to reverse established fibrosis. Therefore, a strong need exists for the development of new therapeutic modalities optimized to reduce and treat fibrosis.

### SUMMARY OF THE INVENTION

Previous approaches for cell therapies of fibrosis have attempted to manipulate myeloid cells, such as macrophages in order to achieve a therapeutic effect. However, it is known that M-CSF is released under fibrotic conditions and can stimulate fibrosis. It is further known that macrophages are responsive to M-CSF. Therefore, there is a danger that these cells are converted into a pro-fibrotic phenotype upon exposure to endogenous M-CSF.

The problem addressed by the invention is therefore to provide myeloid cells for fibrosis therapy that do not show the disadvantages of cell therapies known from the prior art.

This problem is solved by the present invention by the provision of myeloid cells, such as monocytes or macrophages, that are resistant to the induction of a profibrotic gene signature in response to M-CSF. The administration of such myeloid cells to a subject surprisingly leads to the reduction of fibrosis.

Surprisingly, it was found and demonstrated herein that cell therapy with myeloid cells resistant to the induction of a profibrotic gene signature in response to M-CSF, such as monocytes or macrophages show a dominant effect against the background of resident macrophages and monocytes after administration to mice in vivo.

In particular, it has been shown that there is a dominant effect of monocytes deficient for the transcription factors MafB and Maf (Maf-DKO) on the fibrosis-generating microenvironment of the lung. It is also shown that (Maf-DKO) macrophages are resistant to induction by M-CSF of profibrotic signatures observed in macrophages of fibrotic lungs and other organs, such as the liver. Together this indicates that macrophages resistant to the induction of pro-fibrotic signatures by M-CSF can also be used for the treatment of other fibrotic diseases.

MafB and cMaf are specifically upregulated when monocytes differentiate to macrophages and on most tissue resident macrophages. In order to assess the role of self-renewing Maf-DKO macrophages and its regenerative impact in fibrosis in vivo, mice with a LyzM-Cre driver crossed on floxed MafB and Maf alleles generating a myeloid specific deletion of MafB and cMaf (myeloid Maf-DKO) on a cellular level is provided by the invention. The effect of MafB and cMaf deletion can be observed in all cell types where LyzM is expressed, in particular in myeloid cells such as granulocytes, monocytes and macrophages and dendritic cells would delete MafB and cMaf genes.

The role of Maf-DKO macrophages in the development of lung fibrosis over the course of 14 days (peak of fibrotic disease) was investigated in the current gold standard mouse model of bleomycin induced pulmonary fibrosis (IPF). Interestingly, reduced pulmonary fibrosis in the Maf-DKO mice was observed.

To understand the macrophage heterogeneity and their interactions with other cell types in the context of pulmonary fibrosis, whole lung scRNAseq on WT and Maf-DKO mice treated with bleomycin or PBS were performed. The observed reduced pulmonary fibrosis in the Maf-DKO mice was strongly associated with the striking reduction of profibrotic signature genes in monocyte derived Maf-DKO macrophages. Maf-DKO recruited macrophages were thus resistant to the transition into a profibrotic macrophage.

Several recent studies have shown that recruited macrophages drive fibrosis progression by upregulating several known fibrotic genes. It has been proposed that the cross-talk of macrophages with fibroblasts and myofibroblasts in the fibrotic niche mutually induce and maintain their respective activated states by paracrine signaling via M-CSF and PDGF pathways. Maf-DKO recruited macrophages failed to induce PDGF in the fibrotic lung. They were thus resistant to the transition into a profibrotic macrophage and disrupted this paracrine activation circuit.

Accordingly, Maf-DKO recruited macrophages are resistant to profibrotic reprogramming typically seen in WT recruited macrophages.

It is further shown by the invention that intravenous cell transplantation of Maf-DKO monocyte derived macrophage alleviates bleomycin induced pulmonary fibrosis in WT mice.

It is further shown by the invention that Maf-DKO monocyte derived macrophages are resistant to the induction of profibrotic gene signatures and surface markers after exposure to M-CSF.

In order to assess the feasibility of Maf-DKO monocyte cell therapy beyond lung fibrosis, profibrotic gene signatures not only from the similar bleomycin models in mice lungs but also from the literature of recent publications that employ scRNAseq in mouse and human lung fibrosis as well as Covid-19 associated fibrosis, liver fibrosis and fibrotic cancers, were collected and evaluated. These data show that Maf-DKO macrophages fail to upregulate general fibrotic gene signatures across all compared reference datasets. Therefore, it is proposed by the invention that Maf-DKO monocytes and monocyte derived macrophages have a general utility and therapeutic relevance beyond the bleomycin model of pulmonary fibrosis.

The invention further evidences that human Maf-DKO macrophages are resistant to the induction of a profibrotic gene signature in response to M-CSF.

The invention further evidences that human Maf-DKO macrophages that are derived from iPS cells are resistant to the induction of a profibrotic gene signature in response to M-CSF.

Therefore, it is proposed by the invention that Maf-DKO monocytes, monocyte derived macrophages and macrophages derived from iPS cells have a general utility and therapeutic relevance for pulmonary fibrosis, liver fibrosis and fibrotic cancers, e.g. pancreatic cancer

### FIGURES

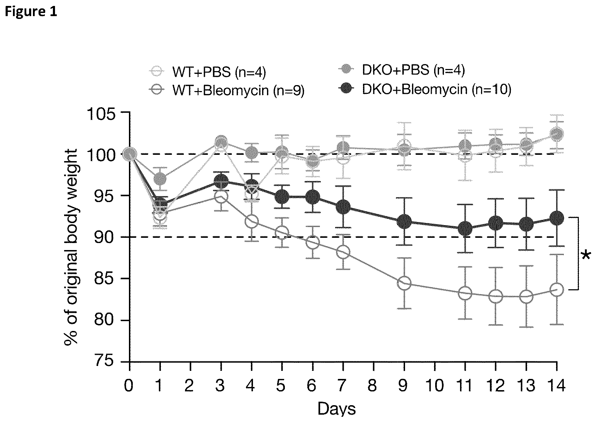

- **Figure 1**: Shows that myeloid Maf-DKO mice are less susceptible to bleomycin induced lung fibrosis, as evidenced by reduced susceptibility to body weight loss. Mice that lost more than 10% of their original body weight at Day14 were defined as severely affected by the disease. This group of severely diseased animals represented 78% of WT mice versus only 30% of myeloid Maf-DKO mice. (2-way annova with gisser-greenhouse correction, * indicated a p-value of 0.0185)

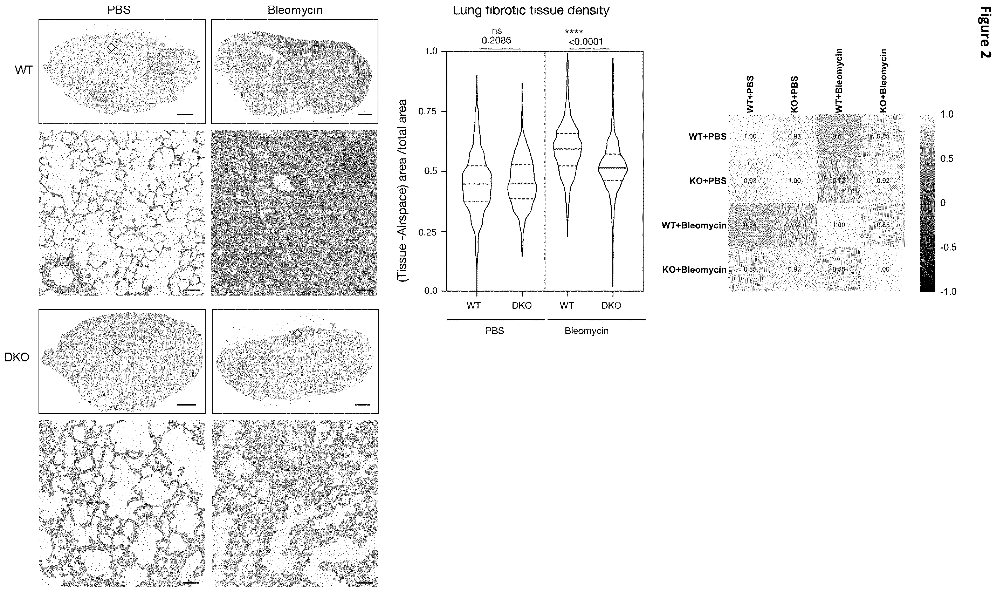

- **Figure 2**: Shows examples of histological images of WT and myeloid Maf-DKO lungs stained with hematoxylin-eosin showing dramatic reduction in fibrotic areas in Maf-DKO lungs. Whole lung section scan images were quantified using image analysis tools to detect tissue area and airspace areas. Violin plots show quantification of tissue density in the lung as a measure of severity of fibrosis, where myeloid Maf-DKO lungs show reduction in tissue density. The tissue density correlation plots show that Maf-DKO lungs correlate well with the control conditions not treated with bleomycin.

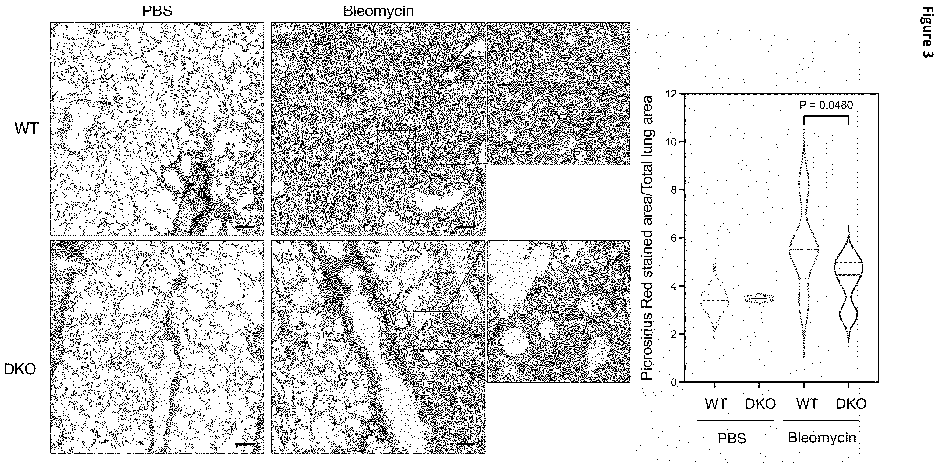

- **Figure 3**: Shows examples of histological images of WT and myeloid Maf-DKO lungs stained with picrosirius red (collagen content) showing dramatic reduction in collagen deposition in myeloid Maf-DKO lungs. Whole lung section scan images were quantified using image analysis tools to detect collagen stained areas. Violin plots show quantification of collagen stained area fraction per area of lung section as a measure of severity of fibrosis, where myeloid Maf-DKO show reduction in collagen deposition.

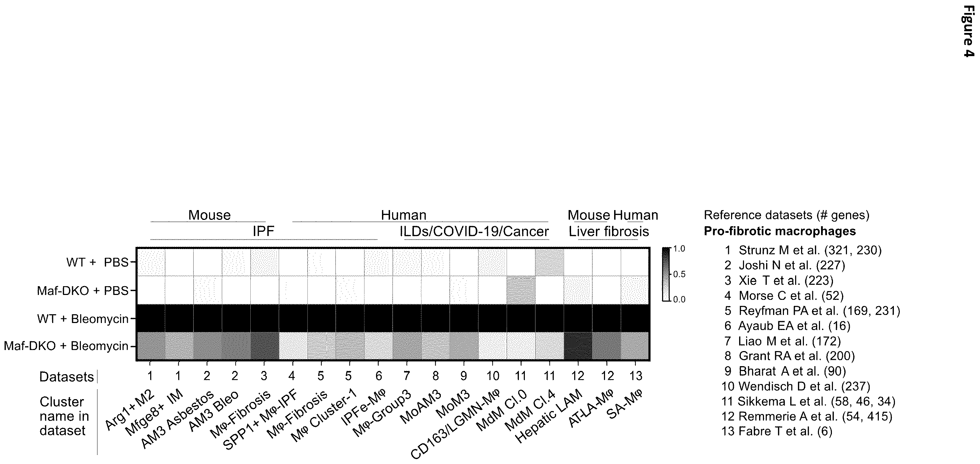

- **Figure 4:**: Shows that Maf-DKO monocyte derived macrophages in the lung of bleomycin treated mice fail to induce profibrotic gene expression signatures. Monocyte derived macrophages were defined by scRNAseq analysis of whole lung and heatmap visualization shows enrichment for mouse and human profibrotic macrophage gene expression signatures in WT or Maf-DKO monocyte derived macrophages in the lung of mice 14 days after vehicle (PBS) or bleomycin treatment. Profibrotic gene expression signatures are based on indicated reference datasets of monocyte and macrophages in mouse and human lung fibrosis selected from idiopathic, Covid-19 and cancer associated lung fibrosis as well as liver fibrosis. Name of the profibrotic macrophage cluster in the reference dataset is indicated where appropriate and the number of profibrotic genes per cluster are indicated in parentheses.

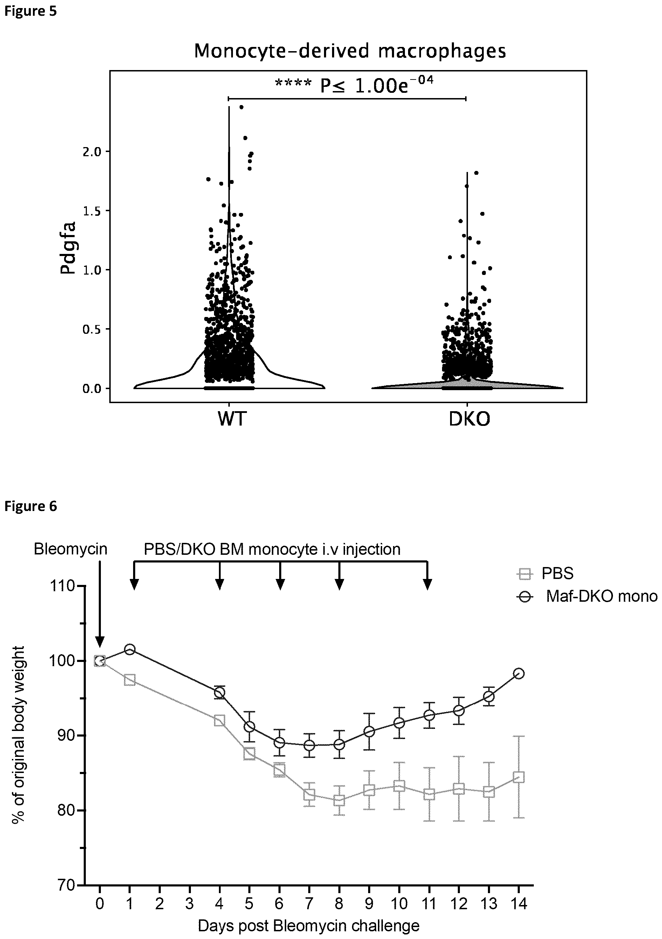

- **Figure 5:**: Shows loss of PDGFA gene expression in individual Maf-DKO and WT monocyte derived macrophages by violin plot quantification of scRNAseq data from bleomycin treated mouse lungs. PDGFA is a macrophage secreted growth factor that substantially contributes to fibrosis progression through myofibroblast activation.
- **Figure 6:**: Shows that Maf-DKO monocyte transplantation alleviates bleomycin induced lung fibrosis. Percent of body weight loss is shown as indication of morbidity in bleomycin treated control mice (PBS) or after multiple i.v. transplantations of 1x 10^6 Maf-DKO monocytes post bleomycin treatment as indicated.

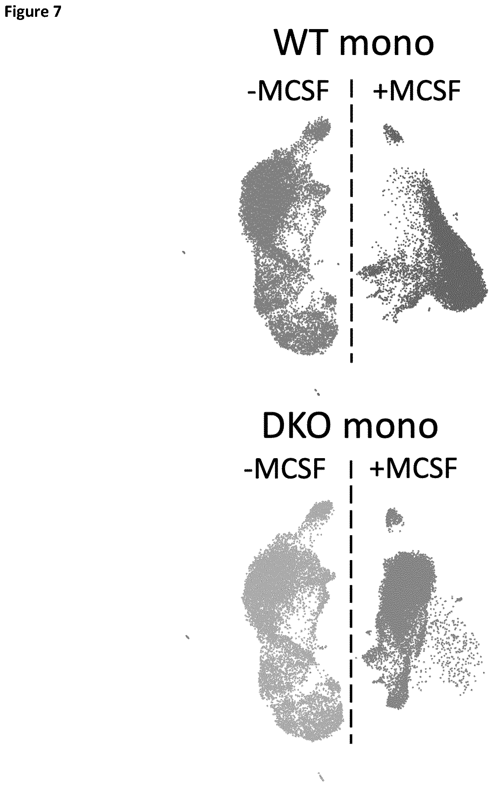

- **Figure 7:**: Shows a UMAP visualization of single-cell RNAseq data of murine WT and Maf-DKO monocyte derived macrophages 24h after stimulation with M-CSF. WT and Maf-DKO monocytes appear transcriptionally highly similar before MCSF stimulation, (-M-CSF, on the left) but show a highly different gene expression 24 hours after stimulation with M-CSF (+M-CSF right).

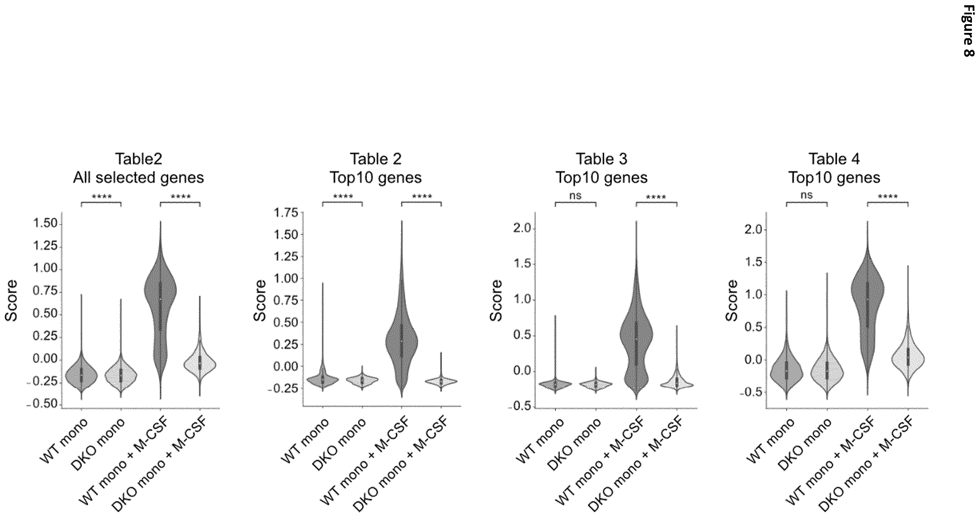

- **Figure 8:**: Shows quantitative violin plots of profibrotic gene expression signatures of total selected genes and top 10 genes from Table 2, 3 and 4 in single-cell RNAseq data of M-CSF stimulated murine WT and Maf-DKO monocyte derived macrophages. In contrast to WT monocyte derived macrophages Maf-DKO monocyte derived macrophages fail to induce profibrotic gene expression signatures (wilcoxson test, **** indicates a P-value of <2e-16).

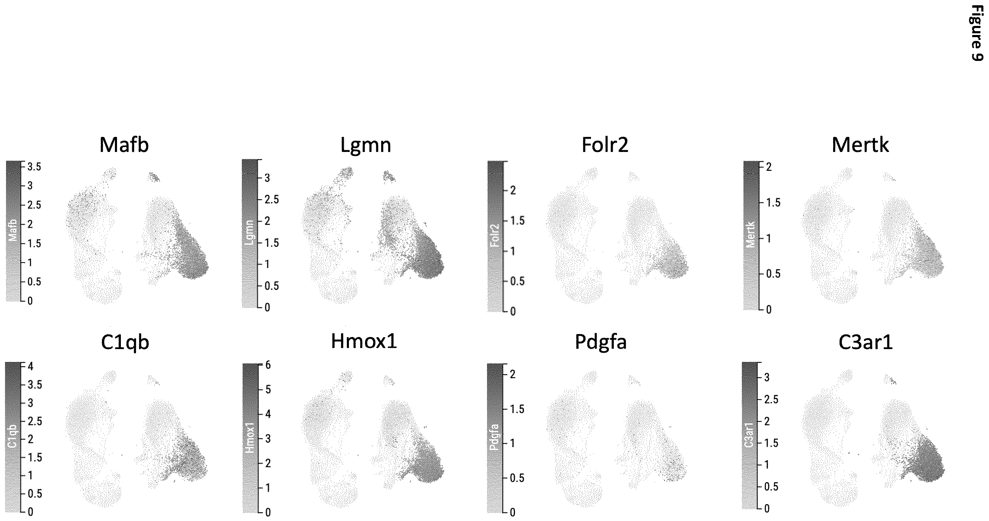

- **Figure 9**: Shows a UMAP visualization of single-cell RNAseq data with expression levels of selected profibrotic genes induced by M-CSF in murine WT monocyte derived macrophages (Table 2) but not in Maf-DKO monocyte derived macrophages.

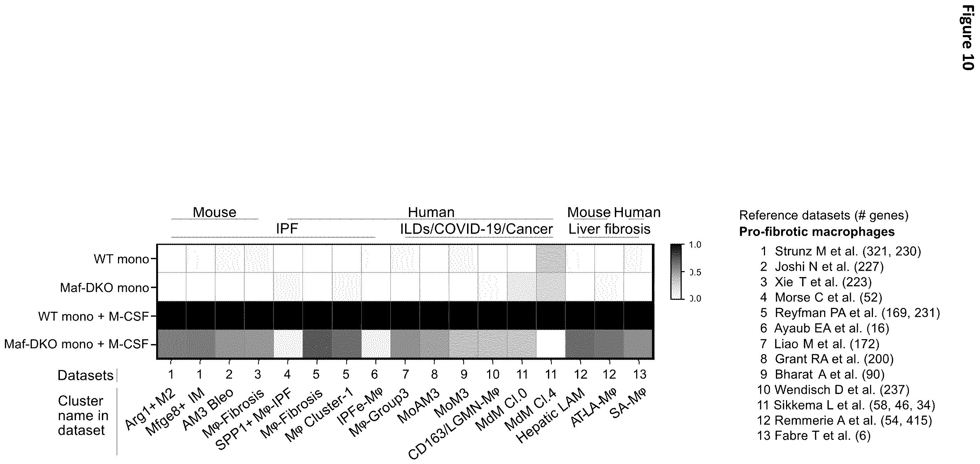

- **Figure 10**: Shows a heatmap visualization for the expression of mouse and human profibrotic macrophage gene signatures from recent literature of scRNAseq reference datasets of mouse and human lung fibrosis as well as Covid-19 associated fibrosis, liver fibrosis and fibrotic cancers indicating a reduced profibrotic gene signature in Maf-DKO monocyte derived macrophagess 24 hours after M-CSF stimulation across all the reference datasets tested. The name of the profibrotic macrophage cluster in the reference dataset is indicated column-wise and the number of profibrotic genes used to describe the profibrotic macrophage cluster in the references are indicated in closed parenthesis.

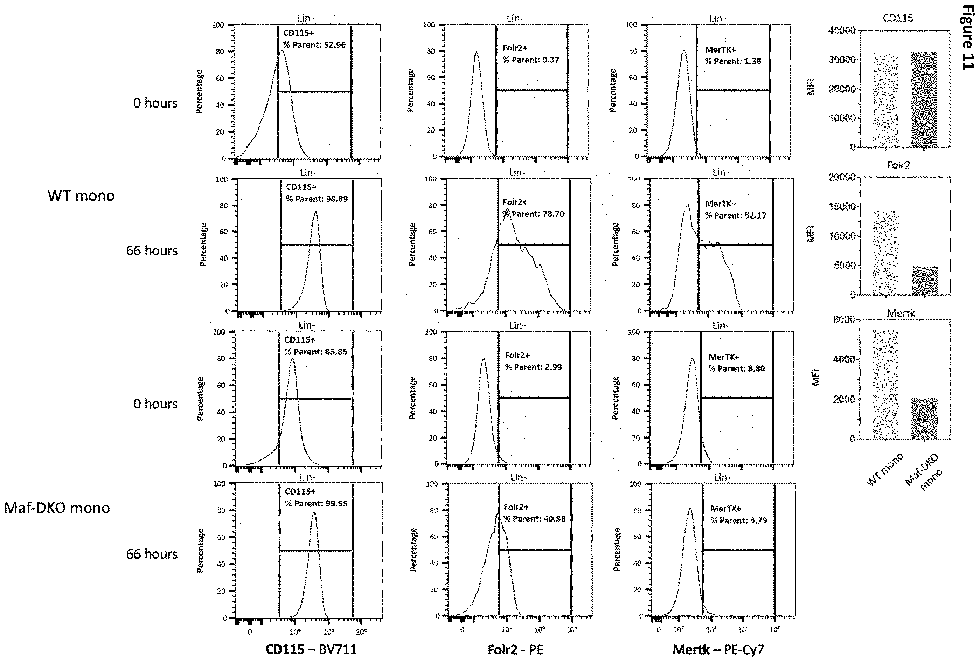

- **Figure 11**: Shows flow cytometric histograms and quantitative bar plots of MFI for cell surface expression of M-CSF induced profibrotic Mertk and Folr2 markers in WT and Maf-DKO murine monocyte before and in monocyte derived macrophages 66 hours after M-CSF stimulation. Mertk and Folr2 showed increased expression in WT in monocyte derived macrophages 66h after M-CSF stimulation compared to Maf-DKO monocyte derived macrophages, whereas expression of the monocyte/macrophage marker M-CSFR (CD115) showed no difference between WT and Maf-DKOc ells.

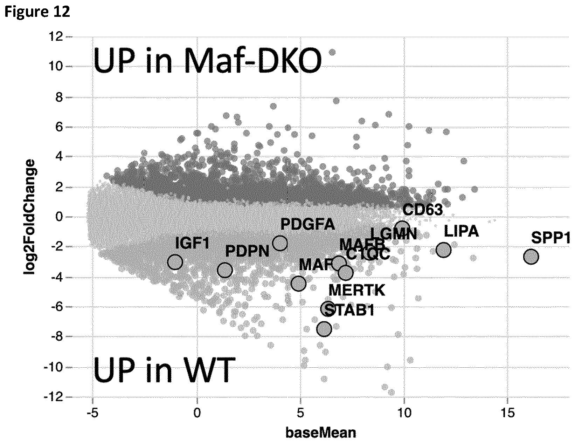

- **Figure 12**: Shows gene expression of selected profibrotic genes from Table 2 in RNAseq data from HUMAN MAF-DKO vs WT iPSC derived macrophages after 2-hour stimulation with M-CSF containing medium. Figure 12 provides evidence that human MAF-DKO macrophages also fail to induce key profibrotic genes in response to M-CSF.

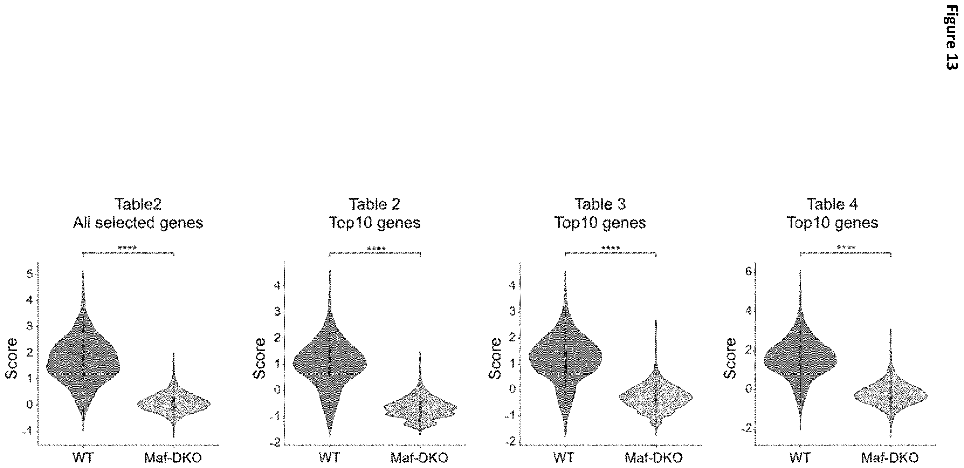

- **Figure 13**: Shows quantitative violin plots of profibrotic gene expression of total selected genes and top 10 genes in Table 2, 3 and 4 in single cell RNAseq data from human iPSC derived macrophages after culture in M-CSF containing medium. Figure 12 provides single cell evidence that HUMAN Maf-DKO macrophages fail to express profibrotic gene signatures in response to M-CSF (wilcoxson test, **** indicates a P-value of <2e-16).

### REFERENCES

Adler M, Mayo A, Zhou X, et al. Principles of Cell Circuits for Tissue Repair and Fibrosis. iScience. 2020;23(2):100841. doi:10.1016/j.isci.2020.100841
Alsinet C, Primo MN, Lorenzi V, Bello E, Kelava I, Jones CP, Vilarrasa-Blasi R, Sancho-Serra C, Knights AJ, Park JE, Wyspianska BS, Trynka G, Tough DF, Bassett A, Gaffney DJ, Alvarez-Errico D, Vento-Tormo R. Robust temporal map of human in vitro myelopoiesis using single-cell genomics. Nat Commun. 2022 May 24;13(1):2885. doi: 10.1038/s41467-022-30557-4. PMID: 35610203; PMCID: PMC9130280.
Aran D, Looney AP, Liu L, et al. Reference-based analysis of lung single-cell sequencing reveals a transitional profibrotic macrophage. Nat Immunol. 2019;20(2):163-172. doi:10.1038/s41590-018-0276-y
Ayaub EA, Poli S, Ng J, et al. Single Cell RNA-seq and Mass Cytometry Reveals a Novel and a Targetable Population of Macrophages in Idiopathic Pulmonary Fibrosis. bioRxiv 2021.01.04.425268; doi: https://doi.org/10.1101/2021.01.04.425268
Aziz A, Soucie E, Sarrazin S, Sieweke MH. MafB/c-Maf deficiency enables self-renewal of differentiated functional macrophages. Science. 2009;326(5954):867-871. doi:10.1126/science.1176056
Bakri Y, Sarrazin S, Mayer UP, et al. Balance of MafB and PU.1 specifies alternative macrophage or dendritic cell fate. Blood. 2005;105(7):2707-2716. doi:10.1182/blood-2004-04-1448
Buchrieser J, James W, Moore MD. Human Induced Pluripotent Stem Cell-Derived Macrophages Share Ontogeny with MYB-Independent Tissue-Resident Macrophages. Stem Cell Reports. 2017;8(2):334-345. doi:10.1016/j.stemcr.2016.12.020
Bharat A, Querrey M, Markov NS, et al. Lung transplantation for patients with severe COVID-19. Sci Transl Med. 2020;12(574):eabe4282. doi:10.1126/scitranslmed.abe4282
Buechler MB, Fu W, Turley SJ. Fibroblast-macrophage reciprocal interactions in health, fibrosis, and cancer. Immunity. 2021;54(5):903-915. doi:10.1016/j.immuni.2021.04.021
Chu VT, Graf R, Wirtz T, et al. Efficient CRISPR-mediated mutagenesis in primary immune cells using CrispRGold and a C57BL/6 Cas9 transgenic mouse line. Proc Natl Acad Sci USA. 2016;113(44):12514-12519. doi:10.1073/pnas.1613884113
Clausen BE, Burkhardt C, Reith W, Renkawitz R, Förster I. Conditional gene targeting in macrophages and granulocytes using LysMcre mice. Transgenic Res. 1999;8(4):265-277. doi:10.1023/a:1008942828960
Cordes SP, Barsh GS. The mouse segmentation gene kr encodes a novel basic domain-leucine zipper transcription factor. Cell. 1994;79(6):1025-1034. doi:10.1016/0092-8674(94)90033-7
DeLuca DS, Levin JZ, Sivachenko A, et al. RNA-SeQC: RNA-seq metrics for quality control and process optimization. Bioinformatics. 2012;28(11):1530-1532. doi:10.1093/bioinformatics/bts196
Eichmann A, Grapin-Botton A, Kelly L, Graf T, Le Douarin NM, Sieweke M. The expression pattern of the mafB/kr gene in birds and mice reveals that the kreisler phenotype does not represent a null mutant. Mech Dev. 1997;65(1-2):111-122. doi:10.1016/s0925-4773(97)00063-4
Fabre T, Barron AMS, Christensen SM, et al. Identification of a broadly fibrogenic macrophage subset induced by type 3 inflammation. Sci Immunol. 2023;8(82):eadd8945. doi:10.1126/sciimmunol.add8945
Freund EC, Lock JY, Oh J, et al. Efficient gene knockout in primary human and murine myeloid cells by non-viral delivery of CRISPR-Cas9. J Exp Med. 2020;217(7):e20191692. doi:10.1084/jem.20191692
Fusaki N, Ban H, Nishiyama A, Saeki K, Hasegawa M. Efficient induction of transgene-free human pluripotent stem cells using a vector based on Sendai virus, an RNA virus that does not integrate into the host genome. Proc Jpn Acad Ser B Phys Biol Sci. 2009;85(8):348-362. doi:10.2183/pjab.85.348
Grant RA, Morales-Nebreda L, Markov NS, et al. Circuits between infected macrophages and T cells in SARS-CoV-2 pneumonia. Nature. 2021;590(7847):635-641. doi:10.1038/s41586-020-03148-w
Gemelli C, Montanari M, Tenedini E, et al. Virally mediated MafB transduction induces the monocyte commitment of human CD34+ hematopoietic stem/progenitor cells. Cell Death Differ. 2006;13(10):1686-1696. doi:10.1038/sj.cdd.4401860
Global burden of 369 diseases and injuries in 204 countries and territories, 1990-2019: a systematic analysis for the Global Burden of Disease Study 2019. Lancet. 396, 1204-1222 (2020
González F, Zhu Z, Shi ZD, et al. An iCRISPR platform for rapid, multiplexable, and inducible genome editing in human pluripotent stem cells. Cell Stem Cell. 2014;15(2):215-226. doi:10.1016/j.stem.2014.05.018
Hegde SP, Zhao J, Ashmun RA, Shapiro LH. c-Maf induces monocytic differentiation and apoptosis in bipotent myeloid progenitors. Blood. 1999;94(5):1578-1589.
Ho IC, Hodge MR, Rooney JW, Glimcher LH. The proto-oncogene c-maf is responsible for tissue-specific expression of interleukin-4. Cell. 1996;85(7):973-983. doi:10.1016/s0092-8674(00)81299-4
Howe KL, Achuthan P, Allen J, et al. Ensembl 2021. Nucleic Acids Res. 2021;49(D1):D884-D891. doi:10.1093/nar/gkaa942
Ignatiadis N and Huber W. Covariate Powered Cross-Weighted Multiple Testing. Journal of the Royal Statistical Society Series B: Statistical Methodology. Volume 83, Issue 4, September 2021, Pages 720-751, https://doi.org/10.1111/rssb.12411
Ignatiadis N, Klaus B, Zaugg JB, Huber W. Data-driven hypothesis weighting increases detection power in genome-scale multiple testing. Nat Methods. 2016;13(7):577-580. doi:10.1038/nmeth.3885
Isogai S, Yamamoto N, Hiramatsu N, et al. Preparation of Induced Pluripotent Stem Cells Using Human Peripheral Blood Monocytes. Cell Reprogram. 2018;20(6):347-355. doi:10.1089/cell.2018.0024
Joshi N, Watanabe S, Verma R, et al. A spatially restricted fibrotic niche in pulmonary fibrosis is sustained by M-CSF/M-CSFR signaling in monocyte-derived alveolar macrophages. Eur Respir J. 2020;55(1):1900646. Published 2020 Jan 16. doi:10.1183/13993003.00646-2019
Karpinski J, Hauber I, Chemnitz J, et al. Directed evolution of a recombinase that excises the provirus of most HIV-1 primary isolates with high specificity. Nat Biotechnol. 2016;34(4):401-409. doi:10.1038/nbt.3467
Kataoka K, Noda M, Nishizawa M. Maf nuclear oncoprotein recognizes sequences related to an AP-1 site and forms heterodimers with both Fos and Jun. Mol Cell Biol. 1994;14(1):700-712. doi:10.1128/mcb.14.1.700-712.1994
Kelly LM, Englmeier U, Lafon I, Sieweke MH, Graf T. MafB is an inducer of monocytic differentiation. EMBOJ. 2000;19(9):1987-1997. doi:10.1093/emboj/19.9.1987
Kerppola TK, Curran T. Maf and Nrl can bind to AP-1 sites and form heterodimers with Fos and Jun. Oncogene. 1994;9(3):675-684.
Kim JI, Ho IC, Grusby MJ, Glimcher LH. The transcription factor c-Maf controls the production of interleukin-4 but not other Th2 cytokines. Immunity. 1999;10(6):745-751. doi:10.1016/s1074-7613(00)80073-4
Lansing F, Paszkowski-Rogacz M, Schmitt LT, et al. A heterodimer of evolved designer-recombinases precisely excises a human genomic DNA locus. Nucleic Acids Res. 2020;48(1):472-485. doi:10.1093/nar/gkz1078
Liao M, Liu Y, Yuan J, et al. Single-cell landscape of bronchoalveolar immune cells in patients with COVID-19. Nat Med. 2020;26(6):842-844. doi:10.1038/s41591-020-0901-9
Liao Y, Smyth GK, Shi W. featureCounts: an efficient general purpose program for assigning sequence reads to genomic features. Bioinformatics. 2014;30(7):923-930. doi:10.1093/bioinformatics/btt656
Love MI, Huber W, Anders S. Moderated estimation of fold change and dispersion for RNA-seq data with DESeq2. Genome Biol. 2014;15(12):550. doi:10.1186/s13059-014-0550-8
Matcovitch-Natan O, Winter DR, Giladi A, et al. Microglia development follows a stepwise program to regulate brain homeostasis. Science. 2016;353(6301):aad8670. doi:10.1126/science.aad8670
Morse C, Tabib T, Sembrat J, et al. Proliferating SPP1/MERTK-expressing macrophages in idiopathic pulmonary fibrosis. Eur Respir J. 2019;54(2):1802441. Published 2019 Aug 22. doi:10.1183/13993003.02441-2018
Perrot CY, Karampitsakos T, Herazo-Maya JD. Monocytes and macrophages: emerging mechanisms and novel therapeutic targets in pulmonary fibrosis. Am J Physiol Cell Physiol. 2023;325(4):C1046-C1057. doi:10.1152/ajpcell.00302.2023
Picelli S, Björklund ÅK, Faridani OR, Sagasser S, Winberg G, Sandberg R. Smart-seq2 for sensitive full-length transcriptome profiling in single cells. Nat Methods. 2013;10(11):1096-1098. doi:10.1038/nmeth.2639
Pollard JW. Tumour-educated macrophages promote tumour progression and metastasis. Nat Rev Cancer. 2004;4(1):71-78. doi:10.1038/nrc1256
Qian BZ, Pollard JW. Macrophage diversity enhances tumor progression and metastasis. Cell. 2010;141(1):39-51. doi:10.1016/j.cell.2010.03.014
Remmerie A, Martens L, Thoné T, et al. Osteopontin Expression Identifies a Subset of Recruited Macrophages Distinct from Kupffer Cells in the Fatty Liver. Immunity. 2020;53(3):641-657.e14. doi:10.1016/j.immuni.2020.08.004
Reyfman PA, Walter JM, Joshi N, et al. Single-Cell Transcriptomic Analysis of Human Lung Provides Insights into the Pathobiology of Pulmonary Fibrosis. Am J Respir Crit Care Med. 2019;199(12):1517-1536. doi:10.1164/rccm.201712-2410OC
Scharenberg SG, Poletto E, Lucot KL, et al. Engineering monocyte/macrophage-specific glucocerebrosidase expression in human hematopoietic stem cells using genome editing [published correction appears in Nat Commun. 2020 Aug 20;11(1):4231. doi: 10.1038/s41467-020-18044-0. Abstract corrected]. Nat Commun. 2020;11(1):3327. Published 2020 Jul 3. doi:10.1038/s41467-020-17148-x
Shi J, Ma Y, Zhu J, et al. A Review on Electroporation-Based Intracellular Delivery. Molecules. 2018;23(11):3044. Published 2018 Nov 21. doi:10.3390/molecules23113044
Sikkema, L., Ramírez-Suástegui, C., Strobl, D.C. et al. An integrated cell atlas of the lung in health and disease. Nat Med 29, 1563-1577 (2023). https://doi.org/10.1038/s41591-023-02327-2
Soucie EL, Weng Z, Geirsdóttir L, et al. Lineage-specific enhancers activate self-renewal genes in macrophages and embryonic stem cells. Science. 2016;351(6274):aad5510. doi: 10. 1126/science.aad5510
Starkey Lewis PJ, Moroni F, Forbes SJ. Macrophages as a Cell-Based Therapy for Liver Disease. Semin Liver Dis. 2019;39(4):442-451. doi:10.1055/s-0039-1688502
Strunz M, Simon LM, Ansari M, et al. Alveolar regeneration through a Krt8+ transitional stem cell state that persists in human lung fibrosis. Nat Commun. 2020;11(1):3559. Published 2020 Jul 16. doi:10.1038/s41467-020-17358-3
Tillmanns S, Otto C, Jaffray E, et al. SUMO modification regulates MafB-driven macrophage differentiation by enabling Myb-dependent transcriptional repression. Mol Cell Biol. 2007;27(15):5554-5564. doi:10.1128/MCB.01811-06
Tran S, Baba I, Poupel L, et al. Impaired Kupffer Cell Self-Renewal Alters the Liver Response to Lipid Overload during Non-alcoholic Steatohepatitis. Immunity. 2020;53(3):627-640.e5. doi:10.1016/j.immuni.2020.06.003
Wang B, Zuo J, Kang W, et al. Generation of Hutat2:Fc Knockin Primary Human Monocytes Using CRISPR/Cas9. Mol Ther Nucleic Acids. 2018;11:130-141. doi:10.1016/j.omtn.2018.01.012
Wende H, Lechner SG, Cheret C, et al. The transcription factor c-Maf controls touch receptor development and function. Science. 2012;335(6074):1373-1376. doi:10.1126/science.1214314
Wendisch D, Dietrich O, Mari T, et al. SARS-CoV-2 infection triggers profibrotic macrophage responses and lung fibrosis. Cell. 2021;184(26):6243-6261.e27. doi:10.1016/j.cell.2021.11.033
Wu TD, Watanabe CK. GMAP: a genomic mapping and alignment program for mRNA and EST sequences. Bioinformatics. 2005;21(9):1859-1875. doi:10.1093/bioinformatics/bti310
Wu TD, Nacu S. Fast and SNP-tolerant detection of complex variants and splicing in short reads. Bioinformatics. 2010;26(7):873-881. doi:10.1093/bioinformatics/btq057
Xie T, Wang Y, Deng N, et al. Single-Cell Deconvolution of Fibroblast Heterogeneity in Mouse Pulmonary Fibrosis. Cell Rep. 2018;22(13):3625-3640. doi:10.1016/j.celrep.2018.03.010
Yoshida T, Ohkumo T, Ishibashi S, Yasuda K. The 5'-AT-rich half-site of Maf recognition element: a functional target for bZIP transcription factor Maf. Nucleic Acids Res. 2005;33(11):3465-3478. Published 2005 Jun 21. doi:10.1093/nar/gki653
Yumlu S, Stumm J, Bashir S, et al. Gene editing and clonal isolation of human induced pluripotent stem cells using CRISPR/Cas9. Methods. 2017;121-122:29-44. doi:10.1016/j.ymeth.2017.05.009
Zhang S, Shrestha CL, Wisniewski BL, et al. Consequences of CRISPR-Cas9-Mediated CFTR Knockout in Human Macrophages. Front Immunol. 2020;11:1871. Published 2020 Aug 18. doi:10.3389/fimmu.2020.01871
Zhao, X. et al. Targeting metabolic dysregulation for fibrosis therapy. Not. Rev. Drug Disco. 19, 57-75 (2020)
Zhou X, Franklin RA, Adler M, et al. Circuit Design Features of a Stable Two-Cell System. Cell. 2018;172(4):744-757.e17. doi:10.1016/j.cell.2018.01.015

### DETAILED DESCRIPTION OF THE INVENTION

### Definitions:

A "chromosome" as used herein is one of the 46 regular human chromosomes. A "gene located on a chromosome" is a gene that is not extrachromosomal.

The term "gene" means a DNA sequence that codes for an RNA or a particular sequence of amino acids which comprise all or part of one or more proteins or enzymes, and may or may not include regulatory DNA sequences, such as promoter sequences, which determine for example the conditions under which the gene is expressed. A "promoter" or "promoter sequence" is a DNA regulatory region capable of binding RNA polymerase in a cell and initiating transcription of a downstream (3' direction) coding sequence. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

Within the context of the present invention the terms "mutant" and "mutation" mean a detectable change in genetic material, i.e. genomic DNA. Mutations include deletion, insertion or substitution of one or more nucleotides. The mutation may occur in the coding region of a gene (i.e. in exons), in introns, or in the regulatory regions (e.g. enhancers, response elements, suppressors, signal sequences, polyadenylation sequences, promoters) of the gene. Generally, a mutation is identified in a subject by comparing the sequence of a nucleic acid or polypeptide expressed by said subject with the corresponding nucleic acid or polypeptide expressed in a control population. Where the mutation is within the gene coding sequence, the mutation may be a "missense" mutation, where it replaces one amino acid with another in the gene product, or a "nonsense" mutation, where it replaces an amino acid codon with a stop codon. A mutation may also occur in a splicing site where it creates or destroys signals for exon-intron splicing and thereby lead to a gene product of altered structure. Within the context of the present invention a mutation is not silent, i.e. it results at least in an alteration of the nucleotide sequence (where the gene product is a functional RNA) or an amino acid sequence (where the gene product is a protein) that renders the gene product non-functional or that reduces expression of the gene product at the RNA-level by at least 80%. Preferred mutations, for example deletions of whole genes, abolish gene expression.

As used herein gene expression is "inhibited" when expression of the gene at the RNA-level is reduced by at least 80% compared to gene expression in the corresponding otherwise identical cell, such as a wildtype cell, as measured by quantitative rt-PCR. Preferably expression of the gene at the RNA-level is reduced by at least 90%, such as by at least 95%.

As used herein gene expression is "abolished" when expression of the gene is not detectable at the RNA-level by q-PCR. In a qPCR an mRNA which is "expressed" and thus present at detectable levels will a) give a sigmoidal fluorescence curve that b) reaches a plateau at least within 38 PCR-cycles, preferably at least within 36 PCR-cycles, and c) produces a PCR-product of the expected length, i.e. corresponding in length to a PCR-product derived from mature mRNA and not from genomic DNA or unprocessed RNA intermediates. Preferably mRNA expression can be confirmed by these three criteria in three repeated qPCR experiments.

"Mutagenesis" as used herein is a laboratory process by which the genetic information of an organism is deliberately changed, resulting in a mutation. Preferred methods for mutagenesis herein are methods based on site-specific endonucleases.

A "site specific endonuclease" as used herein is an enzyme that cleaves a phosphodiester bond within a polynucleotide chain only at a very specific nucleotide sequence in the middle (endo) portion of a double-stranded DNA molecule, which sequence occurs preferably only once within the whole human genome so as to allow specific genetic engineering of a human target cell. Examples of site-specific endonucleases, which are typically used for genetic engineering in human target cells are zinc finger nucleases, TALENs and the CRISPR/Cas9 system.

As used herein the term "expression" may refer to gene expression of a polypeptide or protein, or to gene expression of a polynucleotide, such as mRNAs, miRNAs or IncRNAs, depending on the context. Expression of a polynucleotide may be determined, for example, by measuring the production of RNA transcript levels using methods well known to those skilled in the art. Expression of a protein or polypeptide may be determined, for example, by immunoassay using (an) antibody(ies) that binds the polypeptide specifically, using methods well known to those skilled in the art.

As used herein the "expression of an mRNA" relates to the transcriptional level of gene expression.

In the present invention, known methods can be used to detect such an expression of a gene. Examples of the method for quantitatively detecting an mRNA level in a cell or collection of cells include for example PCR-based methods (real-time PCR, quantitative PCR), and DNA microarray analysis. In addition, an mRNA level can be quantitatively detected by counting the number of reads according to what is called a new generation sequencing method. Exemplary methods, conditions and materials to be used for the determination of the expression level of an mRNA are described in the experimental section of this disclosure. A preferred method for determining expression of an mRNA is qPCR, as explained under "abolished expression" above.

Those skilled in the art can prepare an mRNA or a nucleic acid cDNA to be detected by the aforementioned detection methods by taking the type and state of the specimen and so forth into consideration and selecting a known method appropriate, therefore. When the gene expression level in human macrophages of the invention is compared with the expression level of the same gene in wildtype macrophages, it is compared under otherwise identical conditions, i.e. both types of macrophages are to be cultured and treated in the same manner in order to allow for a scientifically meaningful comparison of mRNA levels.

An "allele" as used herein refers to one of the two copies of the same human gene. The two copies of a gene, one each on the two homologous chromosomes, can be identical or vary slightly in their individual sequences. The term allele is thus used slightly differently from its typical use herein, because it includes identical versions of the same human gene on the same relative place on the two homologous chromosomes. In a diploid cell, the two alleles of a given gene occupy corresponding loci on a pair of homologous chromosomes.

As used herein an "allele" or a "gene" is rendered nonfunctional if no further expression of the protein encoded by the gene or allele is detectable after the procedure that rendered the allele or gene nonfunctional. That is, no new protein is being expressed from an allele or gene that has been rendered nonfunctional. Eventually the protein encoded by an allele or gene that has been rendered nonfunctional will become undetectable in a population of cells consisting of cells with only nonfunctional alleles. The time until the protein encoded by said gene or allele will become undetectable depends on the dynamics of protein and mRNA turnover for said gene.

As used herein the term "deletion" means that a part of a DNA-sequence is missing compared to a wildtype reference sequence.

As used herein an "exon" is any part of a gene that will encode a part of the final mature RNA produced by that gene after introns have been removed by RNA splicing. The term exon refers to both the DNA sequence within a gene and to the corresponding sequence in RNA transcripts. In RNA splicing, introns are removed, and exons are covalently joined to one another as part of generating the mature messenger RNA.

The term "chromosome rearrangement" as used herein is a chromosome abnormality involving a change in the structure of a native chromosome. Usually, chromosome rearrangements are caused by a breakage in the DNA double helices at two different locations, followed by a rejoining of the broken ends to produce a new chromosomal arrangement of genes, different from the gene order of the chromosomes or the chromosomes before it or they were broken. Such changes may involve several different classes of events, like large deletions of more than 10000 base pairs, gene duplications, gene inversions and translocations, within one or between two chromosomes.

The term "karyotyping" as used herein is the analysis of the chromosomes within cells from a sample. Chromosomes are stained and the skilled person then uses a microscope to examine the size, shape, and number of chromosomes in the cells of the cell sample. Usually, the stained sample is photographed to show the arrangement of the chromosomes. Karyotypes describe the chromosome count of an organism and what these chromosomes look like under a light microscope, in particular the length of the chromosomes, the position of the centromeres within those chromosomes, the banding pattern of the stained chromosomes, any differences between the sex chromosomes, and any other physical characteristics.

The term "guide RNA" as used herein relates to "guide RNA" as used in the context of a CRISPR/Cas9 DNA editing system. The guide RNA confers target sequence specificity to the CRISPR-Cas9 system. Guide RNAs are non-coding short RNA sequences which first bind to the Cas9 enzyme and then the guide RNA sequence guides the complex via base pairing to a specific location on the DNA, where Cas9 acts as an endonuclease and cuts the target DNA strand. Examples of guide RNAs are a) a synthetic trans-activating CRISPR RNA (tracrRNA) plus a synthetic CRISPR RNA (crRNA), wherein the crRNA is designed to identify the gene target site of interest, and b) a single guide RNA (sgRNA) that combines both the crRNA and tracrRNA within a single construct.

The term "MAF" denotes the human MAF transcription factor. MAF and other Maf family members form homodimers and heterodimers with each other and with Fos and Jun, consistent with the known ability of the AP-1 proteins to pair with each other (Kerppola and Curran, 1994; Kataoka et al., 1994). The DNA target sequence to which MAF homodimers bind, termed the MAF response element (MARE), is a 13 or 14 bp element which contains a core TRE (T-MARE) or CRE (C-MARE) palindrome respectively, but MAF may also bind to DNA sequences diverging from these consensus sites including composite AP-1/MARE sites and MARE half sites with 5' AT rich extensions (Yoshida et al., 2005). MAF has been shown to stimulate transcription from several promoters, as well as to repress the transcription of other promoters. MAF has also been shown to induce the differentiation of T helper 2 (Th2) cells (Ho et al., 1996) due to its ability to activate the tissue specific transcription of the interleukin-4 (IL-4) (Kim et al., 1999). Furthermore, the over-expression of MAF in myeloid cell lines induces macrophage differentiation (Hegde et al., 1999). The gene for human MAF is located on chromosome 16, location 16q23.2 and is described in detail as Gene ID 4094 in the NCBI Gene database. Sequence and location information refer to the annotation release 109.20201120, which is the current release on December 9, 2020, Reference sequence assembly GCF_000001405.39 of the Genome Reference Consortium Human Build 38 patch release 13. This reference identifies the gene for MAF on the complementary strand between 79,593,838 and 79,600,737.

The term "MAFB" denotes the human MAFB transcription factor. This gene is expressed in a variety of cell types (including lens epithelial, pancreas endocrine, epidermis, chondrocyte, neuronal and hematopoietic cells, in particular macrophages) and encodes a protein containing a typical bZip motif in its carboxy-terminal region. In the bZip domain, MAFB shares extensive homology not only with MAF but also with other Maf-related proteins. MAFB can form a homodimer through its leucine repeat structure and specifically binds Maf-recognition elements (MAREs) palindromes, composite AP-1/MARE sites or MARE halfsites with AT rich 5' extensions (Yoshida et al., 2005). In addition, MAFB can form heterodimers with Maf or Fos through its zipper structure but not with Jun or other Maf family members (Kataoka et al., 1994). MAFB is also known under the name kreisler, kr or Krml1 (for'Kreisler Maf leucine Zipper 1'), because an x-ray induced chromosomic micro-inversion in kreisler mutant mice causes the tissue specific loss of MAFB expression in the developing hindbrain that is responsible for the kreisler phenotype (Cordes et al., 1994; Eichmann et al., 1997). In the hematopoietic system MAFB is expressed selectively in the myeloid lineage and is up-regulated successively during myeloid differentiation from multipotent progenitors to macrophages. Indeed, this induction reflects an important role of MAFB in monocytic and macrophage differentiation. Thus, the overexpression of MAFB in transformed chicken myeloblasts (Kelly et al., 2000, Bakri et al. 2005) and in human hematopoetic progenitors (Gemelli et al., 2006) inhibits progenitor proliferation (Tillmanns et al., 2007) and accelerates the formation of macrophages (Kelly et al., 2000; Bakri et al., 2005; Gemelli et al., 2006), whereas a dominant negative version of MAFB inhibits this process (Kelly et al., 2000). Deletion of MafB in conjunction with Maf in mouse monocytes and macrophages enables extended proliferation (Aziz et al., 2009; Soucie et al., 2016). Together this indicates that MAFB induction is a specific and important determinant of the monocytic program in hematopoietic cells and is important for cell cycle arrest in differentiated monocyte and macrophages.

The gene for human MAFB is located on chromosome 20, location 20q12 and is described in detail as Gene ID 9935 in the NCBI Gene database. Sequence and location information refer to the annotation release 109.20201120, which is the current release on December 9, 2020, Reference sequence assembly GCF_000001405.39 of the Genome Reference Consortium Human Build 38 patch release 13. This reference identifies the gene for MAFB on the complementary strand between 40685848 and 40689236.

M-CSF as used herein, also called CSF1, relates to human colony stimulating factor 1. M-CSF is a cytokine that controls the production, differentiation, proliferation and function of macrophages. Different active isoforms of the protein are found as membrane bound or extracellular disulfide-linked homodimer and are thought to be produced by proteolytic cleavage of membrane-bound precursors.

The gene for human M-CSF is located on chromosome 1, location 1p13.3 and is described in detail as Gene ID 1435 in the NCBI Gene database. Sequence and location information refer to the annotation release 109.20210226. For cell culture experiments recombinant human M-CSF, a 38 kDa homodimer produced in HEK293 cells from Gibco, catalog#PHC9501, was used. ED50 was at most 5 ng/ml as determined by the dose dependent proliferation of M-NSF60 cell.

STAB1, as used herein relates, depending on the context, to the STAB1 gene or to a surface marker, which is the extracellular part of the STAB1 protein, the stabilin1 (also called SCARH2 due to its possible role as a scavenger receptor). STAB1 is described in detail as gene ID 23166 in the NCBI Gene database.

RNF128, as used herein relates, depending on the context, to the RNF128 gene or to the RNF128 protein, an E3 ubiquitin ligase which is also known as GRAIL. RNF128 is described in detail as gene ID 79589 in the NCBI Gene database.

C5AR1, as used herein relates, depending on the context, to complement C5a receptor 1, that enables G protein-coupled receptor activity and complement component C5a receptor activity. C5AR1 is expressed in monocytes and macrophages and involved in complement component C5a signaling pathway. C5AR1 is described in detail as gene ID 728 in the NCBI Gene database.

TMEM37, as used herein relates, depending on the context, to Transmembrane protein 37 which is predicted to enable calcium channel activity and voltage-gated ion channel activity. Its role in regulation of calcium ion channels can influence cellular processes like proliferation and migration indirectly contributing to fibrosis. TMEM37 is described in detail as gene ID 140738 in the NCBI Gene database.

SPP1, as used herein relates, depending on the context, to secreted phosphoprotein 1, also known as Osteopontin, which contributes to fibrosis in multiple organs and is highly expressed in fibrotic tissues especially in macrophage associated diseases like idiopathic pulmonary fibrosis (IPF), liver and kidney fibrosis. Osteopontin contributes to extra cellular matrix remodeling, enhances fibroblast activation and its deletion in mice reduces fibrosis severity in models of lung and skin injuries. SPP1 is described in detail as gene ID 6696 in the NCBI Gene database

RGL1, as used herein relates, depending on the context, to Ral guanine nucleotide dissociation stimulator like 1 and is predicted in regulation of catalytic activity. It is implicated in the activation of Ras signaling pathway and crucial for various cellular functions including cell proliferation, differentiation, adhesion and migration which could be relevant in context of fibrotic responses. RGL1 is described in detail as gene ID 23179 in the NCBI Gene database.

MRC1, as used herein relates, depending on the context, to Mannose Receptor C-type 1 also known as CD206 antigen, a type 1 membrane receptor that mediates endocytosis of glycoproteins by macrophages to promote profibrotic responses thereby contributing to progression of lung and liver fibrosis. MRC1 is described in detail as gene ID 4360 in the NCBI Gene database.

MARCKS, as used herein relates, depending on the context, to myristoylated alanine rich protein kinase C substrate and it plays a role in the structural modulation of the actin cytoskeleton, chemotaxis, motility, cell adhesion, phagocytosis and exocytosis through lipid sequestration or protein docking to membranes. It is therefore involved in a plethora of physiological processes and known to play a significant role in fibrosis by regulating fibroblast migration and adhesion. It is essential for fibroblast motility and influence their ability to move towards damaged tissue areas and in macrophage activation and secretion of inflammatory. MARCKS is described in detail as gene ID 4082 in the NCBI Gene database.

MAFB, as used herein relates, depending on the context, to a basic leucine zipper (bZIP) transcription factor that plays an important role in the regulation of lineage-specific hematopoiesis. MafB plays a role in tissue homeostasis and inflammation and in macrophages it has been shown to negatively regulate macrophage self-renewal capacity, alter macrophage polarization and is implicated in fibrosis due to elevated gene expression in several fibrotic diseases. MAFB is described in detail as gene ID 9935 in the NCBI Gene database.

MAF, as used herein relates, depending on the context, to a basic leucine zipper (bZIP) transcription factor that plays a key role in regulating cell differentiation and proliferation and can have different effector functions depending on its binding partners. Maf has been shown to have redudant functions and could compensate for the loss of Mafb. It has been implicated in fibrosis due to its elevated gene expression in several fibrotic diseases. MAF is described in detail as gene ID 4094 in the NCBI Gene database

LIPA, as used herein relates, depending on the context, to Lipase A, a lysosomal acid lipase. This enzyme functions to catalyze the hydrolysis of cholesteryl esters and triglycerides within lysosomes. Elevated levels of LIPA in monocytes and macrophages has been shown to exacerbate liver fibrosis. LIPA is described in detail as gene ID 3988 in the NCBI Gene database.

LGMN, as used herein relates, depending on the context, to Legumain, a cysteine protease that has a strict specificity for hydrolysis of asparaginyl bonds. It may be involved in the processing of bacterial peptides and endogenous proteins for MHCII presentation in lysosomal and endosomal systems. It is known to be expressed in high levels by tumor associated macrophages in the tumor microenvironment and is associated and has been implicated in pathological fibrosis due to its association with other fibrotic markers in tumors, fibrotic diseases and COVID-19. LGMN is described in detail as gene ID 5641 in the NCBI Gene database.

HMOX1, as used herein relates, depending on the context, to Heme oxygenase 1, an essential enzyme in heme catabolism that cleaves heme to form biliverdin. It is known to play a significant role in IPF due to its elevated expression in lung myofibroblasts indicating its involvement in myofibroblast differentiation via TGFb1 signaling. HMOX1 in macrophages plays critical immunomodulatory effects including macrophage activation that can contribute to fibrotic tissue remodeling. HMOX1 is described in detail as gene ID 3162 in the NCBI Gene database.

DAB2, as used herein relates, depending on the context, to DAB adapter protein 2, a mitogen-responsive phosphoprotein. It is expressed in epithelial cells and plays a role as a tumor suppressor in ovarian cancer cells. It has been implicated in profibrotic macrophage polarization and contributes to tissue repair and fibrotic processes. DAB2 has also been implicated in pulmonary fibrosis and cancer through its interaction with IGF1R and PI3-AKT signaling pathways and in myofibroblast differentiation in response to TGFb1 thereby contributing to fibrosis progression. DAB2 is described in detail as gene ID 1601 in the NCBI Gene database.

CTSD, as used herein relates, depending on the context, to Cathepsin D, a member of A1 family of peptidases is a lysosomal enzyme. It has been linked to fibrosis due its role in extracellular matrix (ECM) remodeling and inflammation. Elevated levels of cathepsin D contributes to fibrogenic processes including chronic kidney disease and pulmonary fibrosis by promoting collagen deposition and influencing myeofibroblast activity. CTSD is described in detail as gene ID 1509 in the NCBI Gene database.

CSTB, as used herein relates, depending on the context, to Cystatin B, an intracellular thiol proteinase inhibitor that reversibly and tightly binds papain, cathepsins L, H and B. Its dysregulation has been associated with increased fibrosis particularly in the liver where higher levels of CSTB can exacerbate fibrogenic responses by inhibiting proteases that would otherwise degrade fibrotic tissue and thereby promoting ECM accumulation. CSTB is described in detail as gene ID 1476 in the NCBI Gene database.

CMKLR1, as used herein relates, depending on the context, to Chemerin chemokine-like receptor 1 that enables adipokinetic hormone binding and receptor activity. It is involved in several processes including macrophage chemotaxis and regulation of calcium mediated signaling. Its expression in monocyte derived macrophages is known to be implicated in lung injury and pulmonary fibrosis where its upregulation correlates with enhanced macrophage migration and activation that contributes to tissue remodeling and fibrogenesis. CMKLR1 is described in detail as gene ID 1240 in the NCBI Gene database.

CD93, as used herein relates, depending on the context, to a cell surface glycoprotein and type I membrane protein and myeloid cell surface marker. It is described to be involved in intercellular adhesion, cytoskeletal remodeling and clearance of apoptotic cells. It plays a significant role in fibrosis particularly through its involvement in modulation of cellular adhesion and extracellular matrix via activation of integrin beta1 and the fibrillogenesis of fibronectin which are critical processes of tumor angiogenesis and fibrotic responses in various diseases. CD93 is described in detail as gene ID 22918 in the NCBI Gene database.

CD63, as used herein relates, depending on the context, to a cell surface protein of the transmembrane 4 superfamily or the tetraspanin family. It mediates signal transduction and is known to play a role in exosome biogenesis. It has been implicated in tumor progression and in fibrosis particularly in the context of macrophage activation and inflammatory responses to influence macrophage polarization and secretion of profibrotic mediators, thereby contributing to fibrotic diseases including lung and liver fibrosis. CD63 is described in detail as gene ID 967 in the NCBI Gene database.

C3AR1, as used herein relates, depending on the context, to complement 3a receptor 1, an anaphylatoxin released during activation of the complement system. It is highly expressed in macrophages and Kupffer cells in the liver and has been implicated in liver disease, including liver fibrosis, NAFLD and NASH. Inflammatory pathways of the C3a/C3aR signaling pathway can exacerbate liver damage and lead to fibrosis progression. C3AR1 is described in detail as gene ID 719 in the NCBI Gene database.

C1QC, as used herein relates, depending on the context, to complement C1q C chain whose collagen-like regions associates with proenzymes C1r and C1s to yield C1, the first component of the serum complement system. Elevated C1q levels have been associated with worsened fibrosis outcomes and correlates to increased Tgfb1 and other markers of fibrotic processes, particularly in the context of pulmonary fibrosis. C1QC is described in detail as gene ID 714 in the NCBI Gene database.

C1QB, as used herein relates, depending on the context, to complement C1q B chain, a paralog of C1QC, whose collagen-like regions associates with proenzymes C1r and C1s to yield C1, the first component of the serum complement system. Elevated C1q levels have been associated with worsened fibrosis outcomes and correlates to increased Tgfb1 and other markers of fibrotic processes particularly in the context of pulmonary fibrosis. C1QB is described in detail as gene ID 713 in the NCBI Gene database.

C1QA, as used herein relates, depending on the context, to complement C1q A chain, a paralog of C1QB, whose collagen-like regions associates with proenzymes C1r and C1s to yield C1, the first component of the serum complement system. Elevated C1q levels have been associated with worsened fibrosis outcomes and correlates to increased Tgfb1 and other markers of fibrotic processes particularly in the context of pulmonary fibrosis. C1QA is described in detail as gene ID 712 in the NCBI Gene database.

PMP22, as used herein relates, depending on the context, to Peripheral myelin protein 22, which is a member of the tetraspanin family and a major component of myelin in the peripheral nervous system. Elevated levels of PMP22 have been shown to regulate macrophage polarization, which is associated to tissue repair and fibrogenesis and exacerbates fibrotic responses in the lung and liver. PMP22 is described in detail as gene ID 5376 in the NCBI Gene database.

PDPN, as used herein relates, depending on the context, to Podoplanin, a type-I or mucin-type integral transmembrane glycoprotein with diverse distribution in human tissues and has been proposed as a marker of lung injury. Increased expression of PDPN is associated with several fibrotic diseases and is thought to contribute to tissue remodeling through its interactions with immune cells and signaling pathways in fibrosis progression. PDPN is described in detail as gene ID 10630 in the NCBI Gene database.

PDGFA, as used herein relates, depending on the context, to platelet derived growth factor subunit A of PDGF and VEGF protein families. It can bind and activate PDGF receptor tyrosine kinases that play a role in a wide range of developmental processes. It plays a critical role in fibrosis acting as the growth factor that stimulates proliferation of fibroblasts, which are essential in the formation of scar tissue. Elevated PDGF signaling is associated with increased myofibroblast activation and extracellular matrix deposition contributing to fibrosis progression in various fibrotic diseases. PDGFA is described in detail as gene ID 5154 in the NCBI Gene database.

MERTK, as used herein relates, depending on the context, to MER proto-oncogene tyrosine kinase transmembrane protein and is a member of the MER/AXL/TYRO3 receptor kinase family. It is expressed in various tissues and involved in critical biological processes such as immune response and phagocytosis by macrophages. MERTK promotes fibrogenesis by enhancing signaling pathways associated with TGFb1 and its inhibition has been shown to prevent and reduce fibrosis across multiple organ systems in animal models. MERTK is described in detail as gene ID 10461 in the NCBI Gene database.

IGF1, as used herein relates, depending on the context, to Insulin-like growth factor 1, which functions similar to insulin and mediates growth and development. It plays a crucial role in fibrosis by promoting macrophage activation and differentiation contributing to the release of profibrotic mediators that can not only contribute to pathological tissue remodeling and fibrosis progression but also facilitate transition of macrophages into myofibroblast-like cells (MMT) via TGFb1-SMAD3 signaling thus exacerbating fibrotic responses in tissues. IGF1 is described in detail as gene ID 3479 in the NCBI Gene database.

IER3, as used herein relates, depending on the context, to Immediate early response 3 which protects cells from Fas- or TNFa-induced apoptosis. It plays a complex role in fibrosis, particularly in fibroblast activation and survival during tissue repair and influences fibroblast survival in fibrotic diseases like IPF and systemic sclerosis. Elevated levels of IER3 in macrophages have been observed in IPF and could modulate macrophage activation and polarization to contribute to the fibrotic process. IER3 is described in detail as gene ID 8870 in the NCBI Gene database.

FOLR2, as used herein relates, depending on the context, to Folate receptor beta, a member of the FOLR family with high affinity for folic acid and several folic acid derivatives to deliver 5-methyltetrahydrofolate to the interior of the cells. It is expressed in various tissues predominantly in activated macrophages and plays a significant role in immunomodulation during fibrosis linked to enhanced fibrosis in chronic kidney disease where folr2 expressing macrophages interact with fibroblasts to promote extracellular matrix remodeling and fibrosis progression. FOLR2 is described in detail as gene ID 2350 in the NCBI Gene database.

ETS2, as used herein relates, depending on the context, to ETS proto-oncogene 2, a transcription factor which regulates genes involved in development, apoptosis and regulation of telomerase. It plays a significant role in fibrosis by promoting epithelial-to-mesenchymal transition (EMT), a key process in fibrosis, and can influence the expression of various markers of fibrosis including collagen in lung and renal fibrosis. ETS2 expression in macrophages and its phosphorylation has been shown to drive persistent expression of inflammatory genes and extracellular matrix components linking it to fibrotic processes in the liver. ETS2 is described in detail as gene ID 2114 in the NCBI Gene database.

CD109, as used herein relates, depending on the context, to a glycosyl phosphatidylinositol (GPI) - linked glycoprotein that localizes to the surface of platelets, activated T-cells, macrophages and endothelial cells. It plays a crucial role as a co-receptor for TGF-beta, which is a key factor in the development of fibrosis. in IPF models, CD109 is expressed in basal cells and several immune cells including macrophages and elevated expression of CD109 has been observed in fibrotic conditions like systemic sclerosis and lung fibrosis, where it influences ECM production and the severity of fibrosis. CD109 is described in detail as gene ID 135228 in the NCBI Gene database.

C5AR2, as used herein relates, depending on the context, to complement C5a receptor 2, a G-protein coupled receptor 1 family member and paralog of C5AR1, is involved in the complement system and innate immune responses. Unlike other classical G-protein coupled receptors, it instead modulates signal transduction via beta-arrestin pathway and may act as a decoy receptor. It acts as a receptor for chemotactic and inflammatory C3a, C4a and C5a anaphylatoxin peptides and it plays a significant role in modulating inflammatory responses associated with fibrosis. C5AR2 can inhibit cytokine release by macrophages, thereby influencing their activation and potentially exacerbating fibrosis in renal fibrosis and atherosclerosis. C5AR2 is described in detail as gene ID 27202 in the NCBI Gene database.

ACP2, as used herein relates, depending on the context, to acid phosphatases 2, a lysosomal acid phosphatase that hydrolyzes orthophosphoric monoesters to alcohol and phosphate. It has been implicated in pathogenesis of fibrosis via modulating macrophage polarization, which is associated with profibrotic mediators leading to enhanced collagen production and myofibroblast differentiation. ACP2 is described in detail as gene ID 53 in the NCBI Gene database.

As used herein a negative regulator is a gene product, in particular a polypeptide, which obstructs the binding of RNA polymerase to the promoter region, which inhibits the activity of an enhancer or which inhibits the activity of an activating transcription factor, thus leading to a reduction of transcription of a target gene, such as C2TA.

CD8+ T cells (also called cytotoxic T lymphocytes, or CTLs) develop in the thymus and express the T-cell receptor. They express a dimeric co-receptor, CD8, usually composed of one CD8a and one CD8β chain. CD8+ T cells recognize peptides presented by MHC Class I molecules, found on all nucleated cells. CD8+ T cells are very important for immune defence against intracellular pathogens, including viruses and bacteria, and for tumour surveillance.

NK cells, also known as natural killer cells or large granular lymphocytes (LGL), are a type of cytotoxic lymphocyte critical to the innate immune system. NK cells can be identified by the presence of CD56 and the absence of CD3 (CD56+, CD3-). NK cells are innate immune cells with analogous functions to that of cytotoxic T cells in the adaptive immune response. NK cells provide rapid responses to virus-infected cells, acting at around 3 days after infection, and respond to tumor formation. NK cells have the ability to recognize and kill stressed cells in the absence of antibodies and MHC. This role is important because harmful cells that are missing MHC I markers cannot be detected and destroyed by other immune cells, such as T lymphocyte cells.

"Recruitment" of cells, such as CD8+T cells and/or NK cells, to a tumor can be tested in a mouse model, for example a humanized mouse model, by extracting the tumor mass, separating the cells and analyzing, for example by FACS after appropriate staining, the number of CD8+ T cells and/or NK cells associated with the tumor mass. A treatment that is able to recruit said cells to the tumor (such as the injection of macrophages of the invention) will over time lead to a situation where these cells make up a higher percentage in terms of cell numbers of the analyzed tumor mass when compared to appropriate controls without said treatment.

The term "proliferating cell" as used herein refers to a cell that is capable of cell division. A cell is a proliferating cell if a population of at least 1000 "proliferating cells" increases in cell number by at least 4-fold after 8 days under suitable cultivation conditions, i.e. when n(192h) / n(0h) is at least 4,00 with n being the total number of cells in the cell population at the indicated time points.

The term "differentiated human cell" as used herein is a cell that does not change cell type and even upon cell division gives rise to two cells of the same cell type. This is in contrast to "pluripotent cells" which can differentiate into all cell types of the adult organism and oligopotent cells, which can differentiate into a few closely related cell types.

A "myeloid cell" as used herein is a cell of hematopoietic origin that is not lymphoid and not erythro-megakaryocytic and not a multi-lineage progenitor with more than myeloid lineage potential. A "myeloid cell" according to the invention is selected from the group consisting of a macrophage, a monocyte, and a monocyte derived macrophage, wherein said myeloid cell, macrophage, monocyte, and monocyte derived macrophage can individually be derived from peripheral blood, umbilical cord blood, placenta, bone marrow or from iPS cells. A "myeloid cell" according to the invention is in some embodiments a genetically engineered myeloid cells, such as a cell in which the expression or activity of at least one of the transcription factors MafB and cMaf is reduced, inhibited or abolished, e.g. by a mutation in the MafB gene and/or a mutation iin the cMaf gene. Preferably, a myeloid cell of the invention is a monocyte or macrophage, in which the expression or activity of both, MafB and cMaf, has been reduced, inhibited or abolished (Maf-DKO). A Maf-DKO macrophage can be derived from a Maf-DKO monocyte or from a Maf-DKO iPS cell subjected to a differentiation protocol giving rise to monocytes and macrophages.

An iPS cell or "induced pluripotent stem cell" as used herein means a cell having pluripotency, which is obtained by reprogramming a somatic cell. Several groups including the group of Professor Shinya Yamanaka et al. of Kyoto University, the group of Rudolf Jaenisch et al. of Massachusetts Institute of Technology, the group of James Thomson et al. of the University of Wisconsin, and the group of Konrad Hochedlinger et al. of Harvard University have succeeded in producing such induced pluripotent stem cells. The induced pluripotent stem cells have attracted great interest as ideal pluripotent cells having neither immunological rejections nor ethical issues. For example, International Publication WO2007/069666 describes nuclear reprogramming factors for somatic cells that include the gene products of Oct family gene, Klf family gene and Myc family gene, and nuclear reprogramming factors for somatic cells that include the gene products of Oct family gene, Klf family gene, Sox family gene and Myc family gene. This publication also describes a method for producing induced pluripotent stem cells by nuclear reprogramming of somatic cells, which comprises a step of allowing the aforementioned nuclear reprogramming factors to come into contact with somatic cells.

A "monocyte" is a mononuclear phagocyte of the peripheral blood. Monocytes vary considerably, ranging in size from 10 to 30 µm in diameter. The nucleus to cytoplasm ratio ranges from 2:1 to 1:1. The nucleus is often band shaped (horseshoe), or reniform (kindey-shaped). It may fold over on top of itself, thus showing brainlike convolutions. No nucleoli are visible. The chromatin pattern is fine, and arranged in skein-like strands. The cytoplasm is abundant and appears blue gray with many fine azurophilic granules, giving a ground glass appearance in Giemsa staining. Vacuoles may be present. More preferably, the expression of specific surface antigens is used to determine whether a cell is a monocyte cell. Phenotypic markers of human monocyte cells include CD11b, CD11c, CD33, CD45 and CD115. Generally, human monocyte cells express CD9, CD11b, CD11c, CDw12, CD13, CD15, CDw17, CD31, CD32, CD33, CD35, CD36, CD38, CD43, CD45, CD49b, CD49e, CD49f, CD63, CD64, CD65s, CD68, CD84, CD85, CD86, CD87, CD89, CD91, CDw92, CD93, CD98, CD101, CD102, CD111, CD112, CD115, CD116, CD119, CDw121b, CDw123, CD127, CDw128, CDw131, CD147, CD155, CD156a, CD157, CD162, CD163, CD164, CD168, CD171, CD172a, CD180, CD131a1, CD213a2, CDw210, CD226, CD281, CD282, CD284, CD286 and optionally CD4, CD14, CD16, CD40, CD45RO, CD45RA, CD45RB, CD62L, CD74, CD141, CD142, CD169, CD170, CD181, CD182, CD184, CD191, CD192, CD194, CD195, CD197, CD206, CX3CR1. Unless specifically excluded, "monocytes" are comprised by the term "macrophage" as used herein.

A "dendritic cell" (DC) is an antigen presenting cell existing in vivo, in vitro, ex vivo, or in a host or subject, or which can be derived from a hematopoietic stem cell, a hematopoietic progenitor or a monocyte. Dendritic cells and their precursors can be isolated from a variety of lymphoid organs, e.g., spleen, lymph nodes, as well as from bone marrow and peripheral blood. The DC has a characteristic morphology with thin sheets (lamellipodia) extending in multiple directions away from the dendritic cell body. DCs express constitutively both MHC class I and class II molecules, which present peptide antigens to CD8+ and CD4+ T cells respectively and can activate naïve T-cells. In addition, human skin and mucosal DCs also express the CD1 gene family, MHC class I-related molecules that present microbial lipid or glycolipid antigens. The DC membrane is also rich in molecules that allow adhesion of T cells (e.g. intercellular adhesion molecule 1 or CD54) or that co-stimulate T-cell activation such as B7-1 and B7-2 (also known as CD80 and CD86 respectively). Generally, DCs express CD85, CD180, CD187 CD205 CD281, CD282, CD284, CD286 and in a subset manner CD206, CD207, CD208 and CD209. Unless specifically excluded, "dendritic cells" are comprised by the term "macrophage" as used herein.

A "macrophage" as used herein comprises macrophages, monocytes and dendritic cells. Preferably, however, a "macrophage" as used herein is a macrophage strictu sensu. A macrophage is a cell exhibiting properties of phagocytosis. The morphology of macrophages varies among different tissues and between normal and pathologic states, and not all macrophages can be identified by morphology alone. However, most macrophages are large cells with a round or indented nucleus, a well-developed Golgi apparatus, abundant endocytotic vacuoles, lysosomes, and phagolysosomes, and a plasma membrane covered with ruffles or microvilli. The key functions of macrophages in innate and adaptive immunity are the phagocytosis and subsequent degradation of senescent or apoptotic cells, microbes and neoplastic cells, the secretion of cytokines, chemokines and other soluble mediators, and the presentation of foreign antigens (peptides) on their surface to T lymphocytes. Macrophages are derived from multipotent progenitor cells, common myeloid progenitor cells and granulocyte-monocyte progenitor cells in the bone marrow of mammalian organisms, which ultimately develop through further progenitor stages into monocytes that then enter the peripheral bloodstream. Unlike neutrophils, with their multilobed nuclei, monocytes have kidney-shaped nuclei and assume a large cell body during further differentiation and activation. Throughout life, some monocytes adhere to and migrate through the endothelium of the capillaries into all organs, where some of them can differentiate into resident tissue macrophages or dendritic cells (see below). Besides monocyte origin, tissue resident macrophages can also develop from early primitive macrophage progenitors of the yolk sac from before the establishment of definitive hematopoiesis, from erythroid-macrophage progenitors (EMP) of diverse hematopoietic sites of the embryo or from embryonic hematopoietic stem cell derived fetal monocytes. These embryo derived macrophages can persist into adulthood and be maintained long term independently of input from adult hematopoietic stem cells and monocytes. Lymphatic tissues, such as the lymph nodes and the spleen, are particularly rich in macrophages but tissue resident macrophages are present in essentially every organ of the body. In some organs the macrophages carry special names, as summarized in Table 1.

**Table 1 Examples of tissue macrophages**

| **Organ** | **Macrophage population** |
|---|---|
| Bone | Osteoclasts |
| Central nervous system | Microglia |
| Connective tissue | Histiocytes |
| Chorion villi | Hofbauer cells |
| Kidney | Mesangial cells |
| Liver | Kupffer cells |
| Peritoneal cavity | Peritoneal macrophages |
| Pulmonary airways | Alveolar macrophages, lung interstitium interstitial macrophages |
| Skin | Epidermal langerhans cells and dermal macrophages |
| Spleen | Marginal zone macrophages, Metallophilic macrophages, Red pulp macrophages, White pulp macrophages |

Further examples of macrophages are peritubular and interstitial testicular macrophages, cardiac macrophages from heart, adipose tissue macrophages from fat tissue, large and small intestinal macrophages from intestine, skeletal muscle macrophages, synovial macrophages from joints, arterial adventitia macrophages, arterial intima macrophages, blood vessel associated macrophages, resident pancreatic macrophages, meningeal macrophages, pleural macrophages and omentum macrophages.

In the context of the invention, the macrophage can be selected from any one of the macrophages mentioned above, preferably the macrophage is selected from the group consisting of microglia, histiocytes, Hofbauer cells, mesangial cells, Kupffer cells, peritoneal macrophages, alveolar macrophage, epidermal or dermal macrophages, marginal zone macrophages, metallophilic macrophages, red pulp macrophages, white pulp macrophages and osteoclasts. Macrophages can also be derived from human iPS cells via in vitro differentiation protocols, as will be described elsewhere.

Macrophages are an important source of cytokines. Functionally, the numerous products can be placed into several groups: (1) cytokines that mediate a proinflammatory response, i.e. help to recruit further inflammatory cells (e.g. IL-1, II-6, TNFs, CC and CXC chemokines, such as IL-8 and monocyte-chemotactic protein 1); (2) cytokines that mediate T cell and natural killer (NK) cell activation (e.g. IL-1, IL-12, IL-15, IL-18); (3) cytokines that exert a feedback effect on the macrophage itself (e.g. IL-1, TNFs, IL-12, IL-18, M-CSF, IFNα/β, IFNy); (4) cytokines that downregulate the macrophage and/or help to terminate the inflammation (e.g. IL-10, TGFβs), (5) cytokines important for wound healing or to support tissue stem cells (e.g. EGF, PDGF, bFGF, TGFβ) or to support blood vessel growth (e.g. VEGF) or neurons (e.g. neurotrophic factors, kinins). The production of cytokines by macrophages can be triggered by microbial products such as LPS, by interaction with type 1 T-helper cells, or by soluble factors including prostaglandins, leukotrienes and, most importantly, other cytokines (e.g. IFNy). Generally, human macrophages express CD11c, CD11b, CD14, CD18, CD26, CD31, CD32, CD36, CD45RO, CD45RB, CD63, CD68, CD71, CD74, CD87, CD88, CD101, CD115, CD119, CD121b, CD155, CD156a, CD204, CD206 CDw210, CD281, CD282, CD284, CD286 and in a subset manner CD163, CD169 CD170, MARCO, FOLR2, LYVE1. Activated macrophages can further express CD23, CD25, CD69, CD105 and HLA-DR, HLA-DP and HLA-DQ

As used herein, a macrophage is resistant to profibrotic polarization by M-CSF, if cultivation of the macrophage (or macrophages) does not lead to the induction of a profibrotic gene signature. Characteristic genes of a profibrotic gene signature comprise genes selected from the group consisting of PMP22, IGF1, C1QC, C1QA, C1QB, FOLR2, TMEM37, MERTK, MAF, MAFB, RGL1, DAB2, C3AR1, PDPN, CD93, MARCKS, STAB1, CD63, C5AR1, HMOX1, LGMN, PDGFA, CD109, MRC1, ACP2, C5AR2, IER3, and ETS2.

A "surface marker" is a molecule, typically a protein or a carbohydrate structure, that is present and accessible on the exterior of the plasma membrane of a cell and that is specific for a particular cell type or a limited number of cell types, thereby being a "marker" for these cell types. Examples of surface markers on human macrophages are CD11c, CD11b, CD14, CD16, CD18, CD26, CD31, CD32, CD33, CD36, CD45RO, CD45RB, CD63, CD64, CD68, CD71, CD74, CD87, CD88, CD101, CD115, CD119, CD121b, CD155, CD156a, CD163, CD169, CD170, CD204, CD206 CDw210, CD281, CD282, CD284, CD286, MARCO, FOLR2, CX3CR1 and LYVE1.

A cell is "positive" for a surface marker if staining with a surface-marker-specific antibody creates a specific fluorescence signal in a FACS experiment. The principles of FACS are explained in detail in the book "practical flow cytometry", 4^{th} edition by Howard M. Shapiro. In a FACS experiment a collection of cells is typically stained with several fluorescent antibodies, each one selectively binding a different surface marker and having a different fluorochrome. This allows the selection of particular cell types within a heterogeneous collection of cells by appropriate gating strategies in a FACS experiment. A specific fluorescence signal by the surface-marker-specific antibody is typically then verified in a one-dimensional histogram plot by comparing the histograms for the staining with all antibodies with the histogram for the staining with the mix of antibodies where only the surface-marker-specific antibody has been omitted (so called "FMO" or "fluorescence minus one" signal). If the two histograms are different such that the staining with the mix of all antibodies produces more fluorescence than the FMO control, then the tested collection of cells is positive for the tested cell surface marker. In terms of visual appearance of the histogram this means that the peak of fluorescence for the staining with the mix of all antibodies is shifted to higher fluorescence values when compared to the FMO control. Preferably the two histograms - all antibodies on the one hand and FMO on the other - overlap by at most 70 area% (area under the curve), such as at most 50 area%, for example by at most 25 area %.

"Secretion" of a cytokine, such as IL18 and/or IL15, can be determined by qualitatively and/or quantitatively measuring the appearance of said cytokine in the supernatant of a cell culture with immunobiochemical methods, for example by ELISA-based methods or a bead-based multiplex assay, such as the Luminex technology, to name but two examples. Briefly, the medium used for cell growth is analyzed with regard to the presence of a particular cytokine to-be-tested BEFORE being added to a collection of cells and AFTER the cells, which are to be tested for cytokine secretion, have been cultured in it. A cytokine is "secreted" by the cells which were cultured in the medium if the concentration of the cytokine in the supernatant has increased during the cultivation period and if this increase in cytokine concentration can be confirmed in three consecutive independent measurements. IL6 can be detected at or even below a concentration of 0.1pg/ml, for example by the meso scale discovery immunoassay "V-PLEX Human IL-6 Kit" of Meso Scale Diagnostics. CXCL10 can be detected at or even below a concentration of 5pg/ml, for example by the LANCE Ultra human CXCL10 detection kit of Perkin Elmer.

The terms "phagocytic cells" and "phagocytes" are used interchangeably herein to refer to a cell that is capable of phagocytosis. There are different main categories of professional phagocytes: mononuclear phagocytes, comprising macrophages sensu strictu, monocytes and dendritic cells as well as polymorphonuclear leukocytes (neutrophils). However, there are also "non-professional" phagocytic cells known to participate in efferocytosis, efferocytosis being the process by which professional and nonprofessional phagocytes dispose of apoptotic cells in a rapid and efficient manner.

The term "progenitor cell" as used herein relates to cells which are descendants of stem cells and which can further differentiate to create specialized cell types. There are many types of progenitor cells throughout the human body. Each progenitor cell is only capable of differentiating into cells that belong to the same tissue or organ. Some progenitor cells have one final target cell that they differentiate to, while others have the potential to terminate in more than one cell type. Progenitor cells are thus an intermediary cell type involved in the creation of mature cells in human tissues and organs, the blood, and the central nervous system. Hematopoietic progenitor cells are an intermediate cell type in blood cell development. They are immature cells that develop from hematopoietic stem cells and eventually differentiate into one of more than ten different types of mature blood cells.

The term "CD34+ multipotent progenitors" as used herein are a CD34 surface antigen expressing stem-cell enriched hematopoietic progenitor population, that are not macrophages, monocytes or dendritic cells.

The term "monoblasts" as used herein relates to committed progenitor cells in bone marrow that differentiated from myeloid progenitor cells in the process of hematopoiesis. They can mature into monocytes which, in turn, can develop into macrophages.

The term "collection of cells" as used herein relates to at least 10000 cells, which cells are alive.

The term "expansion" of cells as used herein is the process of culturing cells under suitable laboratory conditions and increasing the number of living cells by mitotic divisions of the cultured cells.

The term "after having been exposed" as used herein means that cells were cultivated in the continuous presence of a particular agent, such as a cytokine, for the indicated period of time and then tested immediately thereafter.

The term "genetically modified" cell as used herein relates to a cell wherein the cell's DNA has been changed using biotechnological methods. For example, cells wherein the cells' DNA has been manipulated by the use of a CRISPR/Cas9 DNA editing system, wherein the manipulation has left a detectable change in the cells' DNA, are genetically modified cells.

The term "under suitable cultivation conditions" as used herein are conditions under which the phagocytic cells of the invention are able to grow. The cultivation is typically carried out in a cell culture medium supplemented with appropriate growth factors and at a suitable temperature and in a suitable controlled atmosphere. RPMI medium (RPMI derived its name from the Roswell Park Memorial Institute and is a medium frequently used for the culture of human lymphocytes) supplemented with 10% FBS (PAA-GE Healthcare, A15-101), supplemented with 100 units/ml penicillin, 100 ug/ml streptomycin (Thermo Fisher, #15140122), 2 mM GlutaMAX (Thermo Fisher, #35050038), 1 mM sodium pyruvate (Thermo Fisher, #11360-039), 50 ng/ml M-CSF (Thermo Fisher, #PHC9504), and, when indicated, 50 ng/ml GM-CSF (Peprotech, #300-03), as described in example 3, is a suitable cultivation medium. Cultivation is typically at 37°C and 5% CO2, 21% O2.

The term "reactive oxygen species" as used herein relates to unstable molecules that contains oxygen and that easily react with other molecules in a cell. Typically, reactive oxygen species are free radicals. Reactive Oxygen Species are known to be a component of the killing response of immune cells to microbial invasion.

The term "activation" as used herein relates to the phenomenon that external stimuli can induce changes to cell, thereby activating it. Macrophages, for example, can be activated by cytokines such as interferon-gamma (IFN-gamma) and bacterial endotoxins, such as lipopolysaccharide (LPS). Activated macrophages undergo many changes which allow them to kill invading bacteria or infected cells. They release toxic chemicals and proteins which have toxic effects on other cells. Activated macrophages are larger, have increased metabolism, increased levels of lysosomal proteins, and a greater ability to phagocytosis and kill microbes. Activated macrophages also release proteases, neutrophil chemotatic factors; reactive oxygen species such as nitric oxide and superoxide; cytokines such as tumor necrosis factor-alpha (TNF-alpha), interleukin one and eight (IL-1 and IL-8), eicosanoids, as well as growth factors. These products of activated macrophages can result in the kind of tissue destruction which is a hallmark of inflammation

The term "adherent" as used herein relates to the property of adherent cells that they attach to a solid substrate, such as the bottom of a tissue culture flask. In contrast, suspension cells will float and grow suspended in the culture medium, so they don't need to be mechanically or chemically removed.

Lamellipodium as used herein is a projection on the leading edge of the cell. It contains a quasi-two-dimensional actin mesh. A filopodium is a finger-like structure within the lamellipodium that has spread beyond the lamellipodium frontier and thus extends from it.

By "purified" and "isolated" it is meant, when referring to a polypeptide or a nucleotide sequence, that the indicated molecule is present in the substantial absence of other biological macromolecules. When referring to a cell or a population of cells, the term means that said cell or said population of cells is present in the substantial absence of other cells or population of cells. The term "purified" as used herein preferably means at least 75% by weight or number, more preferably at least 85% by weight or number, still preferably at least 95% by weight or number, and most preferably at least 98% by weight or number, of biological macromolecules or cells of the same type are present. An "isolated" nucleic acid molecule, which encodes a particular polypeptide refers to a nucleic acid molecule which is substantially free of other nucleic acid molecules that do not encode the subject polypeptide; however, the molecule may include some additional bases or moieties which do not deleteriously affect the basic characteristics of the composition.

As used herein, the terms "tumor," "tumor cells," "cancer," and "cancer cells," refer to cells which exhibit relatively autonomous growth, so that they exhibit an aberrant growth phenotype characterized by a significant loss of control of cell proliferation (i.e., de-regulated cell division) and/or disruption/non respect of normal tissue organization and architecture. Tumor cells can be malignant or benign, while cancer cells are malignant. A "metastatic cell or tissue" means that the cell can invade, settle in and destroy neighboring and distant body structures.

"Idiopathic pulmonary fibrosis (IPF)", as used herein is a chronic, progressive lung diseases where the lung tissue becomes thickened, stiff and scarred over time. This scarring (fibrosis) impedes normal lung function leading to difficulty to breathing and a decline oxygen absorption. The cause of fibrosis is unknown (idiopathic) and primarily affects the elderly with no cure, typically leading to respiratory failure and mortality.

"Fibrosis occurring in chronic obstructive pulmonary disease (COPD)" as used herein is a chronic, progressive lung condition that causes airflow obstruction causing breathing difficulties. It includes diseases like emphysema and chronic bronchitis which damage the airways and alveoli leading to coughing, wheezing and shortness of breath. Smoking and long-term exposure to pollutants are the leading causes of COPD.

"Fibrosis occurring in lung disease (ILD)" as used herein is known as interstitial lung disease is a group of lung disorders that are characterized by fibrotic scarring in the lung interstitium, the region surrounding the air sacs. This damage leads to shortness of breath, coughing and fatigue due to difficulty in oxygen uptake into the bloodstream. ILDs include autoimmune diseases, exposure to environmental toxins, infections and could also be idiopathic.

"Fibrosis occurring in viral infections (severe COVID-19 and pneumonia)" as used herein is caused by SARS-CoV-2 virus (COVD-19) and pneumonia which is caused by bacteria, virus or fungi and can lead to symptoms like fever, cough, chest pain, fatigue and difficulty in breathing. Both diseases could be life-threatening and could cause fibrotic scarring in the healing phase when the infectious agent causes significant tissue damage in patients with severe pneumonia or COVID-19 or who experienced acute respiratory distress syndrome (ARDS).

"Fibrosis occurring in end-stage liver diseases (including MASH, NASH and liver cirrhosis)" as used herein are result of gradual progression of fibrosis to cirrhosis which can take years depending on the underlying causes (Metabolic, non-alcoholic, chronic viral infection) and lifestyle factors like alcohol consumption or obesity. The disease is characterized by the accumulation of fat in the liver along with inflammation and liver cell damage. Over time, this leads to fibrosis where scar tissue forms eventually leading to cirrhosis. Liver cirrhosis is the final stage of liver disease with irreversible liver fibrosis where extensive scarring has replaced the healthy liver tissue and liver's ability to regenerate leading to disruption of normal liver function and eventual liver failure.

"Fibrosis occurring in autoimmune related diseases (SLE and GvHD)" as used herein is defined as follows: SLE is a complex autoimmune disease where the cause is not fully understood but genetic, environmental, hormonal and immune system defects are known to play a role. The host's immune system mistakenly attacks its own organs like heart, kidney, skin, lung and joints leading to inflammation. Prolonged inflammation leads to fibrotic scarring in organs like kidneys and lungs which can impair organ function over time. GvHD occurs when the donor immune cell attacks the recipient's tissues after a stem cell or bone marrow transplant and can affect various organs. Chronic GvHD can cause fibrosis in lungs and other organs leading to decline in organ function.

"Fibrosis occurring in several cancers (hepatic, pancreatic, gastric, oesophageal, colonic)" as used herein is defined as follows: Fibrotic cancers are characterized by excessive accumulation of fibrous connective tissue as a response to chronic inflammation or injury which leads to increased tumor stiffness and altered signaling that promotes tumor growth and metastasis. The development of fibrosis in a tumor context also complicates treatment by creating a dense physical barrier made of extracellular matrix that hinders drug delivery and immune cell infiltration decreasing the effectiveness of immunotherapy and chemotherapy.

As used herein, the term "subject" denotes a mammal, preferably a human being. Likewise, as used herein, the term "patient" denotes a mammalian patient, preferably a human patient.

In the context of the invention, the term "treating" or "treatment", as used herein, means reversing, alleviating, inhibiting the progress of, or preventing the disease or condition to which such term applies, or one or more symptoms of such disease or condition.

The term "ex-vivo" as used herein means outside of a living body.

The term "in-vitro" as used herein means outside of a living body and within a laboratory environment. For example, cells which are cultured "in-vitro" are cultured in controlled, and often artificial, culture media.

All numerical designations, e.g., pH, temperature, time, concentration, and molecular weight, including ranges, are approximations which are varied (+) or (-) by increments of 0.1. It is to be understood, although not always explicitly stated that all numerical designations are preceded by the term "about." It also is to be understood, although not always explicitly stated, that the reagents described herein are merely examples and that equivalents of such are known in the art.

It is to be understood that this invention is not limited to the particular materials and methods described herein. It is also to be understood that the terminology used herein is for the purpose of describing particular embodiments and is not intended to limit the scope of the present invention, which will be limited only by the appended claims. As used herein, the singular forms "a", "an", and "the" include plural reference unless the context clearly indicates otherwise. Unless defined otherwise, all technical and scientific terms used herein have the same meanings as commonly understood by one of ordinary skill in the art to which this invention belongs. The following references provide one of skill with a general definition of many of the terms used in this invention - unless defined otherwise herein - and ranked in increasing order of priority: Singleton et al., Dictionary of Microbiology and Molecular Biology (3rd ed. 2006); The Glossary of Genomics Terms (JAMA. 2013; 309(14):1533-1535), Janeway's Immunobiology, 9th edition and "Practical Flow Cytometry", 4th edition by H.M. Shapiro.

All publications mentioned herein are cited for the purpose of describing and disclosing the cell lines, protocols, reagents and vectors which are reported in the publications, and which might be used in connection with the invention. Nothing herein is to be construed as an admission that the invention is not entitled to antedate such disclosure by virtue of prior invention.

### DETAILED DESCRIPTION

In one aspect the present invention relates to a myeloid cell for use in therapy, such as the treatment of fibrosis, in a patient, wherein in said myeloid cell the expression level of the mRNA of certain genes is at least 2-fold lower, at least 3-fold lower, preferably at least 7-fold lower, more preferably at least 10-fold lower, even more preferably at least 15-fold lower, most preferably 30-fold lower even most preferably 60-fold lower than the expression level of said at least one mRNA of said at least one gene in an otherwise identical myeloid cell, such as wildtype myeloid cell (see Table 2 in the examples of the invention). Further most preferably, the present invention relates to a myeloid cell for use in therapy, such as the treatment of fibrosis, in a patient, wherein in said myeloid cell the expression level of the mRNA of certain genes is not upregulated compared to the expression level of said at least one mRNA of said at least one gene in an otherwise identical myeloid cell, such as wildtype myeloid cell (see Table 2 in the examples of the invention).

The expression level of genes can be e.g. analyzed by RNAseq analysis as described in the examples of the invention hereinbelow.

In one embodiment, when ranked based on the RNAseq results in myeloid cells, the top 10 genes that are not upregulated in myeloid cells are selected from the group consisting of PMP22, IGF1, C1QC, C1QA, C1QB, FOLR2, TMEM37, MERTK, MAF and MAFB. Accordingly, the present invention relates to a myeloid cell for use in therapy, such as the treatment of fibrosis, in a patient, wherein in said myeloid cell the expression level of at least one mRNA of at least one gene or more genes, such as one, two, three, four or five or more genes selected from the group consisting of PMP22, IGF1, C1QC, C1QA, C1QB, FOLR2, TMEM37, MERTK, MAF and MAFB is not upregulated.

In one embodiment, when ranked based on the RNAseq results in myeloid cells, the top 10 genes that are not upregulated in myeloid cells are selected from the group consisting of PMP22, IGF1, C1QC, C1QA, C1QB, FOLR2, TMEM37, and MERTK. Accordingly, the present invention relates to a myeloid cell for use in therapy, such as the treatment of fibrosis, in a patient, wherein in said myeloid cell the expression level of at least one mRNA of at least one gene or more genes, such as one, two, three, four or five or more genes selected from the group consisting of PMP22, IGF1, C1QC, C1QA, C1QB, FOLR2, TMEM37, and MERTK is not upregulated.

In one embodiment, the present invention relates to a myeloid cell for use in therapy, such as the treatment of fibrosis, in a patient, wherein in said myeloid cell the expression level of at least one mRNA of at least one gene or more genes, such as one, two, three, four or five or more genes selected from the group consisting of PMP22, IGF1, C1QC, C1QA, C1QB, FOLR2, TMEM37, MERTK, MAF, MAFB, RGL1, DAB2, C3AR1, PDPN, CD93, MARCKS, STAB1, CD63, C5AR1, HMOX1, LGMN, PDGFA, CD109, MRC1, ACP2, C5AR2, IER3, and ETS2. The expression level of these genes is at least 3-fold lower than the expression level of said at least one mRNA of said at least one gene in an otherwise identical myeloid cell, such as wildtype myeloid cell.

In one embodiment, the present invention relates to a myeloid cell for use in therapy, such as the treatment of fibrosis, in a patient, wherein in said myeloid cell the expression level of at least one mRNA of at least one gene or more genes, such as one, two, three, four or five or more genes selected from the group consisting of PMP22, IGF1, C1QC, C1QA, C1QB, FOLR2, TMEM37, MERTK, MAF, MAFB, RGL1, DAB2, C3AR1, PDPN, CD93, MARCKS, CD63, HMOX1, LGMN, PDGFA, CD109, MRC1, ACP2, C5AR2, IER3, and ETS2. The expression level of these genes is at least 3-fold lower than the expression level of said at least one mRNA of said at least one gene in an otherwise identical myeloid cell, such as wildtype myeloid cell.

In one embodiment, the present invention relates to a myeloid cell for use in therapy, such as the treatment of fibrosis, in a patient, wherein in said myeloid cell the expression level of at least one mRNA of at least one gene or more genes, such as one, two, three, four or five or more genes selected from the group consisting of PMP22, IGF1, C1QC, C1QA, C1QB, FOLR2, TMEM37, MERTK, RGL1, DAB2, C3AR1, PDPN, CD93, MARCKS, CD63, HMOX1, LGMN, PDGFA, CD109, MRC1, ACP2, C5AR2, IER3, and ETS2. The expression level of these genes is at least 3-fold lower than the expression level of said at least one mRNA of said at least one gene in an otherwise identical myeloid cell, such as wildtype myeloid cell.

Preferably, the present invention relates to a myeloid cell for use in therapy, such as the treatment of fibrosis, in a patient, wherein in said myeloid cell the expression level of at least one mRNA of at least one gene or more genes, such as one, two, three, four or five or more genes selected from the group consisting of RGL1, DAB2, C3AR1, PDPN, CD93, MARCKS, STAB1, CD63, C5AR1, HMOX1, LGMN and PDGFA is at least 7-fold lower than the expression level of said at least one mRNA of said at least one gene in an otherwise identical myeloid cell, such as wildtype myeloid cell. In one embodiment, in said myeloid cell the expression level of at least one mRNA of at least one gene or more genes, such as one, two, three, four or five or more genes selected from the group consisting of RGL1, DAB2, C3AR1, PDPN, CD93, MARCKS, and CD63, HMOX1, LGMN and PDGFA is at least 7-fold lower than the expression level of said at least one mRNA of said at least one gene in an otherwise identical myeloid cell, such as wildtype myeloid cell.

More preferably, the present invention relates to a myeloid cell for use in therapy, such as the treatment of fibrosis, in a patient, wherein in said myeloid cell the expression level of at least one mRNA of at least one gene or more genes, such as one, two, three, four or five or more genes selected from the group consisting of RGL1, DAB2, C3AR1, PDPN, CD93, MARCKS, and CD63 is at least 10-fold lower than the expression level of said at least one mRNA of said at least one gene in an otherwise identical myeloid cell, such as wildtype myeloid cell.

Even more preferably, the present invention relates to a myeloid cell for use in therapy, such as the treatment of fibrosis, in a patient, wherein in said myeloid cell the expression level of at least one mRNA of at least one gene or more genes, such as one, two, three, four or five or more genes selected from the group consisting of RGL1, DAB2, C3AR1, PDPN, and CD93 is at least 15-fold lower than the expression level of said at least one mRNA of said at least one gene in an otherwise identical myeloid cell, such as wildtype myeloid cell.

Most preferably, the present invention relates to a myeloid cell for use in therapy, such as the treatment of fibrosis, in a patient, wherein in said myeloid cell the expression level of at least one mRNA of at least one gene or more genes, such as one, two or three genes selected from the group consisting of RGL1, DAB2, and C3AR1 is at least 30-fold lower than the expression level of said at least one mRNA of said at least one gene in an otherwise identical myeloid cell, such as wildtype myeloid cell.

Even most preferably, the present invention relates to a myeloid cell for use in therapy, such as the treatment of fibrosis, in a patient, wherein in said myeloid cell the expression level of at least one mRNA of at least one gene or two genes selected from the group consisting of RGL1, and DAB2 is at least 60-fold lower than the expression level of said at least one mRNA of said at least one gene in an otherwise identical myeloid cell, such as wildtype myeloid cell.

In a further aspect of the invention the DEGs can be ranked on the basis of the top upregulated genes in WT monocytes upon M-CSF treatment (see Table 3 in the examples of the invention). Said top DEGs according to this aspect of the invention are selected from the group consisting of DAB2, STAB1, PMP22, IGF1, C1QC, PDPN, C1QA, C3AR1, C1QB and FOLR2, preferably from the group consisting of DAB2, PMP22, IGF1, C1QC, PDPN, C1QA, C3AR1, C1QB and FOLR2. Said top DEGs are either not upregulated or show an at least 10-fold downregulated expression of their mRNA in the myeloid cells of the invention when compared to the expression level of their mRNA in an otherwise identical myeloid cell, such as wildtype myeloid cell (see Table 3 in the examples of the invention).

Accordingly, the invention relates to a myeloid cell for use in therapy, such as the treatment of fibrosis, in a patient, wherein in said myeloid cell the expression level of at least one mRNA of at least one gene or more genes, such as one, two, three, four or five or more genes selected from the group consisting of DAB2, STAB1, PMP22, IGF1, C1QC, PDPN, C1QA, C3AR1, C1QB and FOLR2, preferably from the group consisting of DAB2, PMP22, IGF1, C1QC, PDPN, C1QA, C3AR1, C1QB and FOLR2, is at least 10-fold downregulated or wherein at least one of said genes is not upregulated when compared to the expression level of their mRNA in an otherwise identical myeloid cell, such as wildtype myeloid cell (see Table 3 in the examples of the invention).

In a further aspect of the invention, it was observed that genes that were upregulated by M-CSF in WT monocytes were identified that were also well correlated to profibrotic genes described in over 15 relevant reference fibrosis datasets that defined 20 profibrotic macrophage clusters using scRNAseq (see Figure 13 and Table 4 in the examples of the invention). When ranked on the basis of the number of occurrences in the 20 defined profibrotic macrophage clusters in literature, the top DEGs expressed in WT monocytes post M-CSF treatment are selected from the group consisting of LGMN, SPP1, CSTB, LIPA, DAB2, CD63, MAFB, MARCKS, C1QC and HMOX1. Said top DEGs are either not upregulated or show an at least 2-fold downregulated expression of their mRNA in the myeloid cells of the invention when compared to the expression level of their mRNA in an otherwise identical myeloid cell, such as wildtype myeloid cell (see Table 4 in the examples of the invention).

Accordingly, the invention relates to a myeloid cell for use in therapy, such as the treatment of fibrosis, in a patient, wherein in said myeloid cell the expression level of at least one mRNA of at least one gene or more genes, such as one, two, three, four or five or more genes selected from the group consisting of LGMN, SPP1, CSTB, LIPA, DAB2, CD63, MAFB, MARCKS, C1QC and HMOX1 is at least 10-fold downregulated or wherein at least one of said genes is not upregulated when compared to the expression level of their mRNA in an otherwise identical myeloid cell, such as wildtype myeloid cell (see Table 4 in the examples of the invention).

The myeloid cell of the invention is a myeloid cell as defined in the definition section above. Preferably, the myeloid cell of the invention is a myeloid cell which has been exposed to M-CSF before the RNAseq analysis.

Additionally, or alternatively, the myeloid cell of the invention is resistant to M-CSF induced profibrotic polarization after having been exposed to M-CSF in a range of 50 ng/ml to 200 ng/ml for 2-72 hours. The myeloid cell of the invention can be genetically modified, for example by introduction of gene encoding a surface protein, such as a chimeric antigen receptor, but the myeloid cell of the invention can also be a genetically unmodified myeloid cell, such as a macrophage, for example derived from iPS cells.

Preferred myeloid cells for use in therapy according to the invention are myeloid cells comprising at least one mutation in both alleles of a gene located on a chromosome, wherein the macrophage is resistant to M-CSF induced profibrotic polarization. Myeloid cells of the invention are anti-fibrotic and thus useful for cell-therapy, in particular for anti-fibrosis therapy.

More preferably, myeloid cells for use in therapy according to invention are Maf-DKO cells and are resistant to M-CSF induced profibrotic polarization.

In particular, the human myeloid cell for therapy of the invention is resistant to profibrotic polarization by exposure to M-CSF.

Most preferably, the myeloid cell of the invention is a Maf-DKO monocyte or a Maf-DKO macrophage which has been exposed to M-CSF (e.g. to 50 ng/ml M-CSF) before the RNAseq analysis. Even most preferably, the myeloid cell of the invention is a Maf-DKO monocyte or a Maf-DKO macrophage which has been exposed to M-CSF before the RNAseq analysis, wherein said Maf-DKO monocyte or a Maf-DKO macrophage has been derived from an iPS cell.

Resistant to profibrotic polarization means for example that the human myeloid cell, such as the human monocyte or human macrophage, for use in therapy according to the invention has been exposed to M-CSF for 24 hours, in particular for 48 hours, such as for 72 hours, or even longer. For example, the human myeloid cell, such as the human monocyte or human macrophage, can be exposed to 50ng/ml M-CSF for 24 hours, or even, for example to 50ng/ml M-CSF for 48 hours, such as for 72 hours, to achieve resistance to profibrotic polarization.

The myeloid cell of the invention is preferably a human myeloid cell, which is resistant to profibrotic polarization.

More preferably, the myeloid cell of the invention is a human monocyte, which is resistant to profibrotic polarization.

Most preferably, the myeloid cell of the invention is a human macrophage, which is resistant to profibrotic polarization. The patient of the invention is preferably a human patient.

The "otherwise identical myeloid cell", such as a "wildtype myeloid cell" has preferably been exposed M-CSF (e.g. to 50 ng/ml M-CSF for 48 hours) before RNAseq analysis.

The present invention also relates to a method for treating a subject, preferably a human subject affected with fibrosis, which method comprises administering to a subject a myeloid cell of the invention or a collection of myeloid cells of the invention, wherein said myeloid cell shows a gene expression pattern as described hereinbefore, in a therapeutically effective amount for treating said fibrosis.

The myeloid cell, such as the human myeloid cell for use in therapy according to the invention can be characterized by their gene expression profile. A typical feature of human myeloid cell can be, for example, expression of the STAB1 gene. In particular, the expression level of STAB-1 mRNA in the myeloid cell is, preferably after stimulation with M-CSF, lower, such as at least 8-fold, for example at least 10-fold, such as at least 12-fold or even at least 13-fold lower than the expression level of STAB1 mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the C5AR1 gene. In particular, the expression level of C5AR1 mRNA in the myeloid cell is, preferably after stimulation with M-CSF, lower, such as at least 3-fold, for example at least 6-fold, such as at least 9-fold lower than the expression level of C5AR1 mRNA in an otherwise identical myeloid cell, such as wildtype myeloid cell, tested under otherwise equivalent cultivation conditions.

Alternatively, or additionally, a typical feature of the human myeloid cell according to the invention can be, for example, expression of the SPP1 gene. In particular, the expression level of SPP1 mRNA in the myeloid cell is, preferably after stimulation with M-CSF, lower, such as at least 2-fold, for example at least 2.3-fold, such as at least 2.6-fold or even at least 2.8-fold lower than the expression level of SPP1 mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the Rgl1 gene. In particular, the expression level of Rgl1 mRNA in the myeloid cell is, preferably after stimulation with M-CSF, lower, such as at least 20-fold, for example at least 40-fold, such as at least 60-fold or even at least 80-fold lower than the expression level of Rgl1 mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the MRC1 gene. In particular, the expression level of MRC1 mRNA in the myeloid cell is, preferably after stimulation with M-CSF, lower, such as at least 3-fold, for example at least 4-fold, such as at least 5-fold lower than the expression level of MRC1 mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the MARCKS gene. In particular, the expression level of MARCKS mRNA in the myeloid cell, is, preferably after stimulation with M-CSF, lower, such as at least 3-fold, for example at least 6-fold, such as at least 9-fold or even at least 13-fold lower than the expression level of MARCKS mRNA in an otherwise identical myeloid cell, such as wildtype myeloid cell, tested under otherwise equivalent cultivation conditions.

Alternatively, or additionally, a typical feature of a myeloid cell according to the invention can be, for example, expression of the LIPA gene. In particular, the expression level of LIPA mRNA in the myeloid cell is, preferably after stimulation with M-CSF, lower, such as at least 2-fold, for example at least 2.1-fold, such as at least 2.2-fold lower than the expression level of LIPA mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the LGMN gene. In particular, the expression level of LGMN mRNA in the myeloid cell, is, preferably after stimulation with M-CSF, lower, such as at least 2-fold, for example at least 4-fold, such as at least 6-fold or even at least 8-fold lower than the expression level of LGMN mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the HMOX1 gene. In particular, the expression level of HMOX1 mRNA in the myeloid cell is, preferably after stimulation with M-CSF, lower, such as at least 2-fold, for example at least 4-fold, such as at least 6-fold or even at least 8-fold or 9-fold lower than the expression level of HMOX1 mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the DAB2 gene. In particular, the expression level of DAB2 mRNA in the myeloid cell is, preferably after stimulation with M-CSF, lower, such as at least 15-fold, for example at least 30-fold, such as at least 45-fold or even at least 60-fold or 64-fold lower than the expression level of DAB2 mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the CSTB gene. In particular, the expression level of CSTB mRNA in the myeloid cell is, preferably after stimulation with M-CSF, lower, such as at least 2-fold, for example at least 2.2-fold, such as at least 2.4-fold or even at least 2.6-fold lower than the expression level of CSTB mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the CD93 gene. In particular, the expression level of CD93 mRNA in the myeloid cell is, preferably after stimulation with M-CSF, lower, such as at least 3-fold, for example at least 7-fold, such as at least 11-fold or even at least 15-fold lower than the expression level of CD93 mRNA in an otherwise identical myeloid cell, such as wildtype myeloid cell, tested under otherwise equivalent cultivation conditions.

Alternatively, or additionally, a typical feature of the myeloid cell can be, for example, expression of the CD63 gene. In particular, the expression level of CD63 mRNA in the myeloid cell is, preferably after stimulation with M-CSF, lower, such as at least 3-fold, for example at least 6-fold, such as at least 8-fold or even at least 10-fold lower than the expression level of CD63 mRNA in an otherwise identical myeloid cell, such as wildtype myeloid cell.

Alternatively, or additionally, a typical feature of the myeloid cell according to the invention can be, for example, expression of the C3AR1 gene. In particular, the expression level of C3AR1 mRNA in the myeloid cell is, preferably after stimulation with M-CSF, lower, such as at least 10-fold, for example at least 20-fold, such as at least 30-fold or even at least 40-fold or 44-fold lower than the expression level of C3AR1 mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the PDPN gene. In particular, the expression level of PDPN mRNA in the myeloid cell is, preferably after stimulation with M-CSF, lower, such as at least 2-fold, for example at least 7-fold, such as at least 12-fold or even at least 15-fold lower than the expression level of PDPN mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the PDGFA gene. In particular, the expression level of PDGFA mRNA in the myeloid cell is, preferably after stimulation with M-CSF, lower, such as at least 2-fold, for example at least 4-fold, such as at least 6-fold or even at least 7-fold or 8-fold lower than the expression level of PDGFA mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the IER3 gene. In particular, the expression level of IER3 mRNA in the myeloid cell is, preferably after stimulation with M-CSF, lower, such as at least 2-fold, for example at least 2.5-fold, such as at least 3.0-fold or even at least 3.3-fold lower than the expression level of IER3 mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the ETS2 gene. In particular, the expression level of ETS2 mRNA in the myeloid cell is, preferably after stimulation with M-CSF, lower, such as at least 2-fold, for example at least 2,5-fold, such as at least 3-fold lower than the expression level of ETS2 mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the CD109 gene. In particular, the expression level of CD109 mRNA in the myeloid cell is, preferably after stimulation with M-CSF, lower, such as at least 3-fold, for example at least 4-fold, such as at least 5-fold or even at least 5.9-fold lower than the expression level of CD109 mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the C5AR2 gene. In particular, the expression level of C5AR2 mRNA in the myeloid cell is, preferably after stimulation with M-CSF, lower, such as at least 2-fold, for example at least 2.5-fold, such as at least 3.0-fold or even at least 3.5-fold or 3.8-fold lower than the expression level of C5AR2 mRNA in an otherwise identical myeloid cell, such as wildtype myeloid cell, tested under otherwise equivalent cultivation conditions.

Alternatively, or additionally, a typical feature of the myeloid cell according to the invention can be, for example, expression of the ACP2 gene. In particular, the expression level of ACP2 mRNA in the myeloid cell is, preferably after stimulation with M-CSF, lower, such as at least 2-fold, for example at least 3-fold, such as at least 4-fold or even at least 5-fold or 5.4-fold lower than the expression level of ACP2 mRNA in an otherwise identical myeloid cell, such as wildtype myeloid cell, tested under otherwise equivalent cultivation conditions.

Alternatively, or additionally, a typical feature of the myeloid cell according to the invention can be, for example, that one or more genes are, after stimulation with M-CSF, are not upregulated in said myeloid cell in particular, one or more, such as one, two, three, four, five or more genes selected from the group consisting of TMEM37, C1QC, C1QB, PMP22, MERTK, IGF1, FOLR2, C1QA, MAF and MAFB.

In a preferred embodiment, in the myeloid cell according to the invention the expression of the TMEM37 gene is, after stimulation with M-CSF, not upregulated.

In a preferred embodiment, in the myeloid cell according to the invention the expression of the C1QC gene is, after stimulation with M-CSF, not upregulated.

In a preferred embodiment, in the myeloid cell according to the invention the expression of the C1QB gene is, after stimulation with M-CSF, not upregulated.

In a preferred embodiment, in the myeloid cell according to the invention the expression of the PMP22 gene is, after stimulation with M-CSF, not upregulated.

In a preferred embodiment, in the myeloid cell according to the invention the expression of the MERTK gene is, after stimulation with M-CSF, not upregulated.

In a preferred embodiment, in the myeloid cell according to the invention the expression of the IGF1 gene is, after stimulation with M-CSF, not upregulated.

In a preferred embodiment, in the myeloid cell according to the invention the expression of the FOLR2 gene is, after stimulation with M-CSF, not upregulated.

In a preferred embodiment, in the myeloid cell according to the invention the expression of the C1QA gene is, after stimulation with M-CSF, not upregulated.

In a preferred embodiment, in the myeloid cell according to the invention the expression of the MAF gene is, after stimulation with M-CSF, not upregulated.

In a preferred embodiment, in the myeloid cell according to the invention the expression of the MAFB gene is, after stimulation with M-CSF, not upregulated.

The myeloid cell for use in the treatment of fibrosis according to the invention can also be characterized by combinations of any one of the above-described gene expression markers, in particular above-described preferred gene expression markers.

In some embodiments, the myeloid cell for use in the treatment of fibrosis according to the invention comprises at least one mutation in both alleles of a gene located on a chromosome. The mutation can render the gene non-functional and/or it can inhibit gene expression or even abolish gene expression.

The mutation can be an insertion or a frameshift. However, a preferred mutation is a deletion, for example a deletion within a promotor, an exon or a splice site. While small deletions of only a few base pairs can have an effect on gene function, preferably the deletion is a deletion of at least 50 base pairs, such as at least 100 base pairs, at least 200 base pairs, at least 500 base pairs or even at least 1000 base pairs.

A preferred myeloid cell for use in the treatment of fibrosis according to the invention comprises at least two different mutations, and preferably said two mutations are deletions within at least two different genes.

As explained above, the present invention preferably provides a myeloid cell comprising at least one mutation in both alleles of a gene located on a chromosome, wherein the myeloid cell is resistant to M-CSF induced profibrotic polarization, for use in methods of treating of fibrosis. The present invention provides a new selectable phenotype for cells comprising at least one mutation in both alleles of a gene located on a chromosome, for which can be screened and thus yet unknown genes whose mutation renders the myeloid cell resistant to M-CSF induced profibrotic-polarization. Thus, the nature of the gene is not particularly limiting, as long as biallelic mutation of the gene produces the desired phenotype. However, a preferred myeloid cell for use in therapy according to the invention have biallelic mutations in genes which are involved in M-CSF mediated downregulation of profibrotic genes, such as negative regulators, and in particular negative transcriptional regulators, of profibrotic genes. Preferably the gene can be selected from the group consisting of MAFB and MAF.

A preferred myeloid cell for use in therapy according to the invention, comprising at least one mutation in both alleles of a gene located on a chromosome, wherein the myeloid cell is resistant to M-CSF induced profibrotic polarization, is a myeloid cell, wherein said mutated gene is MAFB. It is preferred that MAFB is the only transcription factor-encoding gene comprising biallelic deletions. Alternatively it is preferred that MAFB is the only protein-coding gene comprising biallelic deletions. Alternatively it is preferred that MAFB is the only gene comprising biallelic deletions. If further transcription factors - other than MAF and/or MAFB - are deleted, myeloid cell function might be compromised or differentiation of induced pluripotent stem cells into macrophages might not be possible. For example, Buchrieser et al., 2017, have shown that induced pluripotent stem cells having a deletion of the transcription factor RUNX1 or SPI1 cannot be differentiated into iPSC-derived macrophages.

The preferred myeloid cell for use in the treatment of fibrosis according to the invention, wherein said mutated gene is MAFB, can also be characterized by the nature and size of the deletions. For example, all deletions can be in exonic parts of the MAFB. For example, the deletions can be larger than 50 base pairs each, such as at least 100 base pairs each or at least 250 base pairs each. For example, the deletions of or within the MAFB gene can be from 100 base pairs to 10000 base pairs each, such as from 500 base pairs to 3000 base pairs each, in particular from 700 base pairs to 2000 base pairs. The preferred myeloid cell for use in the treatment of fibrosis according to the invention, wherein said mutated gene of the invention is MAFB, can show one or more, such as all, of these deletions.

The preferred myeloid cell for use in the treatment of fibrosis according to the invention, wherein said mutated gene is MAFB, can also be characterized by the location of the deletion in a particular chromosome. For example, the MAFB deletions are preferred within the region on human chromosome 22 from 40685000 to 40690000, such as from 40685200 to 40689800, for example from 40685400 to 40689600, such as from 40685600 to 40689400 and in particular from 40685700 to 40689300.

A preferred myeloid cell for use in the treatment of fibrosis according to the invention, comprising at least one mutation in both alleles of a gene located on a chromosome, wherein the myeloid cell, for example the human macrophage, is resistant to M-CSF induced profibrotic -polarization, is a myeloid cell, for example the human macrophage, wherein said mutated gene is MAF. It is preferred that MAF is the only transcription factor-encoding gene comprising biallelic deletions. Alternatively it is preferred that MAF is the only protein-coding gene comprising biallelic deletions. Alternatively it is preferred that MAF is the only gene comprising biallelic deletions.

The preferred myeloid cell for use in the treatment of fibrosis according to the invention, wherein said mutated gene is MAF can also be characterized by the nature and size of the deletions. For example, all deletions can be in exonic parts of the MAF. For example, the deletions can be larger than 50 base pairs each, such as at least 100 base pairs each or at least 250 base pairs each. For example, the deletions of or within the MAF gene can be from 100 base pairs to 10000 base pairs each, such as from 500 base pairs to 3000 base pairs each, in particular from 700 base pairs to 2000 base pairs. The preferred myeloid cell for use in therapy according to the invention wherein said mutated gene of the invention is MAF can show one or more, such as all, of these deletions.

The human myeloid for use in the treatment of fibrosis according to the invention, wherein said mutated gene is MAF can also be characterized by the location of the deletion in a particular chromosome. For example, the MAF deletions are preferred within the region on human chromosome 16 from 79593000 to 79602000, such as from 79593200 to 79601500, for example from 79593400 to 79601100, and in particular from 79593600 to 79600900.

The myeloid cell for use in the treatment of fibrosis according to the invention, wherein the myeloid cell is resistant to M-CSF induced profibrotic -polarization, may be prepared by manipulation of a cell. Cells, from which the human myeloid cell can be derived, can be cells which are progenitors for the development of human myeloid cell, for example the human macrophage,, for example cells selected from the group consisting of iPS cells; blood monocytes; monocytes, monoblasts, myeloid or CD34+ multipotent progenitors from cord blood; monocytes, monoblasts, myeloid or CD34+ multipotent progenitors from bone marrow; mobilized monoblasts, myeloid or CD34+ multipotent progenitors from adult blood; and monocytes, monoblasts, myeloid or CD34+ progenitors from extramedullary hematopoiesis. The myeloid cell for use in therapy according to the invention can also be derived from macrophages, in particular macrophages which are relatively easily accessible such as alveolar macrophages or macrophages from fat tissue. A preferred cell from which the myeloid cell of the invention can be derived is a human iPS cell. Another preferred cell from which the myeloid cellof the invention can be derived is a CMP (common myeloid progenitor). Another preferred cell from which the myeloid cellof the invention can be derived is a GMP (granulocyte/macrophage progenitor).

The preparation of human induced pluripotent stem cells is by now well established in the art. Isogai et al., 2018, for example, describe the preparation of iPS cells from human blood monocytes. Fusaki et al., 2009, describe the generation of human iPS cell lines with a modified Sendai virus. In particular when human induced pluripotent stem cells are used as the starting material for myeloid cell, for example the human macrophage, differentiation it is preferred that MAF and/or MAFB is/are the only transcription factor-encoding gene(s) comprising biallelic deletions. Alternatively it is preferred that MAF and/or MAFB are the only protein-coding genes comprising biallelic deletions. Alternatively it is preferred that MAF and/or MAFB are the only genes comprising biallelic deletions. If further genes are deleted, macrophage function might be compromised or differentiation of induced pluripotent stem cells into macrophages might not be possible.

Human mononuclear phagocytes for the biallelic mutation of genes can also be derived from primary human blood monocytes, which can be obtained by leukapheresis and elutriation, or from monocyte derived macrophages, for which culture conditions are well known to the art. Human macrophages can also be obtained from bronchoalveolar lavage and by differentiation of CD34+ hematopoietic stem and progenitor cells obtained from umbilical cord blood, stem cell mobilized peripheral blood or bone marrow, using differentiation protocols well known to the art.

Cas9 and gRNAs can be expressed by publicly and commercially available DNA expression plasmids well known in the art (for example Santa Cruz Sc-418922, https://www.scbt.com/de/p/control-crispr-cas9-plasmid). DNA expression plasmids can be introduced into human mononuclear phagocyte target cells by electroporation or lipid-based transfection protocols well known to the art. Cas9 and gRNA can also be introduced into target cells as a ribonucleic/protein complex by electroporation. Cas9/gRNA mediated gene editing has been demonstrated in mononuclear phagocytes using such methods (Zhang et al., 2020; Wang et al., 2018; Freund et al., 2020) and in human CD34+ hematopoietic stem and progenitor cells differentiating to macrophages (Scharenberg et al., 2020). Target genes, such as the MAFB and MAF genes, could also be deleted utilizing engineered site directed recombinases (Lansing et al., 2020; Karpinski et al., 2016), which can be introduced by methods known to the art, such as electroporation of the protein, coding mRNA or DNA expression plasmids, and which have been used for gene editing in mononuclear phagocytes (Shi et al., 2018).

González et al., 2014, describe an iCRISPR platform for rapid, multiplexable and inducible genome editing in human pluripotent stem cells based on the introduction of a doxycycline-inducible Cas9 expression cassette into the AAVS1 locus. This strategy can be used to introduce an inducible Cas9-expression cassette into a wide variety of self-generated or publicly available human iPS cell lines. Such modified cell lines can then be used for targeting selected genes, such as MAF and/or MAFB.

The invention further relates to the following embodiments
1. A myeloid cell for use in therapy in a patient characterized in that the expression level of the mRNA of profibrotic genes in said myeloid cell is at least 2-fold lower, at least 3-fold lower, preferably at least 7-fold lower, more preferably at least 10-fold lower, even more preferably at least 15-fold lower, most preferably 30-fold lower even most preferably 60-fold lower than the expression level of said mRNA of said profibrotic genes in an otherwise identical myeloid cell, such as wildtype myeloid cell.
2. The myeloid cell for therapeutic use according to 1, wherein the therapeutic use is the treatment of fibrosis.
3. The myeloid cell for therapeutic use according to any one of 1 or 2, wherein the myeloid cell is selected from the group consisting of a macrophage, a monocyte, and a monocyte derived macrophage, wherein said myeloid cell, macrophage, monocyte, and monocyte derived macrophage can independently be derived from peripheral bone marrow, blood, umbilical cord blood, placenta or from iPS cells.
4. The myeloid cell for use in the treatment of fibrosis in a patient according to any one of 1 to 3, wherein the myeloid cell is a macrophage and wherein in said macrophage, at least one gene selected from the group consisting of GPNMB, LGMN, SPP1, TREM2, CSTB, EMP1, and LIPA is significantly less expressed.
5. The myeloid cell for use in the treatment of fibrosis in a human patient according to any one of 1 to 3, wherein in said myeloid cell the expression level of at least one mRNA of at least one gene or more genes, such as one, two, three, four or five or more genes selected from the group consisting of PMP22, IGF1, C1QC, C1QA, C1QB, FOLR2, TMEM37, MERTK, MAF, MAFB, RGL1, DAB2, C3AR1, PDPN, CD93, MARCKS, STAB1, CD63, C5AR1, HMOX1, LGMN, PDGFA, CD109, MRC1, ACP2, C5AR2, IER3, and ETS2 is at least 3-fold lower than the expression level of said at least one mRNA of said at least one gene in an otherwise identical myeloid cell, such as wildtype myeloid cell.
6. The myeloid cell for use in the treatment of fibrosis in a patient according to any one of 1 to 5, wherein in said myeloid cell the expression level of at least one mRNA of at least one gene or more genes, such as one, two, three, four or five or more genes selected from the group consisting of RGL1, DAB2, C3AR1, PDPN, CD93, MARCKS, STAB1, CD63, C5AR1, HMOX1, LGMN and PDGFA is at least 7-fold lower than the expression level of said at least one mRNA of said at least one gene in an otherwise identical myeloid cell, such as wildtype myeloid cell.
7. The myeloid cell for use in the treatment of fibrosis in a patient according to any one of 1 to 6, wherein in said myeloid cell the expression level of at least one mRNA of at least one gene or more genes, such as one, two, three, four or five or more genes selected from the group consisting of RGL1, DAB2, C3AR1, PDPN, CD93, MARCKS, and CD63 is at least 10-fold lower than the expression level of said at least one mRNA of said at least one gene in an otherwise identical myeloid cell, such as wildtype myeloid cell.
8. The myeloid cell for use in the treatment of fibrosis in a patient according to any one of 1 to 7, wherein in said myeloid cell the expression level of at least one mRNA of at least one gene or more genes, such as one, two, three, four or five or more genes selected from the group consisting of RGL1, DAB2, C3AR1, PDPN, and CD93 is at least 15-fold lower than the expression level of said at least one mRNA of said at least one gene in an otherwise identical myeloid cell, such as wildtype myeloid cell.
9. The myeloid cell for use in the treatment of fibrosis in a patient according to any one of 1 to 8, wherein in said myeloid cell the expression level of at least one mRNA of at least one gene or more genes, such as one, two or three genes selected from the group consisting of RGL1, DAB2, and C3AR1 is at least 30-fold lower than the expression level of said at least one mRNA of said at least one gene in an otherwise identical myeloid cell, such as wildtype myeloid cell.
10. The myeloid cell for use in the treatment of fibrosis in a patient according to any one of 1 to 9, wherein in said myeloid cell the expression level of at least one mRNA of at least one gene or two genes selected from the group consisting of RGL1, and DAB2 is at least 60-fold lower than the expression level of said at least one mRNA of said at least one gene in an otherwise identical myeloid cell, such as wildtype myeloid cell.
11. The myeloid cell for use in the treatment of fibrosis in a patient according to 1, wherein in said myeloid cell the expression level of at least one mRNA of at least one gene or more genes, such as one, two, three, four or five or more genes selected from the group consisting of PMP22, IGF1, C1QC, C1QA, C1QB, FOLR2, TMEM37, MERTK, MAF and MAFB is not upregulated.
12. The myeloid cell for use in the treatment of fibrosis in a patient according to 1, wherein in said myeloid cell the expression level of at least one mRNA of at least one gene or more genes, such as one, two, three, four or five or more genes selected from the group consisting of DAB2, STAB1, PMP22, IGF1, C1QC, PDPN, C1QA, C3AR1, C1QB and FOLR2, is either not upregulated or shows an at least 10-fold downregulated expression of their mRNA when compared to the expression level of their mRNA in an otherwise identical myeloid cell, such as wildtype myeloid cell.
13. The myeloid cell for use in the treatment of fibrosis in a patient according to 1, wherein in said myeloid cell the expression level of at least one mRNA of at least one gene or more genes, such as one, two, three, four or five or more genes selected from the group consisting of LGMN, SPP1, CSTB, LIPA, DAB2, CD63, MAFB, MARCKS, C1QC and HMOX1, is either not upregulated or shows an at least 2-fold downregulated expression of their mRNA when compared to the expression level of their mRNA in an otherwise identical myeloid cell, such as wildtype myeloid cell.
14. The myeloid cell for therapeutic use according to any one of 2 to 13, wherein said fibrosis is idiopathic pulmonary fibrosis (IPF), fibrosis occurring in chronic obstructive pulmonary disease (COPD), interstitial lung disease (ILD), viral infections (severe COVID-19 and pneumonia) and in other diseases such as end-stage liver diseases (including MASH, NASH and liver cirrhosis), autoimmune related diseases (SLE and GvHD) and several cancers (hepatic, pancreatic, gastric, oesophageal, colonic).
15. The myeloid cell for therapeutic use according to any one of 2 to 14, wherein said fibrosis is idiopathic pulmonary fibrosis (IPF).
16. The myeloid cell for therapeutic use according to any one of 1 to 15, wherein the human myeloid cell, and in particular the human macrophage, is resistant to M-CSF induced profibrotic polarization.
17. The myeloid cell for therapeutic use according to any one of 1 to 16, wherein the human myeloid cell, and in particular the human macrophage, is resistant to the induction of a profibrotic gene signature after having been exposed to M-CSF in a range of 50ng/ml to 200 ng/ml for 2 hours, 24 hours, 48 hours, or 72 hours.
18. The myeloid cell for therapeutic use according to any one of 1 to 17, wherein the myeloid cell is genetically modified.
19. The myeloid cell for therapeutic use according to any one of 1 to 18, wherein the myeloid cell is a genetically unmodified myeloid cell.
20. The myeloid cell for therapeutic use according to any one of 1 to 19, wherein the myeloid cell is derived from an iPS cell.
21. The myeloid cell for therapeutic use according to any one of 1 to 20, wherein the myeloid cell is a human myeloid cell.
22. The myeloid cell for therapeutic use according to any one of 1 to 21, wherein the myeloid cell is a myeloid cell according to any one of 23 to 111.
23. A myeloid cell, comprising at least one mutation in both alleles of a gene located on a chromosome, wherein the myeloid cell is resistant to M-CSF induced profibrotic polarization,.
24. The myeloid cell according to 23, wherein the myeloid cell is resistant to M-CSF induced profibrotic polarization after having been exposed to M-CSF in the range of 50ng/ml to 200 ng/ml for a time in the range of 24 hours to 48 hours.
25. The human myeloid cell according to 23 or 24, wherein the myeloid cell is resistant to M-CSF induced profibrotic polarization after having been exposed to 50ng/ml M-CSF for 48 hours.
26. The myeloid cell according to any one of 23 to 25, wherein said myeloid cell shows at least 3-fold decreased expression of at least one mRNA of at least one gene in the myeloid cell in comparison to expression of the mRNA in an otherwise identical myeloid cell, such as wildtype myeloid cell, wherein the at least one gene is selected from the group consisting of PMP22, IGF1, C1QC, C1QA, C1QB, FOLR2, TMEM37, MERTK, MAF, MAFB, RGL1, DAB2, C3AR1, PDPN, CD93, MARCKS, STAB1, CD63, C5AR1, HMOX1, LGMN, PDGFA, CD109, MRC1, ACP2, C5AR2, IER3, and ETS2.
27. The myeloid cell according to any one of 23 to 26, wherein said myeloid cell shows at least 7-fold decreased expression of at least one mRNA of at least one gene in the myeloid cell in comparison to expression of the mRNA in an otherwise identical myeloid cell, such as wildtype myeloid cell, wherein the at least one gene is selected from the group consisting of RGL1, DAB2, C3AR1, PDPN, CD93, MARCKS, STAB1, CD63, C5AR1, HMOX1, LGMN and PDGFA.
28. The myeloid cell according to any one of 23 to 27, wherein said myeloid cell shows at least 10-fold decreased expression of at least one mRNA of at least one gene in the myeloid cell in comparison to expression of the mRNA in an otherwise identical myeloid cell, such as wildtype myeloid cell, wherein the at least one gene is selected from the group consisting of RGL1, DAB2, C3AR1, PDPN, CD93, MARCKS, and CD63.
29. The myeloid cell according to any one of 23 to 28, wherein said myeloid cell shows at least 15-fold decreased expression of at least one mRNA of at least one gene in the myeloid cell in comparison to expression of the mRNA in an otherwise identical myeloid cell, such as wildtype myeloid cell, wherein the at least one gene is selected from the group consisting of RGL1, DAB2, C3AR1, PDPN, and CD93.
30. The myeloid cell according to any one of 23 to 29, wherein said myeloid cell shows at least 30-fold decreased expression of at least one mRNA of at least one gene in the myeloid cell in comparison to expression of the mRNA in an otherwise identical myeloid cell, such as wildtype myeloid cell, wherein the at least one gene is selected from the group consisting of RGL1, DAB2, and C3AR1.
31. The myeloid cell according to any one of 23 to 30, wherein said myeloid cell shows at least 30-fold decreased expression of at least one mRNA of at least one gene in the myeloid cell in comparison to expression of the mRNA in an otherwise identical myeloid cell, such as wildtype myeloid cell, wherein the at least one gene is selected from the group consisting of RGL1, and DAB2.
32. The myeloid cell according to any one of 23 to 31, wherein in said myeloid cell the expression of at least one mRNA is not upregulated in the myeloid cell in comparison to expression of the mRNA in an otherwise identical myeloid cell, such as wildtype myeloid cell, wherein the at least one mRNA is selected from the group consisting of PMP22, IGF1, C1QC, C1QA, C1QB, FOLR2, TMEM37, MERTK, MAF and MAFB.
33. The myeloid cell according to any one of 23 to 31, wherein in said myeloid cell the expression of at least one mRNA is not upregulated or shows an at least 10-fold downregulated expression in the myeloid cell in comparison to expression of the mRNA in an otherwise identical myeloid cell, such as wildtype myeloid cell, wherein the at least one mRNA is selected from the group consisting of DAB2, STAB1, PMP22, IGF1, C1QC, PDPN, C1QA, C3AR1, C1QB and FOLR2.
34. The myeloid cell according to any one of 23 to 31, wherein in said myeloid cell the expression of at least one mRNA is not upregulated or shows an at least 2-fold downregulated expression in the myeloid cell in comparison to expression of the mRNA in an otherwise identical myeloid cell, such as wildtype myeloid cell, wherein the at least one mRNA is selected from the group consisting of LGMN, SPP1, CSTB, LIPA, DAB2, CD63, MAFB, MARCKS, C1QC and HMOX1.
35. The myeloid cell according to any one of 1 to 31, wherein in said myeloid cell the expression level of the C5AR1 mRNA in the myeloid cell is at least 2-fold, for example at least 4-fold, such as at least 6-fold or even at least 9-fold lower than the expression level of C5AR1 mRNA in an otherwise identical myeloid cell, such as wildtype myeloid cell.
36. The myeloid cell according to any one of 1 to 31, wherein the expression level of the SPP1 mRNA in the myeloid cell is at least 2-fold, for example at least 2.3-fold, such as at least 2.6-fold or even at least 2.8-fold lower than the expression level of SPP1 mRNA in an otherwise identical myeloid cell, such as wildtype myeloid cell.
37. The myeloid cell according to any one of 1 to 31, wherein the expression level of the RGI1 mRNA in the myeloid cell is at least 20-fold, for example at least 40-fold, such as at least 60-fold or even at least 80-fold lower than the expression level of RGI1 mRNA in an otherwise identical myeloid cell, such as wildtype myeloid cell.
38. The myeloid cell according to any one of 1 to 31, wherein the expression level of the MRC1 mRNA in the myeloid cell is at least 2-fold, for example at least 3-fold, such as at least 4-fold or even at least 5-fold lower than the expression level of MRC1 mRNA in an otherwise identical myeloid cell, such as wildtype myeloid cell.
39. The myeloid cell according to any one of 1 to 31, wherein the expression level of the MARCKS mRNA in the myeloid cell is at least 6-fold, such as at least 10-fold or even at least 13-fold lower than the expression level of MARCKS mRNA in an otherwise identical myeloid cell, such as wildtype myeloid cell.
40. The myeloid cell according to any one of 1 to 31, wherein the expression level of the LlPA mRNA in the myeloid cell is at least 2-fold, for example at least 2.3-fold, such as at least 2.6-fold or even at least 2.8-fold lower than the expression level of LIPA mRNA in an otherwise identical myeloid cell, such as wildtype myeloid cell.
41. The myeloid cell according to any one of 1 to 31, wherein the expression level of the LGMN mRNA in the myeloid cell is at least 2-fold, for example at least 4-fold, such as at least 6-fold or even at least 8-fold lower than the expression level of LGMN mRNA in an otherwise identical myeloid cell, such as wildtype myeloid cell.
42. The myeloid cell according to any one of 1 to 31, wherein the expression level of the HMOX1 mRNA in the myeloid cell is at least 2-fold, for example at least 4-fold, such as at least 6-fold or even at least 8-fold or 9-fold lower than the expression level of HMOX1 mRNA in an otherwise identical myeloid cell, such as wildtype myeloid cell.
43. The myeloid cell according to any one of 1 to 31, wherein the expression level of the DAB2 mRNA in the myeloid cell is at least 15-fold, for example at least 30-fold, such as at least 45-fold or even at least 60-fold or 65-fold lower than the expression level of DAB2 mRNA in an otherwise identical myeloid cell, such as wildtype myeloid cell.
44. The myeloid cell according to any one of 1 to 31, wherein the expression level of the CTSB mRNA in the myeloid cell is at least 2-fold, for example at least 2-fold, for example at least 2.2-fold, such as at least 2.4-fold or even at least 2.6-fold lower than the expression level of CTSB mRNA in an otherwise identical myeloid cell, such as wildtype myeloid cell.
45. The myeloid cell according to any one of 1 to 31, wherein the expression level of the CD93 mRNA in the myeloid cell is at least 3-fold, for example at least 7-fold, such as at least 11-fold or even at least 15-fold lower than the expression level of CD93 mRNA in an otherwise identical myeloid cell, such as wildtype myeloid cell.
46. The myeloid cell according to any one of 1 to 31, wherein the expression level of the CD63 mRNA in the myeloid cell is at least 3-fold, for example at least 6-fold, such as at least 8-fold or even at least 10-fold lower than the expression level of CD63 mRNA in an otherwise identical myeloid cell, such as wildtype myeloid cell.
47. The myeloid cell according to any one of 1 to 31, wherein the expression level of the C3AR1 mRNA in the myeloid cell is at least 10-fold, for example at least 20-fold, such as at least 30-fold or even at least 40-fold or 44-fold lower than the expression level of C3AR1 mRNA in an otherwise identical myeloid cell, such as wildtype myeloid cell.
48. The myeloid cell according to any one of 1 to 31, wherein the expression level of the PDPN mRNA in the myeloid cell is at least 2-fold, for example at least 7-fold, such as at least 12-fold or even at least 15-fold lower than the expression level of PDPN mRNA in an otherwise identical myeloid cell, such as wildtype myeloid cell.
49. The myeloid cell according to any one of 1 to 31, wherein the expression level of the PDGFA mRNA in the myeloid cell is at least 2-fold, for example at least 4-fold, such as at least 6-fold or even at least 7-fold or 8-fold lower than the expression level of PDGFA mRNA in an otherwise identical myeloid cell, such as wildtype myeloid cell.
50. The myeloid cell according to any one of 1 to 31, wherein the expression level of the IER3 mRNA in the myeloid cell is at least 2-fold, for example at least 2.5-fold, such as at least 3.0-fold or even at least 3.3-fold lower than the expression level of IER3 mRNA in an otherwise identical myeloid cell, such as wildtype myeloid cell.
51. The myeloid cell according to any one of 1 to 31, wherein the expression level of the ETS2 mRNA in the myeloid cell is at least 1.5-fold, for example at least 2-fold, such as at least 2.5-fold or even at least 3-fold lower than the expression level of ETS2 mRNA in an otherwise identical myeloid cell, such as wildtype myeloid cell.
52. The myeloid cell according to any one of 1 to 31, wherein the expression level of the CD109 mRNA in the myeloid cell is at least 1.5-fold, for example at least 1.5-fold, for example at least 3-fold, such as at least 4.5-fold or even at least 5.9-fold lower than the expression level of CD109 mRNA in an otherwise identical myeloid cell, such as wildtype myeloid cell.
53. The myeloid cell according to any one of 1 to 31, wherein the expression level of the C5AR2 mRNA in the myeloid cell is at least 1.5-fold, for example at least 2-fold, for example at least 2.5-fold, such as at least 3.0-fold or even at least 3.5-fold or 3.8-fold lower than the expression level of C5AR2 mRNA in an otherwise identical myeloid cell, such as wildtype myeloid cell.
54. The myeloid cell according to any one of 1 to 31, wherein the expression level of the ACP2 mRNA in the myeloid cell is at least 2-fold, for example at least 3-fold, such as at least 4-fold or even at least 5-fold or 5.4-fold lower than the expression level of ACP2 mRNA in an otherwise identical myeloid cell, such as wildtype myeloid cell.
55. The myeloid cell according to any one of 1 to 54, wherein said myeloid cell does not express the TMEM37 gene.
56. The myeloid cell according to any one of 1 to 54, wherein said myeloid cell does not express the C1QC gene.
57. The myeloid cell according to any one of 1 to 54, wherein said myeloid cell does not express the C1QB gene.
58. The myeloid cell according to any one of 1 to 54, wherein said myeloid cell does not express the PMP22 gene.
59. The myeloid cell according to any one of 1 to 54, wherein said myeloid cell does not express the MERTK gene.
60. The myeloid cell according to any one of 1 to 54, wherein said myeloid cell does not express the IGF1 gene.
61. The myeloid cell according to any one of 1 to 54, wherein said myeloid cell does not express the FOLR2 gene.
62. The myeloid cell according to any one of 1 to 54, wherein said myeloid cell does not express the C1QA gene.
63. The myeloid cell according to any one of 23 to 62, wherein said myeloid cell is a human myeloid cell.
64. The myeloid cell according to any one of 23 to 62, wherein said myeloid cell is a macrophage.
65. The myeloid cell according to any one of 23 to 64, wherein the expression of the at least one mRNA in the myeloid cell comprising at least one mutation in both alleles of a gene located on a chromosome, is at least 50-fold lower in comparison to expression of the same mRNA in an otherwise identical myeloid cell, such as wildtype myeloid cell.
66. The myeloid cell according any one of 23 to 65, wherein the expression of the at least two mRNAs in the myeloid cell comprising at least one mutation in both alleles of a gene located on a chromosome, is at least 50-fold lower in comparison to expression of the same at least three mRNAs in an otherwise identical myeloid cell, such as wildtype myeloid cell.
67. The myeloid cell according to any one of 23 to 66 wherein said mutation renders the gene non-functional.
68. The myeloid cell according to any one of 23 to 67, wherein said mutation inhibits gene expression.
69. The myeloid cell according to any one of 23 to 68, wherein said mutation abolishes gene expression
70. The myeloid cell according to any one of 23 to 69, wherein said mutation is a deletion.
71. The myeloid cell according to 70, wherein said deletion is within a promotor, an exon or a splice site.
72. The myeloid cell according to any one of 70 to 71, wherein said deletion is a deletion of at least 50 base pairs.
73. The myeloid cell according to 23 to 72, comprising at least two different mutations.
74. The myeloid cell according to 73 wherein said two mutations are deletions within at least two different genes.
75. The myeloid cell according to any one of 23 to 74, wherein said gene is MAFB.
76. The myeloid cell according to 75, wherein said mutation is a mutation within the region on human chromosome 22 from 40685000 to 40690000.
77. The myeloid cell according to 76, wherein said mutation is a mutation within the region on human chromosome 22 from 40685200 to 40689800, for example from 40685400 to 40689600, such as from 40685600 to 40689400 and in particular from 40685700 to 40689300.
78. The myeloid cell according to any one of 75 to77, wherein MAFB has been rendered nonfunctional by a deletion of at least 50 base pairs.
79. The myeloid cell according to 78, wherein the deletion is a deletion of at least 100 base pairs.
80. The myeloid cell according to any one of 78 to 79, wherein the deletion is a deletion of at least 250 base pairs.
81. The myeloid cell according to any one of 78 to 80, wherein the deletion is a deletion of from 100 base pairs to 10000 base pairs.
82. The myeloid cell according to 81, wherein the deletion is a deletion of from 500 base pairs to 3000 base pairs, such as from 600 base pairs to 1500 base pairs.
83. The myeloid cell according to any one of 74 to 82, further comprising a mutation in the gene MAF.
84. The myeloid cell according to 83, wherein the mutation in MAF is a deletion.
85. The myeloid cell according to 84, wherein said mutation is a mutation within the region on human chromosome 16 from 79593000 to 79602000.
86. The myeloid cell according to 85, wherein said mutation is a mutation within the region on human chromosome 16 from 79593200 to 79601500, for example from 79593400 to 79601100, and in particular from 79593600 to 79600900.
87. The myeloid cell according to any one of 85 to 86, wherein MAF has been rendered nonfunctional by a deletion of at least 50 base pairs.
88. The myeloid cell according to 87, wherein the deletion is a deletion of at least 100 base pairs.
89. The myeloid cell according to any one of 87 to 88, wherein the deletion is a deletion of at least 250 base pairs.
90. The myeloid cell according to any one of 88 to 89, wherein the deletion is a deletion of from 100 base pairs to 10000 base pairs.
91. The myeloid cell according to 90, wherein the deletion is a deletion of from 500 base pairs to 3000 base pairs, such as from 600 base pairs to 1500 base pairs.
92. The myeloid cell according to any one of 98 to 105, further comprising a mutation in the gene MAFB.
93. The myeloid cell according to 74, wherein said two genes are MAFB and MAF.
94. The myeloid cell according to 93, wherein both alleles of MAFB and both alleles of MAF have been rendered nonfunctional by deletions of at least 50 base pairs.
95. The myeloid cell according to 94, wherein all deletions comprise exonic DNA.
96. The myeloid cell according to any one of 94 to 95, wherein the deletions are at least 100 base pairs each.
97. The myeloid cell according to any one of 94 to 96, wherein the deletions are at least 250 base pairs each.
98. The myeloid cell according to any one of 96 to 97, wherein the deletions are from 100 base pairs to 10000 base pairs each.
99. The myeloid cell according to 98, wherein the deletions are from 500 base pairs to 3000 base pairs, such as from 600 base pairs to 1500 base pairs.
100. The myeloid cell according to any one of 93 to 99, wherein the MAFB deletions are within the region on human chromosome 22 from 40685000 to 40690000.
101. The myeloid cell according to 100, wherein the MAFB deletions are within the region on human chromosome 22 from 40685200 to 40689800, for example from 40685400 to 40689600, such as from 40685600 to 40689400 and in particular from 40685700 to 40689300.
102. The myeloid cell according to any one of 93 to 101, wherein the MAF deletions are within the region on human chromosome 16 from 79593000 to 79602000.
103. The myeloid cell according to 102 wherein the MAF deletions are within the region on human chromosome 16 from 79593200 to 79601500, for example from 79593400 to 79601100, and in particular from 79593600 to 79600900.
104. The myeloid cell according to any one of 1 to 103, wherein the myeloid cell has 46 chromosomes.
105. The myeloid cell according to any one of 1 to 104, wherein the myeloid cell does not contain a chromosome with a chromosome rearrangement.
106. The myeloid cell according to any one of 1 to 105, wherein said gene located on a chromosome comprising biallelic deletions is the only gene comprising biallelic deletions.
107. The myeloid cell according to any one of 1 to 106, wherein said gene located on a chromosome comprising biallelic deletions is the only protein coding gene comprising biallelic deletions.
108. The myeloid cell according to any one of 1 to 107, wherein said gene located on a chromosome comprising biallelic deletions is the transcription-factor encoding gene comprising biallelic deletions.
109. The myeloid cell according to any one of 1 to 108, wherein MAF and/or MAFB is/are the only transcription factor-encoding gene(s) comprising biallelic deletions.
110. The myeloid cell according to any one of 1 to 109, wherein MAF and/or MAFB is/are the only protein-coding gene(s) comprising biallelic deletions.
111. The myeloid cell according to any one of 1 to 110, wherein MAF and/or MAFB is/are the only gene(s) comprising biallelic deletions.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", fourth edition (Sambrook, 2012); "Handbook of Experimental Immunology" (Weir, 1997); "Short Protocols in Molecular Biology" (Ausubel, 2002); "Polymerase Chain Reaction: Principles, Applications and Troubleshooting", (Babar, 2011); "Current Protocols in Immunology" (Coligan, 2002). These techniques may be considered in making and practicing the invention.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

Various references are cited throughout this specification, each of which is incorporated herein by reference in its entirety.

The foregoing description will be more fully understood with reference to the following Examples. Such Examples, are, however, exemplary of methods of practicing the present invention and are not intended to limit the scope of the invention.

### EXAMPLES

The following examples are provided so as to describe to the skilled artisan how to make and use methods and compositions described herein and are not intended to limit the scope of the present disclosure.

### Materials and methods

### Animals

Myeloid Maf-DKO (MafB^{flox/flox}/cMaf^{flox/flox}LysM^{Cre/+}) and WT (MafB^{flox/flox}/cMaf^{flox/flox}) mice were generated by breeding of indicated mouse strains and male mice of 4 to 6 months of age were used for all experiments. MafB flox and cMaf flox mice were described previously (Wende et al., 2012; Matcovitch-Natan et al., 2016). LysMCre knock in mice were obtained from Jackson Laboratories (Clausen et al., 1999). Maf-DKO and WT littermates were obtained by crossing MafB^{flox/flox}/cMaf^{flox/flox} with MafB^{flox/flox}cMaf^{flox/flox}LysM^{Cre/+} mice. C57BL/6 CD45.2 and CD45.1 male mice were obtained from Charles River. Mice were housed under specific pathogen-free conditions in individually ventilated cages in a controlled 12-hour reverse light/dark cycle and were provided with food and water ad libitum. In vivo procedures were performed following the protocols approved by the ethics committee Nº014 in accordance with institutional, national and European regulations (approval number: #3292-2015122109359224, #26802-2020073118362710)

### Bleomycin induced lung fibrosis

4-6 months old, male Maf-DKO and WT control mice were used for bleomycin induced fibrosis experiments. Mice were briefly anesthetized using isoflurane 4% and oropharyngeally administered bleomycin sulphate (1.3-1.5mg/kg) or sterile PBS in a final volume of 50µl. In case of batch/lot variability, the bleomycin sulphate (Merck or Sigma) was freshly titrated using 3 months old C57BI/6J WT male mice to obtain a comparable response of 80-85% weight loss at day14. After oropharyngeal instillation the mice were closely monitored until complete recovery from anaesthesia and then returned to their cages for routine care and handling. The animals were monitored daily and sacrificed after 14 days.

### Generation of bone marrow monocyte derived macrophages (BM-MDM)

Total bone marrow cell suspensions were obtained from myeloid Maf-DKO or WT) mice. Briefly, bones were harvested and flushed with cold PBS to obtain total bone marrow cell suspension. Red blood cell lysis was performed using Haemolysis buffer (Morphisto). The resulting single cell suspension was washed and counted and processed with the EasySep^{™} Mouse Monocyte Isolation kit (STEM CELL Technologies) according to manufacturer's protocol to obtain an enriched population of monocytes that were further characterized by flow cytometry to be roughly 90-95% classical (Ly6c+) and 5-10% non-classical monocytes (Ly6c-). These enriched monocytes were then pooled and seeded at 0.5 - 1 million cells/ml in CERO 3D incubator and bioreactor (OMNI Life sciences) using DMEM basal medium (DMEM, 10% FCS, 1% Penicillin/Streptomycin, 1% Glutamax, 1% Na-Pyr) supplemented with of 200 ng/ml recombinant M-CSF and cultivated as a suspension culture under 37 ºC and 5% CO₂. The medium was replaced every 5 days and the cells were amplified and harvested at indicated timepoints for analysis.

### Single-cell RNA sequencing of mouse monocytes cultured in M-CSF

scRNA-seq dataset was generated using a pool of 2 donors per genotype (WT and Maf-DKO) that were age and sex matched. 1 million cells of freshly harvested bone marrow enriched monocytes or enriched monocytes after 24h of 3D suspension culture in presence of M-CSF were then harvested and fixed following the Chromium fixed RNA profiling protocol (10x Genomics). The fixed samples were further processed using Chromium Fixed RNA kit (mouse). Prepared libraries were then sequenced using the Illumina NovaSeq S4 system targeting a read depth of 400 million reads/cell.

The initial quality controls steps including alignment, filtering, barcode and UMI counting was performed using the 10x cell ranger multi pipeline v7.1.0. Further quality control for doublet identification, removal of mitochondrial gene fraction, low quality cells, principal component analysis and initial UMAP visualization was performed using Scanpy that resulted in a total of 44 493 cells (ca. 10 000 cells per genotype + treatment condition). The differential expression of genes before and after 24h M-CSF exposure was performed for each genotype on unclustered cells using Scanpy. A lenient cutoff for significant genes with a log2FC of 1 and P-value =< 0.05 was used to include small and meaningful changes in gene expression in the analysis. The human orthologs of these genes were generated using pyorthomap and pybiomart packages using the ENSEMBL database.

Profibrotic genes from 15 published scRNAseq datasets of mouse and human fibrosis were collected for 20 defined profibrotic macrophage clusters within each dataset. The list was filtered further based on genes detected in the dataset and only genes with human orthologs were considered for generating a ranked list of profibrotic genes based on number of occurrences within the reference datasets.

### Bulk and single cell RNA sequencing of human macrophages:

iPS-cell derived MAF-DKO macrophages and WT macrophages obtained from EB-differentiation cultures essentially as described **in example 8** were harvested on day 20 post EB-seeding by collecting the cells in suspension. For bulk RNA sequencing, 500000 cells per well were replated in 6 well plates (3ml medium per well) and then kept for 5 days for final differentiation in the presence of M-CSF and GM-CSF (essentially as described in **example 8**; partial medium change for fresh medium every second day). On day 5, cells were collected and replated in complete medium (incl. M-CSF and GM-CSF, as described in **example 8**) in Nunclon 12-well plates at 100000 cells per well, and kept for two more days (2ml medium per well). On day 7 after harvest of the suspension cells the medium was changed for fresh complete medium (incl. M-CSF and GM-CSF, both at 50 ng/ml). Cells were then harvested 2 hours after medium change - 10000 DAPI-negative, CD45 positive, CD11b positive cells were sorted directly into RLT plus lysis buffer and RNA was extracted with the RNeasy micro kit (Quiagen, Cat. No. 74034). Differences in gene expression patterns were then analyzed by RNAseq, as described in detail (Picelli et al., 2013) with RNA isolated from 10000 cells, sequencing depth of 38 million fragments per library. Alignment of the fragments to the human reference (hg38) was done with GSNAP (v2020-12-16; Wu et al., 2005; Wu et al., 2010) and Ensembl gene annotation 98 (Howe et al., 2021) was used to detect splice sites. The uniquely aligned fragments were counted with featureCounts (2.0.1; Liao et al., 2014) and the support of the same Ensembl annotation. Simultaneously, sequenced libraries underwent a quality control with RNA-SeQC (1.1.8; DeLuca et al., 2012) which includes exonic, intronic and intergenic distribution of the reads and rRNA rate within each library. Normalization of the raw fragments counts based on the library size and testing for differential expression between the two conditions DKO 2h and WT 2h was performed with the DESeq2 (v1.24.0; Love et al., 2014) and IHW (1.12.0; Ignatiadis et al., 2016; Ignatiadis and Huber, 2017) package in R (3.6.3; [R Core Team (2020). R: A language and environment for statistical computing. R Foundation for Statistical Computing, Vienna, Austria. URL https://www.R-project.org/]). Genes, which have an adjusted p value (padj) < 0.05 and/or log2FoldChange > 0.58 or < -0.58 were considered as differentially expressed.

For single cell RNA sequencing, about 800,000 iPSC-cell derived MAF-DKO and WT macrophages cultured in M-CSF containing medium as mentioned above were harvested on Day 17 post EB seeding by collecting the cells in suspension. The cells were fixed following the Chromium fixed RNA profiling protocol (10x Genomics). The fixed samples were further processed using Chromium Fixed RNA kit (human). Prepared libraries were then sequenced using the Illumina NovaSeq S4 system targeting a read depth of 400 million reads/cell and processed for downstream analysis following the in-house pipeline.

Statistical analysis: All experiments were performed in at least duplicates and representative data are shown. Results were tested for statistical significance using Student's t test or two-way annova and multiple comparison testing using GraphPad prism software.

### RESULTS

### Example 1

### Myeloid Maf-DKO mice are less susceptible to pulmonary fibrosis

In order to assess the role of self-renewing Maf-DKO macrophages and their regenerative impact in fibrosis in vivo, a myeloid specific conditional deletion of the transcription factors MafB and cMaf was generated (myeloid Maf-DKO), whereby myeloid cells expressing the lysosomal gene Lyz2, such as granulocytes, monocytes and macrophages delete MafB and cMaf genes. Since expression of MafB and cMaf is specifically high in most tissue resident macrophages and when recruited monocytes differentiate to macrophages, this deletion will affect macrophages.

The role of Maf-DKO macrophages in the development of lung fibrosis was investigated in the mouse model of bleomycin induced pulmonary fibrosis (IPF) over the course of 14 days, the peak of fibrotic disease. Several studies have shown in experimental mouse models of bleomycin induced pulmonary fibrosis (Strunz et al., 2020; Aran et al., 2019) and in human lung fibrosis (Reyfman et al., 2019) that MafB and cMaf were highly upregulated in broncho-alveolar myeloid cells. The example shows that Maf-DKO mice were less susceptible to the weight loss associated with bleomycin-induced fibrosis, displaying only a moderate weight loss over 14 days in comparison to WT bleomycin challenged mice. A group of severely diseased animals, which showed a loss of more than 10% of their original body weight at Day14 represented 78% of WT mice versus only 30% of Maf-DKO mice **(****Figure 1****).** Examination of lung tissue samples using gross macroscopic high resolution lung images showed a drastic reduction in fibrotic areas **(****Figure 2****)** and collagen deposition **(****Figure 3****)** in the myeloid Maf-DKO bleomycin challenged lungs compared to WT bleomycin challenged mice.

### Example 2

### Recruited lung Maf-DKO macrophages are resistant to profibrotic reprogramming

Several recent studies have shown that recruited macrophages drive fibrosis progression in the lung by upregulating several profibrotic genes (Misharin et al., 2017; Aran et al., 2019, Joshi et al., 2020). Using whole lung scRNAseq on WT and Maf-DKO mice treated with bleomycin or PBS the example evidences most dramatic gene expression changes in monocyte-derived macrophages of the lungs **(****Figure 4****).** In order to identify broadly relevant and common macrophage fibrosis genes of those changes a list of fibrotic macrophage gene signatures was curated from 15 publications of mouse and human data sets from lung fibrosis, liver fibrosis, Cancer and COVID-19 related fibrosis that described 20 profibrotic macrophage subtypes (Strunz et al., 2019; Aran et al., 2019; Joshi et al., 2020; Xie et al., 2018; Morse et al., 2019; Reyfman et al., 2019; Ayaub et al., 2021; Wendisch et al., 2021; Liao et al., 2020; Grant et al., 2021; Bharat et al., 2020; Sikkema et al., 2023; Tran et al., 2020; Remmerie et al., 2020; Fabre et al., 2023). The example shows a strong reduction of fibrotic gene signatures in Maf-DKO compared to WT recruited macrophages **(****Figure 4****).** Therefore, the example demonstrates that Maf-DKO monocytes and macrophages have general utility and therapeutic relevance for fibrosis.

PDGF secretion by recruited macrophages and M-CSF secretion by activated fibroblasts or myofibroblasts in the fibrotic niche has been shown to lead to mutual cross-stimulation of these cell types and a feed-forward loop that drives fibrosis (Zhou et al., 2018; Joshi et al., 2020; Adler et al., 2020; Buechler et al., 2021). The example evidences that Maf-DKO monocyte-derived macrophages in bleomycin treated mice **(****Figure 5****).** lack a strong induction of the Pdgfa gene compared to WT cells, indicating that Maf-DKO monocyte-derived macrophages remain non-responsive to M-CSF upon entering a fibrotic niche and fail to secrete PDGFA, thus rupturing the positive feed forward loop with fibrosis driving myofibroblasts.

### Example 3

### Maf-DKO monocyte transplantation alleviates bleomycin induced pulmonary fibrosis in WT mice.

Since gene expression data revealed dramatic changes of profibrotic gene expression in WT but not Maf-DKO macrophages derived from recruited monocyte during bleomycin induced fibrosis, the effect of transplanting Maf-DKO monocytes intravenously was analyzed. It is shown by the invention that transplanted Maf-DKO monocytes alleviate bleomycin induced pulmonary fibrosis in WT mice. Maf-DKO monocytes were intravenously administered every 2-3 days up to 14 days into WT mice after bleomycin induced fibrosis. It was observed that mice treated with Maf-DKO monocytes showed a significant reduction in weight loss compared to vehicle (PBS) treated control mice, indicating a strong improvement of disease severity (**Figure 6**). The example thus demonstrates that intravenously administered therapeutic Maf-DKO monocytes have a dominant beneficial effect on fibrotic disease development despite the presence of endogenous WT monocytes and macrophages. The example also demonstrates a therapeutic state of a monocyte-derived macrophages that can resolve fibrotic disease.

### Example 4

### Maf-DKO monocyte derived macrophages fail to upregulate profibrotic gene signatures in response to M-CSF

To characterize the nature of a therapeutic fibrosis resolving monocyte derived macrophage the gene expression of Maf-DKO monocytes and macrophages was analyzed by scRNAseq. The cytokine M-CSF is induced in fibrotic disease (Joshi et al., 2020), including in the lung, and leads to activation of fibrotic genes in recruited monocyte derived macrophages (Joshi et al., 2020). Therefore WT and Maf-DKO monocytes isolated from bone marrow cell suspensions were analyzed before and after stimulation with 200ng/ml M-CSF for 24 hours. It was observed that fresh WT and Maf-DKO monocytes were transcriptionally highly similar but Maf-DKO monocyte derived macrophages showed a highly distinct transcriptional response to M-CSF exposure **(****Figure 7****).**

It was observed that therapeutic fibrosis resolving Maf-DKO monocyte derived macrophages have a characteristic reaction highly distinct from WT monocyte derived macrophages to the fibrosis inducing cytokine M-CSF **(****Figure 7****).** The example demonstrates that therapeutic fibrosis resolving Maf-DKO monocyte derived macrophages fail to upregulate characteristic profibrotic genes. These profibrotic genes that were not upregulated by M-CSF in in therapeutic fibrosis resolving Maf-DKO monocyte derived macrophages were further characterized and ranked by different approaches: either by the fold difference between WT and Maf-DKO upregulation **(Table 2),** by fold up-regulation in WT monocyte derived macrophages in response to M-CSF **(see Table 3)** or by the number of times listed in profibrotic signatures in the literature selected from the following publicartions (Strunz et al., 2019; Aran et al., 2019; Joshi et al., 2020; Xie et al., 2018; Morse et al., 2019; Reyfman et al., 2019; Ayaub et al., 2021; Wendisch et al., 2021; Liao et al., 2020; Grant et al., 2021; Bharat et al., 2020; Sikkema et al., 2023; Tran et al., 2020; Remmerie et al., 2020; Fabre et al., 2023). Failure of Maf-DKO monocyte derived macrophages to induce genes from these ranked fibrotic gene signatures is shown for the indicated gene sets in violin plots (**Figure 8****)** or for individual prominent examples (**figure 9****).** This included the failure of Maf-DKO monocyte derived macrophages to upregulate PDGFA after exposure to M-CSF, indicating that the effect observed in the profibrotic niche of bleomycin induced fibrotic lungs (**example 2,** **Figure 5****)** is inherent to Maf-DKO monocyte derived macrophages

**Table 2: Ordered list of genes with at least 3-fold difference of gene upregulation in WT compared to Maf-DKO monocyte derived macrophages 24 hours after M-CSF exposure.**

| **Mouse gene name** | **Human gene name** | **logfold changes WT** | **logfold changes DKO** | **fold change WT** | **fold change DKO** | **fold difference WTvs DKO** | **Fibrosis importance** |
|---|---|---|---|---|---|---|---|
| Clqc | C1QC | 9,91026 | 0 | 961,2446936 | 0 | >961.2446936 | 6 |
| Clqa | C1QA | 9,721919 | 0 | 843,4797392 | 0 | >843.4797392 | 5 |
| Maf | MAF | 5,4850545 | 0 | 43,78843962 | 0 | >43.78843962 | 3 |
| Clqb | C1QB | 8,641639 | 0 | 398,3857366 | 0 | >398.3857366 | 5 |
| Folr2 | FOLR2 | 8,109606 | 0 | 275,2069861 | 0 | >275.2069861 | 4 |
| Mafb | MAFB | 4,48253 | 0 | 21,35506755 | 0 | >21.35506755 | 8 |
| Tmem37 | TMEM37 | 7,2376137 | 0 | 149,9172284 | 0 | >149.9172284 | 4 |
| Mertk | MERTK | 7,064787 | 0 | 132,8791049 | 0 | >132.8791049 | 5 |
| Pmp22 | PMP22 | 10,154239 | 0 | 1138,542487 | 0 | >1138.542487 | 5 |
| Igf1 | IGF1 | 10,054178 | 0 | 1062,185781 | 0 | >1062.185781 | 3 |
| Rgl1 | RGL1 | 5,18017 | 0,50897217 | 35,25655637 | 0,423036 | 83,3417381 | 2 |
| Dab2 | DAB2 | 13,522304 | 7,515356 | 11764,73641 | 181,95639 | 64,65690137 | 8 |
| C3ar1 | C3AR1 | 8,79058 | 3,4601576 | 441,8210519 | 10,005537 | 44,15765632 | 5 |
| Pdpn | PDPN | 9,846049 | 5,903653 | 919,3565083 | 58,865503 | 15,61791642 | 3 |
| Cd93 | CD93 | 2,7168856 | 0,44143537 | 5,574520144 | 0,3579547 | 15,57325523 | 2 |
| Marcks | MARCKS | 2,0065484 | -0,37247956 | 3,018197288 | 0,2275463 | 13,26410407 | 8 |
| Stab1 | STAB1 | 10,397152 | 6,6925025 | 1347,511398 | 102,4294 | 13,15551413 | 4 |
| Cd63 | CD63 | 5,5294867 | 2,4267333 | 45,18929753 | 4,3767459 | 10,32486196 | 8 |
| C5ar1 | C5AR1 | 6,2337584 | 3,1873493 | 74,25723606 | 8,1093575 | 9,156981444 | 0 |
| Hmox1 | HMOX1 | 3,66043 | 1,1980253 | 11,64442914 | 1,2942543 | 8,997018057 | 6 |
| Lgmn | LGMN | 3,7792485 | 1,3242717 | 12,72989322 | 1,5040645 | 8,463662009 | 15 |
| Pdgfa | PDGFA | 4,523742 | 1,9097288 | 22,00287051 | 2,7573846 | 7,979616052 | 0 |
| Cd 109 | CD109 | 2,5529795 | 0,865528 | 4,868449988 | 0,8220064 | 5,922642681 | 0 |
| Mrc1 | MRC1 | 2,2419126 | -1,5072478 | 3,730237429 | 0,6482183 | 5,754600315 | 2 |
| Acp2 | ACP2 | 3,241463 | 1,3574528 | 8,457527066 | 1,5623238 | 5,413427784 | 5 |
| C5ar2 | C5AR2 | 2,6992953 | 1,2840477 | 5,494845918 | 1,4352126 | 3,828593829 | 0 |
| ler3 | IER3 | 3,122974 | 1,7356384 | 7,711819118 | 2,3302683 | 3,309412571 | 3 |
| Ets2 | ETS2 | 3,1548138 | 1,8506634 | 7,906223399 | 2,6066599 | 3,033085865 | 0 |

| | | | | | | | |
|---|---|---|---|---|---|---|---|
| In the tables: **Log fold change** relates to log2 scaled fold change values compared to control. WT and Maf-DKO monocyte derived macrophages 24h after M-CSF exposure compared to corresponding unstimulated control monocytes. **Fold change** relates to fold difference in gene expression compared to control. WT and Maf-DKO monocyte derived macrophages 24h after M-CSF exposure compared to corresponding unstimulated control monocytes. Calculated as (2^log2FC)-1 for positively regulated genes and 1-(2^log2FC) for negatively regulated genes. Genes that were not differentially expressed in Maf-DKO monocyte derived macrophages 24h after M-CSF exposure were indicated with a value of "0". The fold change difference of these genes calculated as WT vs Maf-DKO must therefore be a value higher than the fold change of the gene induced in WT upon M-CSF and indicated with a value of ">fold change in WT". **Fold difference WT vs DKO** relates to the ratio of fold difference WT vs Maf-DKO calculated as (Fold difference in WT post M-CSF/Fold difference in Maf-DKO post M-CSF). **Fold difference WT vs DKO** relates to the ratio of fold difference WT vs Maf-DKO calculated as (Fold difference in WT post M-CSF/Fold difference in Maf-DKO post M-CSF) | | | | | | | |

When ranked based on the RNAseq results in Maf-DKO monocytes post M-CSF, the top genes that are not upregulated in Maf-DKO monocyte derived macrophages post M-CSF treatment are selected from the group consisting of PMP22, IGF1, C1QC, C1QA, C1QB, FOLR2, TMEM37, MERTK, MAF and MAFB and the top DEGs that are less expressed in Maf-DKO monocytes post M-CSF treatment are selected from the group consisting of RGL1, DAB2, C3AR1, PDPN, CD93, MARCKS, STAB1, CD63, C5AR1, HMOX1, LGMN and PDGFA **(****Figure 8** **and** **9****).**

When ranked on the basis of top upregulated genes in WT monocytes upon M-CSF treatment **(Table 3)** genes are selected from the group consisting of DAB2, STAB1, PMP22, IGF1, C1QC, PDPN, C1QA, C3AR1, C1QB and FOLR2 **(****Figure 8** **and** **9****).**

**Table 3: Ordered List of genes with at least Log2FC>3 in WT monocytes upon 24 hours M-CSF treatment**

| **Mouse gene name** | **Human gene name** | **logfold changes WT** | **logfold changes DKO** | **fold change WT** | **fold change DKO** | **fold difference WTvs DKO** | **Fibrosis importance** |
|---|---|---|---|---|---|---|---|
| Dab2 | DAB2 | 13,522304 | 7,515356 | 11764,73641 | 181,9563907 | 64,65690137 | 8 |
| Stab1 | STAB1 | 10,397152 | 6,6925025 | 1347,511398 | 102,4293984 | 13,15551413 | 4 |
| Pmp22 | PMP22 | 10,154239 | 0 | 1138,542487 | 0 | >1138.542487 | 5 |
| Igf1 | IGF1 | 10,054178 | 0 | 1062,185781 | 0 | >1062.185781 | 3 |
| Clqc | C1QC | 9,91026 | 0 | 961,2446936 | 0 | >961.2446936 | 6 |
| Pdpn | PDPN | 9,846049 | 5,903653 | 919,3565083 | 58,86550315 | 15,61791642 | 3 |
| Clqa | C1QA | 9,721919 | 0 | 843,4797392 | 0 | >843.4797392 | 5 |
| C3ar1 | C3AR1 | 8,79058 | 3,4601576 | 441,8210519 | 10,00553672 | 44,15765632 | 5 |
| Clqb | C1QB | 8,641639 | 0 | 398,3857366 | 0 | >398.3857366 | 5 |
| Folr2 | FOLR2 | 8,109606 | 0 | 275,2069861 | 0 | >275.2069861 | 4 |
| Tmem37 | TMEM37 | 7,2376137 | 0 | 149,9172284 | 0 | >149.9172284 | 4 |
| Mertk | MERTK | 7,064787 | 0 | 132,8791049 | 0 | >132.8791049 | 5 |
| C5ar1 | C5AR1 | 6,2337584 | 3,1873493 | 74,25723606 | 8,10935749 | 9,156981444 | 0 |
| Cmklr1 | CMKLR1 | 6,2238836 | 4,7526913 | 73,7438825 | 25,9589294 | 2,840790595 | 3 |
| Cd 63 | CD63 | 5,5294867 | 2,4267333 | 45,18929753 | 4,376745927 | 10,32486196 | 8 |
| Maf | MAF | 5,4850545 | 0 | 43,78843962 | 0 | >43.78843962 | 3 |
| Rgl1 | RGL1 | 5,18017 | 0,50897217 | 35,25655637 | 0,42303601 | 83,3417381 | 2 |
| Pdgfa | PDGFA | 4,523742 | 1,9097288 | 22,00287051 | 2,757384612 | 7,979616052 | 0 |
| Mafb | MAFB | 4,48253 | 0 | 21,35506755 | 0 | >21.35506755 | 8 |
| Lgmn | LGMN | 3,7792485 | 1,3242717 | 12,72989322 | 1,504064459 | 8,463662009 | 15 |
| Hmox1 | HMOX1 | 3,66043 | 1,1980253 | 11,64442914 | 1,294254282 | 8,997018057 | 6 |
| Ctsd | CTSD | 3,6070423 | 2,410787 | 11,18506718 | 4,317643276 | 2,590549164 | 5 |
| Spp1 | SPP1 | 3,5630553 | 2,2586439 | 10,81915754 | 3,785414525 | 2,858116983 | 14 |
| Acp2 | ACP2 | 3,241463 | 1,3574528 | 8,457527066 | 1,5623238 | 5,413427784 | 5 |
| Ets2 | ETS2 | 3,1548138 | 1,8506634 | 7,906223399 | 2,606659933 | 3,033085865 | 0 |
| ler3 | IER3 | 3,122974 | 1,7356384 | 7,711819118 | 2,330268274 | 3,309412571 | 3 |

Further, it was observed that genes that were upregulated by M-CSF in WT monocyte derived macrophages were identified that were found In profibrotic gene signatures described in over 15 relevant reference fibrosis datasets that defined 20 profibrotic macrophage clusters using scRNAseq **(see Table 4).** When ranked on the basis of the number of occurrences in the 20 defined profibrotic macrophage clusters in literature, the top differentially expressed genes (DEGs) expressed in WT monocyte derived macrophages 24h after M-CSF exposure are selected from the group consisting of LGMN, SPP1, CSTB, LIPA, DAB2, CD63, MAFB, MARCKS, C1QC and HMOX1 **(****Figure 8** **and** **9****).**

**Table 4: Ordered List of genes with at least Log2FC>3 in WT monocyte derived macrophages 24 hours after M-CSF exposure.**

| **Mouse gene name** | **Human gene name** | **logfold changes WT** | **logfold changes DKO** | **fold change WT** | **fold change DKO** | **fold difference WT vs DKO** | **Fibrosis importance** |
|---|---|---|---|---|---|---|---|
| Lgmn | LGMN | 3,779249 | 1,324272 | 12,7298932 | 1,50406446 | 8,463662009 | 15 |
| Spp1 | SPP1 | 3,563055 | 2,258644 | 10,8191575 | 3,78541452 | 2,858116983 | 14 |
| Cstb | CSTB | 2,541734 | 1,496364 | 4,82288449 | 1,82130688 | 2,648035068 | 11 |
| Lipa | LIPA | 2,290674 | 1,492452 | 3,89284541 | 1,8136674 | 2,146394319 | 10 |
| Dab2 | DAB2 | 13,5223 | 7,515356 | 11764,7364 | 181,956391 | 64,65690137 | 8 |
| Cd63 | CD63 | 5,529487 | 2,426733 | 45,1892975 | 4,37674593 | 10,32486196 | 8 |
| Mafb | MAFB | 4,48253 | 0 | 21,3550675 | 0 | >21.35506755 | 8 |
| Marcks | MARCKS | 2,006548 | -0,37248 | 3,01819729 | 0,22754626 | 13,26410407 | 8 |
| C1qc | C1QC | 9,91026 | 0 | 961,244694 | 0 | >961.2446936 | 6 |
| Hmox1 | HMOX1 | 3,66043 | 1,198025 | 11,6444291 | 1,29425428 | 8,997018057 | 6 |
| Pmp22 | PMP22 | 10,15424 | 0 | 1138,54249 | 0 | >1138.542487 | 5 |
| C1qa | C1QA | 9,721919 | 0 | 843,479739 | 0 | >843.4797392 | 5 |
| C3ar1 | C3AR1 | 8,79058 | 3,460158 | 441,821052 | 10,0055367 | 44,15765632 | 5 |
| C1qb | C1QB | 8,641639 | 0 | 398,385737 | 0 | >398.3857366 | 5 |
| Mertk | MERTK | 7,064787 | 0 | 132,879105 | 0 | >132.8791049 | 5 |
| Ctsd | CTSD | 3,607042 | 2,410787 | 11,1850672 | 4,31764328 | 2,590549164 | 5 |
| Acp2 | ACP2 | 3,241463 | 1,357453 | 8,45752707 | 1,5623238 | 5,413427784 | 5 |
| Stab1 | STAB1 | 10,39715 | 6,692503 | 1347,5114 | 102,429398 | 13,15551413 | 4 |
| Folr2 | FOLR2 | 8,109606 | 0 | 275,206986 | 0 | >275.2069861 | 4 |
| Tmem37 | TMEM37 | 7,237614 | 0 | 149,917228 | 0 | >149.9172284 | 4 |
| Igf1 | IGF1 | 10,05418 | 0 | 1062,18578 | 0 | >1062.185781 | 3 |
| Pdpn | PDPN | 9,846049 | 5,903653 | 919,356508 | 58,8655032 | 15,61791642 | 3 |
| Cmklr1 | CMKLR1 | 6,223884 | 4,752691 | 73,7438825 | 25,9589294 | 2,840790595 | 3 |
| Maf | MAF | 5,485055 | 0 | 43,7884396 | 0 | >43.78843962 | 3 |
| ler3 | IER3 | 3,122974 | 1,735638 | 7,71181912 | 2,33026827 | 3,309412571 | 3 |

### Example 5

### Maf-DKO monocyte derived macrophages fail to upregulate profibrotic surface markers upon M-CSF exposure

Further, the nature of a therapeutic fibrosis resolving monocyte was further characterized by flow cytometry (FACS), evaluating specific macrophage and fibrosis associated cell surface markers 66 hours after M-CSF stimulation in WT and Maf-DKO monocyte derived macrophages. Whereas no significant difference was observed for the M-CSF receptor (CD115) monocyte/macrophage specific surface marker, Maf-DKO monocytes failed to upregulate profibrotic FOLR2 and MERTK cell surface markers 66 hours after M-CSF stimulation. The invention thus discloses a therapeutic fibrosis resolving monocyte that fails to induce profibrotic FOLR2 and MERTK cell surface markers upon M-CSF exposure **(****Figure 11****).**

### Example 6

### Generation of human MAF-DKO iPS cells

To further characterize the nature of therapeutic fibrosis resolving monocytes and macrophages in humans, human MAF-DKO iPS cells deficient for MAFB and MAF were generated for further differentiation to monocytes and macrophages (example 7).

### a) iPSC generation

Methods for the generation of human induced pluripotent stem cells are well known in the art. A human iPS cell line was prepared from dermal fibroblasts obtained from the prepuce of the penis of a healthy human neonate male. Reprogramming was with non-integrating Sendai virus containing POU5F1, SOX2, KLF4 and MYC (Fusaki et al., 2009). Virus screening for HIV1, HIV2, hepatitis virus B and C and mycoplasm was negative. Karyotypic showed a normal 46 (X,Y) karyotype. Into this cell line a doxycycline-inducible Cas9-expression cassette was introduced into the AAVS1 locus through TALEN-mediated gene targeting, essentially as described in González et al., 2014, providing cell line BIHi001-A-1.

### b) iPSC culture

iPSCs were cultured under feeder-free conditions in StemFlex Medium (Thermo Fisher, #A3349401) on vitronectin-coated (Thermo Fisher, # A14700) tissue-culture plates at 37°C, 5% CO2 under normoxic conditions. Cells were passaged with ReLeSR (Stem Cell Technologies, #05872) when reaching 60-80% confluency and maintained in the presence of 10 µM of the ROCK inhibitor Y-27632 (biomol, #Cay10005583) on the day after passaging. Single-cell suspensions of iPSC were obtained by incubation with Accutase (Merck Millipore, #SF006).

### c) Genome editing

The CRISPR/Cas9 system was used to delete the coding sequence of MAF (Gene ID: 4094, 373 amino acids) and MAFB (Gene ID: 9935, 323 amino acids) in human cells. Towards this end, a healthy-donor-derived iPSC line harbouring a doxycycline-inducible Cas9 expression cassette integrated into the AAVS1 locus (BIHi001-A-1, https://hpscreg.eu/cell-line/BIHi001-A-1; also called iBCRT Cas9v1-3G-KI.16) with sgRNA-expressing plasmids targeting MAF and MAFB (pU6-(Bbsl)sgRNA_CAG-venus-bpA, Addgene ID 86985, https://www.addgene.org/86985/) was transfected according to a published protocol (Yumlu et al., 2017).

To delete the complete CDS, 2 sgRNAs per gene were designed to target a sequence in the 5'UTR at least 20 nt upstream of the start codon and in the 3'UTR at least 20 nt downstream of the stop codon of each gene using the CrispRGold program for sequence design (https://crisprgold.mdc-berlin.de/; Chu et al., 2016). The 2 sgRNAs for each gene were expressed together from the sgRNA-expression vector pU6-(Bbsl)sgRNA_CAG-venus-bpA, which also encodes a fluorescent reporter gene (Venus, derived from YFP) for positive selection of transfected cells by FACS.

**Table 1: sgRNA and protospacer sequences for CRISPR/Cas9-mediated deletion of MAF and MAFB CDS. The position of the target-specific, 20-nucleotide long protospacer is marked by "N" within the sgRNA sequence. The genomic target sequences of the sgRNAs expressed from pU6-(Bbsl)sgRNA_CAG-venus-bpA with the indicated protospacer sequences are located in the 5'UTR and 3'UTR, respectively, of MAF and MAFB. UTR, untranslated region.**

| **sgRNA** | | |
|---|---|---|
| NNNNNNNNNNNNNNNNNNNNGTTTTAGAGCTAGAAATAGCAAGTTAAAATAAGGCTAGTCCGTTAT CAACTTGAAAAAGTGGCACCGAGTCGGTGCTTT | | |
| | | |

| **Locus** | **Protospacer sequence for 5'UTR** | **Protospacer sequence for 3'UTR** |
|---|---|---|
| MAF | ATCTGGCGGAGCGGCGGCGG | ACCCTGATAAGTGCTCCGCG |
| MAFB | CGGCCGCAAAGTTTCCCGGG | TGACCTGTTTGACTTGAGCG |

SEQ ID No.1 (MAF-5'UTR): 
5'ATCTGGCGGAGCGGCGGCGGGTTTTAGAGCTAGAAATAGCAAGTTAAAATAAGGCTAGTCCGTTATCAA
CTTGAAAAAGTGGCACCGAGTCGGTGCTTT-3'
SEQ ID No.2 (MAF-3'UTR): 
5'ACCCTGATAAGTGCTCCGCGGTTTTAGAGCTAGAAATAGCAAGTTAAAATAAGGCTAGTCCGTTATCAAC
TTGAAAAAGTGGCACCGAGTCGGTGCTTT-3'
SEQ ID No.3 (MAFB-5'UTR): 
5'CGGCCGCAAAGTTTCCCGGGGTTTTAGAGCTAGAAATAGCAAGTTAAAATAAGGCTAGTCCGTTATCAA
CTTGAAAAAGTGGCACCGAGTCGGTGCTTT-3'
SEQ ID No.4 (MAFB-3'UTR):

5'TGACCTGTTTGACTTGAGCGGTTTTAGAGCTAGAAATAGCAAGTTAAAATAAGGCTAGTCCGTTATCAAC
TTGAAAAAGTGGCACCGAGTCGGTGCTTT-3'

Using Lipofectamine 3000 (Thermo Fisher, #L3000001), BIHi001-A-1 was transfected with sgRNA expression vectors together with pCAG-mTrex2-bpA (Addgene ID 86984, ) to enhance the propensity of indel formation. Control cells, labelled with "WT" or "wild-type" throughout the text, were treated the same way except for using the pU6-(Bbsl)sgRNA_CAG-venus-bpA without inserting any protospacer sequence. Transfected BIHi001-A-1 cells were treated with 1 µg/m! doxycycline hyclate (Sigma-Aldrich, #D9891) to induce Cas9 expression, followed by FACS-isolation of cells expressing the Venus reporter gene from the sgRNA-carrying vector. Sorted cells were seeded at low density to allow the isolation of single-cell-derived colonies. In the first round of transfection, sgRNAs targeting MAF were used and colonies were screened for full-length deletion of the MAF CDS using PCR and Sanger sequencing of the edited locus. Cells were cultured under conditions for iPS cells, as described above. MAF KO iPSC were subjected to a second round of transfection using MAFB-targeting sgRNAs, followed by PCR and sequencing analysis of the obtained colonies. Three MAF/MAFB DKO (MAF-DKO) iPSC clones were isolated.

### Example 7

### Generation of human MAF/MAFB DKO macrophages

Human MAF/MAFB DKO macrophages were generated by directed differentiation of human MAF/MAFB DKO iPSC into macrophages. Directed differentiation of iPSC into macrophages was based on previously published Embryoid bodies (EBs) based protocols (Buchrieser et al., 2017). Embryoid bodies (EBs) were formed by seeding a single-cell suspension of wild-type or MAF-DKO iPSC at a density of 12,500 cells/well into an ultra-low attachment U-shaped-bottom 96-well plate (Nunclon Sphera, Thermo Fisher, #174925), resuspended in EB medium consisting of StemFlex Medium (Thermo Fisher, #A3349401) supplemented with 50 ng/ml BMP-4 (R&D Systems, #314-BP), 20 ng/ml SCF (R&D systems, 255-SC), 50 ng/ml VEGF (Peprotech, AF-100-20A) and 10 µM Y-27632. The 96-well plate was centrifuged for 3 minutes at 100 g to collect the cells at the bottom, and placed at 37°C, 5% CO2 for 4 days. After 1 and 2 days, half of the EB medium was replaced with freshly prepared EB medium.

Wild-type and MAF-DKO EBs were indistinguishable by size or morphology.

After 4 days, EBs were selected manually, resuspended in EB differentiation medium consisting of X-VIVO 15 (Lonza, #BE02-060F) supplemented with 2 mM GlutaMAX (Thermo Fisher, #35050038), 0.055 mM 2-mercaptoethanol (Sigma-Aldrich, #M6250), 100 ng/ml human M-CSF (Thermo Fisher, #PHC9504), 25 ng/ml human IL-3 (R&D systems, #203-IL), and seeded into ultra-low attachment 6-well tissue-culture plates (8 EBs/well) or 90 mm tissue-culture dishes (24 EBs/dish; Nunclon Sphera, Thermo Fisher, #174932 and #174945). Two thirds of the culture medium were exchanged with fresh differentiation medium every 5 days. Production of monocytes/macrophages from EBs started around day 15 post EB seeding, and suspension cells were harvested, counted and used for immunophenotyping by flow cytometry or EdU incorporation assays.

For further experiments the cells harvested from the supernatant of the EB cultures were replated for final macrophage differentiation in RPMI supplemented with 10% FBS (PAA-GE Healthcare, A15-101), supplemented with 100 units/ml penicillin, 100 ug/ml streptomycin (Thermo Fisher, #15140122), 2 mM GlutaMAX (Thermo Fisher, #35050038), 1 mM sodium pyruvate (Thermo Fisher, #11360-039), 50 ng/ml M-CSF (Thermo Fisher, #PHC9504), seeded into ultra-low attachment 12-well tissue-culture plates (Nunclon Sphera, Thermo Fisher #174931, #174932) and 50 ng/ml GM-CSF (Peprotech, #300-03); 37°C, 5% CO2. Cells were counted manually using a Neubauer hemocytometer or automatically with a CASY cell counter (OMNI Life Science).

Deletion of MAF and MAFB had no effect on myeloid differentiation capacity, and, similar to wild-type EBs, MAF-DKO EBs started releasing monocytes/macrophages into the EB differentiation medium around day 15 post EB seeding. MAF-DKO suspension cells were viable and contained macrophages.

### Example 8

### Human MAF-DKO macrophages are resistant to M-CSF induction of profibrotic genes

Generation of macrophages through monocyte intermediates from myeloid cells released from EBs subjected to a macrophage differentiation protocol is well known in the art (Alsinet et al., 2022). The protocol is therefore ideally suited to characterize the response of human MAF-DKO monocytes and monocyte derived macrophages to a fibrotic stimulus.

To generate macrophages from EBs, briefly the supernatant cells were harvested from the EB cultures on day 20 post EB-seeding as mentioned in example 7 and plated in macrophage differentiation medium containing 50 ng/ml M-CSF and GM-CSF for 5 days, both wild-type and MAF-DKO macrophages. After medium exhaustion, both wild-type and MAF-DKO macrophages were then exposed to fresh M-CSF containing medium for 2 hours and harvested and subject to cell sorting (DAPI-negative, CD45 positive, CD11b positive cells) and analyzed by bulk-RNA sequencing.

The transcriptomic signatures from bulk-RNA sequencing of human wildtype and MAF-DKO macrophages showed that also human iPSC derived wild-type macrophages expressed well-defined fibrosis genes **(****Figure 12****),** as defined in example 4 as profibrotic genes induced by 24 hours of M-CSF in wild-type mouse monocytes and monocyte derived macrophages shown in **table 2.**

Interestingly, both mouse and human Maf-DKO macrophages failed to induce profibrotic genes after exposure to M-CSF **(****Figure 12****).** This Indicated that the characteristic of fibrosis resolving monocyte derived macrophages are conserved in human macrophages.

### Example 9

### Single human MAF-DKO macrophages are resistant to M-CSF induction of profibrotic gene signatures

Human macrophages were independently generated essentially as described in example 8 with minor modifications. Briefly, in order to generate macrophages from EBs, the supernatant cells were harvested from the EB cultures that contained 100ng/ml human M-CSF on day 17 post EB-seeding as described in example 7 and plated in macrophage differentiation medium containing 50 ng/ml M-CSF and GM-CSF for 7 days. After medium exhaustion, cells were harvested 24h after exposure to fresh M-CSF containing medium and fixed following the Chromium fixed RNA profiling protocol (10x Genomics). The fixed samples were further processed using Chromium Fixed RNA kit (human). Prepared libraries were then sequenced using the Illumina NovaSeq S4 system targeting a read depth of 400 million reads/cell and processed for downstream analysis.

Analysis of transcriptomic signatures of wild-type and MAF-DKO macrophages from the single-cell data set revealed that the profibrotic gene signatures defined in the literature as shown in example 4 (Strunz et al., 2019; Aran et al., 2019; Joshi et al., 2020; Xie et al., 2018; Morse et al., 2019; Reyfman et al., 2019; Ayaub et al., 2021; Wendisch et al., 2021; Liao et al., 2020; Grant et al., 2021; Bharat et al., 2020; Sikkema et al., 2023; Tran et al., 2020; Remmerie et al., 2020; Fabre et al., 2023) and profibrotic genes defined in table 2 induced in mouse Maf-DKO monocyte derived macrophages by 24 hour exposure of M-CSF were also induced in human wild-type iPSC-derived macrophages 24 hours after exposure to fresh M-CSF (Figure 13). Importantly human iPSC derived MAF-DKO macrophages were also resistant to induction of the profibrotic gene expression signature as defined in example 4 and table 2 for mouse monocyte derived macrophages after 24 hours of M-CSF exposure **(****Figure 13****).** The example demonstrates that the characteristic resistance of fibrosis resolving macrophages to the induction of profibrotic gene signatures in response to M-CSF are conserved in human macrophages.

### Summary

Surprisingly, it was found and demonstrated herein that cell therapy with macrophages resistant to M-CSF induction of a profibrotic gene signature shows a dominant effect in resolving fibrosis against the background of endogenous resident macrophages and monocytes after administration to a recipient.

In a particular example this dominant fibrosis resolving effect was demonstrated to be achieved with genetically engineered monocyte derived macrophages resistant to M-CSF induction of a profibrotic gene signature.

The transcription factors MafB and cMaf are specifically expressed when monocytes differentiate to macrophages and in tissue resident macrophages. Surprisingly, conditional deletion of MafB and cMaf in myeloid cells as provided by the invention revealed macrophages in a fibrotic niche of bleomycin induced fibrotic lungs resistant to the induction of fibrotic gene signatures. Moreover, conditional deletion of MafB and cMaf in myeloid cells resulted in strong reduction of bleomycin induced fibrosis in the lung and strongly improved health status of individuals. In particular, the observation of reduced pulmonary fibrosis in the Maf-DKO mice was strongly associated with the striking reduction of profibrotic signature genes in monocyte derived Maf-DKO macrophages such as PDGF. Secretion of PDGF by macrophages and M-CSF secretion by fibroblasts or myofibroblasts in the fibrotic niche leads to mutual cross-stimulation of these cell types and a feed-forward loop that drives fibrosis (Zhou et al., 2018; Joshi et al., 2019; Adler et al., 2020; Buechler et al., 2021). The invention evidences that Maf-DKO monocyte-derived macrophages in bleomycin treated mice and M-CSF stimulated Maf-DKO monocyte derived macrophages show a strongly reduced induction of the Pdgfa gene compared to WT cells, indicating that Maf-DKO monocytes remain non-responsive to M-CSF upon entering a fibrotic niche and fail to secrete PDGFA, thus rupturing the positive feed forward loop with fibrosis driving myofibroblasts.

It is shown by the invention that Maf-DKO monocytes/macrophages cell transplantation alleviates bleomycin induced pulmonary fibrosis in WT mice. Maf-DKO monocytes were intravenously administered every 2-3 days up to 14 days into WT mice after bleomycin induced fibrosis. Surprisingly, it was observed a significant improvement in weight loss in mice administered with Maf-DKO monocytes **(****Figure 5****).** Further, it was observed a significant alteration in the macrophage composition in the lungs using flow cytometry apparently reverting to homeostatic conditions in mice that received Maf-DKO monocytes. Histological staining and image analysis to measure fibrotic tissue density as well as the collagen deposition in the lungs with preliminary indication in a reduction in fibrotic areas and collagen deposition in the mice that received DKO monocytes.

in a more particular example, genetic engineering of monocytes to delete MafB and Maf revealed monocyte derived macrophages resistant to M-CSF induction of a profibrotic gene signatures. In a further more particular example these engineered monocytes had a dominant fibrosis resolving effect and improved health status when administered therapeutically to individuals with bleomycin induced fibrosis in the lung.

Fibrosis resolving myeloid cells such as macrophages can thus be obtained by inducing resistance to M-CSF induced fibrotic gene signatures. This may be achieved by genetic engineering such as deletion, mutation or inactivation of positive regulators of M-CSF induced fibrotic gene signatures. In a particular example the invention demonstrates that this can be achieved by deletion of MafB and Maf on both alleles respectively. In another example this may also be achieved by deletion, inactivation or mutation of other positive regulators of M-CSF induced fibrotic gene signature genes in myeloid cells. In another example it may also be achieved by genetically engineering myeloid cells to overexpress negative regulators of M-CSF induced fibrotic gene signature genes. In further examples it may also be achieved by exposing myeloid cells such as monocytes or macrophages to compounds that induce resistance of M-CSF induced fibrotic gene signature, such as cytokines, growth factors, extracellular matrix components, signaling molecules, metabolites, epigenetic modifiers or compounds that cause stable epigenetic memory such as in trained immunity, such for example but not limited to beta-glucan or BCG.

The feasibility of using myeloid cells resistant to M-CSF induced profibrotic programming as a cell therapy beyond lung fibrosis was assessed. It was evidenced by the invention that myeloid cells such as macrophages resistant to M-CSF induced expression of profibrotic signatures genes such as monocyte derived macrophages with deletions of Maf and MafB are resistant to the induction of fibrotic macrophage gene signatures from 15 different recent datasets from lung fibrosis, liver fibrosis, Cancer and COVID-19 related fibrosis that described 20 profibrotic macrophage subtypes (Strunz et al., 2019; Aran et al., 2019; Joshi et al., 2019; Xie et al., 2018; Morse et al., 2019; Reyfman et al., 2019; Ayaub et al., 2021; Wendisch et al., 2021; Liao et al., 2020; Grant et al., 2021; Bharat et al., 2020; Sikkema et al., 2023; Tran et al., 2020; Remmerie et al., 2020; Fabre et al., 2023). Surprisingly, a striking reduction of fibrotic gene signatures in Maf-DKO monocyte, macrophage and dendritic cell clusters and more specifically in the recruited monocyte derived Maf-DKO macrophages was observed. This demonstrates that myeloid cells such as macrophages resistant to M-CSF induced expression of profibrotic signatures genes such as monocyte derived macrophages with deletions of Maf and MafB have general therapeutic utility for the treatment of fibrotic disease, such as, but not limited to pulmonary fibrosis, liver fibrosis and fibrotic cancers, e.g. pancreatic cancer

Importantly, human iPSC derived MAF-DKO macrophages were also resistant to the induction of profibrotic gene expression by M-CSF. Profibrotic gene signatures induced by 24 hour exposure to M-CSF in mouse WT but not Maf-DKO monocyte derived macrophages were also induced in human iPSC derived WT but not in Maf-DKO macrophages. This indicates that the characteristics of fibrosis resolving macrophages are conserved in human macrophages. This indicates that human Maf-DKO macrophages have essentially an identical anti-fibrotic phenotype as mouse Maf-DKO macrophages and thus have general utility for use as cellular therapy against fibrotic disease, such as, but not limited to pulmonary fibrosis, liver fibrosis and fibrotic cancers, e.g. pancreatic cancer

## Claims

1. A myeloid cell for use in the treatment of fibrosis, wherein said myeloid cell is resistant to M-CSF induced expression of a profibrotic gene signature.

2. The myeloid cell for use according to claim 1, wherein said myeloid cell is resistant to M-CSF induced expression of a profibrotic gene signature after exposure to M-CSF for at least 24 hours.

3. The myeloid cell for use according to claim 1 or 2, wherein in said myeloid cell the expression level of at least one mRNA of at least one gene or more genes selected from the group consisting of PMP22, IGF1, C1QC, C1QA, C1QB, FOLR2, TMEM37, MERTK, RGL1, DAB2, C3AR1, PDPN, CD93, MARCKS, STAB1, CD63, C5AR1, HMOX1, LGMN, PDGFA, CD109, MRC1, ACP2, C5AR2, IER3, and ETS2 is at least 3-fold lower than the expression level of said at least one mRNA of said at least one gene in an otherwise identical myeloid cell.

4. The myeloid cell for use according to any one of claims 1 to 3, wherein in said myeloid cell the expression level of at least one mRNA of at least one gene or more genes selected from the group consisting of RGL1, DAB2, C3AR1, PDPN, CD93, MARCKS, STAB1, CD63, C5AR1, HMOX1, LGMN and PDGFA is at least 7-fold lower than the expression level of said at least one mRNA of said at least one gene in an otherwise identical myeloid cell.

5. The myeloid cell for use according to any one of claims 1 to 4, wherein in said myeloid cell the expression level of at least one mRNA of at least one gene or more genes, such as one, two, three, four or five or more genes selected from the group consisting of PMP22, IGF1, C1QC, C1QA, C1QB, FOLR2, TMEM37, and MERTK is not upregulated when compared to the expression level of said at least one mRNA of said at least one gene in an otherwise identical myeloid cell.

6. The myeloid cell for use according to any one of claims 1 to 5, wherein said fibrosis is idiopathic pulmonary fibrosis (IPF), fibrosis occurring in chronic obstructive pulmonary disease (COPD), interstitial lung disease (ILD), viral infections such as severe COVID-19 and pneumonia, and in other diseases such as end-stage liver diseases including MASH, NASH and liver cirrhosis, autoimmune related diseases such as SLE and GvHD and cancer such as hepatic, pancreatic, gastric, esophageal, and colonic cancer.

7. The myeloid cell for use according to any one of claims 1 to 6, wherein said fibrosis is idiopathic pulmonary fibrosis (IPF).

8. The myeloid cell for use according to any one of claims 1 to 7, wherein said myeloid cell is selected from the group consisting of a macrophage, a monocyte, and a monocyte derived macrophage.

9. The myeloid cell for therapeutic use according to claim 8, wherein said wherein said myeloid cell, macrophage, monocyte, and monocyte derived macrophage are independently derived from bone marrow, peripheral blood, umbilical cord blood, placenta or from iPS cells.

10. The myeloid cell for use according to any one of claims 1 to 9, wherein said myeloid cell is genetically modified.

11. The myeloid cell for use according to any one of claims 1 to 10, wherein said myeloid cell comprises at least one mutation in both alleles of a gene located on a chromosome.

12. The myeloid cell for use according to any one of claims 1 to 12, wherein said myeloid cell is a genetically engineered myeloid cell, such as a cell in which the expression or activity of the transcription factors MAFB and/or MAF is reduced, inhibited or abolished.

13. The myeloid cell for use according to any one of claims 1 to 12, wherein said myeloid cell is a MAF-DKO monocyte or a MAF-DKO macrophage.

14. The myeloid cell for use according to any one of claims 1 to 13, wherein said myeloid cell is a human myeloid cell.

15. A collection of myeloid cells for use in the treatment of fibrosis comprising myeloid cells according to any one of claims 1 to 14, wherein the number of myeloid cells in said collection is at least 10000000.
